# EUROPEAN PATENT SPECIFICATION

(11) **EP 1 123 296 B1**
(45) Date of publication and mention of the grant of the patent: **17.09.2003**
(21) Application number: 99970992.6
(22) Date of filing: 19.10.1999
(51) Int. Cl.: C07D 487/04, A61K 31/519, C07D 231/14, C07D 401/12

(54) **PYRAZOLOPYRIMIDINONE cGMP PDE5 INHIBITORS FOR THE TREATMENT OF SEXUAL DYSFUNCTION**
PYRAZOLOPYRIMIDINONE, CGMP PDE5 INHIBITOREN, ZUR BEHANDLUNG VON SEXUELLEN FUNKTIONSSTÖRUNGEN
INHIBITEURS DE cGMP PDE5 DE PYRAZOLOPYRIMIDINONE SERVANT A TRAITER LE DYSFONCTIONNEMENT SEXUEL

(30) Priority: 23.10.1998 GB 9823102; 23.10.1998 GB 9823101
(43) Date of publication of application: 16.08.2001
(73) Proprietor: Pfizer Limited, Sandwich, Kent CT13 9NJ (GB); PFIZER INC., New York, N.Y. 10017 (US)
(72) Inventor: BUNNAGE, Mark, Edward Pfizer Central Research, Sandwich Kent CT13 9NJ (GB); MAW, Graham, Nigel Pfizer Central Research, Kent CT13 9NJ (GB); RAWSON, David, James Pfizer Central Research, Sandwich Kent CT13 9NJ (GB); WOOD, Anthony Pfizer Central Research, Sandwich Kent CT13 9NJ (GB); MATHIAS, John, Paul Pfizer Central Research, Sandwich Kent CT13 9NJ (GB); STREET, Stephen, Derek, A. Pfizer Central Research, Sandwich Kent CT13 9NJ (GB)
(74) Representative: McNeeney, Stephen Phillip
(86) International application number: IB9901706
(87) International publication number: WO00024745

(56) References cited:
- EP-A- 0 463 756

## Description

### Field of the Invention

This invention relates to pharmaceutically useful compounds, in particular compounds which are useful in the inhibition of cyclic guanosine 3',5'-monophosphate phosphodiesterases (cGMP PDEs), such as type 5 cyclic guanosine 3',5'-monophosphate phosphodiesterases (cGMP PDE5). The compounds therefore have utility in a variety of therapeutic areas, including male erectile dysfunction (MED).

### Prior Art

International patent application WO 94/28902 discloses the use of certain pyrazolopyrimidinone compounds in the treatment of impotence.

EP 463 756 A1 discloses certain pyrazolopyrimidone compounds for use in the treatment of cardiovascular disorders.

### Disclosure of the Invention

According to a first aspect of the invention there is provided compounds of formulae IA and IB: 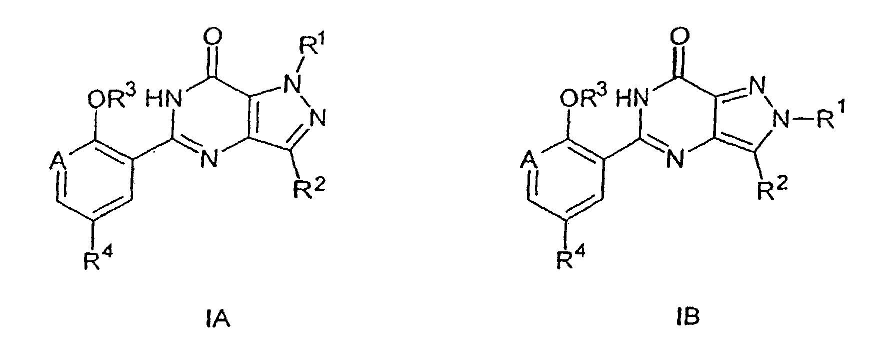 wherein
A represents N;
R¹ represents H, lower alkyl, Het, alkylHet, aryl or alkylaryl, which latter five groups are all optionally substituted (and/or, in the case of lower alkyl, optionally terminated) by one or more substituents selected from halo, cyano, nitro, lower alkyl, OR⁵, C(O)R⁶, C(O)OR⁷, C(O)NR⁸R⁹, NR^{10a}R^{10b} and SO₂NR^{11a}R^{11b};
R² represents C(O)NR¹²R¹³, C(O)OR¹², NR¹²R¹³, N(H)SO₂R¹², N(H)SO₂NR¹²R¹³, N(H)C(O)R¹², OR^{12a}, lower alkyl (which alkyl group is interrupted by one or more of O, S or N(R¹²) and/or substituted or terminated by C(O)NR¹²R¹³, C(O)OR¹² or aryl or Het¹), cyano, aryl or Het¹;
R³, R¹² and R¹³ independently represent H or lower alkyl, which alkyl group is optionally substituted and/or optionally terminated by one or more substituents selected from aryl, Het, halo, cyano, nitro, OR⁵, C(O)R⁶, C(O)OR⁷, C(O)NR⁸R⁹, NR^{10a}R^{10b} and SO₂NR^{11a}R^{11b};
R⁴ represents SO₂NR¹⁴R¹⁵;
R¹⁴ and R¹⁵, together with the nitrogen to which they are attached, form Het;
Het represents an optionally substituted four- to twelve-membered heterocyclic group, which group contains at least one nitrogen atom and, optionally, one or more further heteroatoms selected from nitrogen, oxygen and sulphur;
Het¹ represents an optionally substituted four- to twelve-membered heterocyclic group, which group contains at least one nitrogen atom or at least one oxygen atom and, optionally, one or more further heteroatoms selected from nitrogen, oxygen and sulphur; and
R⁵, R⁶, R⁷, R⁸, R⁹, R^{11a}, R^{11b} and R^{12a} independently represent, at each occurrence when used herein, H or lower alkyl;
R^{10a} and R^{10b}, at each occurrence when used herein, either independently represent, H or lower alkyl or, together with the nitrogen atom to which they are attached, represent azetidinyl, pyrollidinyl or piperidinyl;
or a pharmaceutically, or a veterinarily, acceptable derivative thereof;
which compounds are referred to together hereinafter as "the compounds of the invention".

The term "aryl", when used herein, includes six- to ten-membered carbocyclic aromatic groups, such as phenyl and naphthyl. Each "aryl" group identified herein is optionally substituted with one or more substituents selected from halo, cyano, nitro, lower alkyl, OR⁵, C(O)R⁶, C(O)OR⁷, C(O)NR⁸R⁹, NR^{10a}R^{10b}, SO₂NR^{11a}R^{11b} and N(H)SO₂R¹².

The terms "Het" and "Het¹", when used herein, include four- to twelve-membered, preferably four- to ten-membered, ring systems, which may be wholly or partly aromatic in character. Each "Het/Het¹" group identified herein is optionally substituted with one or more substituents selected from halo, cyano, nitro, lower alkyl (which alkyl groups may itself be optionally substituted or terminated as defined below), OR⁵, C(O)R⁶, C(O)OR⁷, C(O)NR⁸R⁹, NR^{10a}R^{10b}, SO₂NR^{11a}R^{11b} and N(H)SO₂R¹². The term thus includes groups such as optionally substituted azetidinyl, pyrrolidinyl, imidazolyl, indolyl, oxadiazolyl, thiadiazolyl, triazolyl, tetrazolyl, oxatriazolyl, thiatriazolyl, pyridazinyl, morpholinyl, pyrimidinyl, pyrazinyl, pyridinyl, quinolinyl, isoquinolinyl, piperidinyl, benzodioxalyl, pyrazolyl, imidazopyridinyl, furanyl, tetrahydrofuranyl and piperazinyl, e.g. 4-R¹⁶-piperazinyl, wherein R¹⁶ represents H or lower alkyl, which latter group is optionally substituted or terminated by one or more substituents selected from aryl, Het, halo, cyano, nitro, OR⁵, C(O)R⁶, C(O)OR⁷, C(O)NR⁸R⁹, NR^{10a}R^{10b}, SO₂NR^{11a}R^{11b} and N(H)SO₂R¹².

"Het" and "Het¹" groups may also be in the form of an N-oxide.

Azetidinyl, pyrollidinyl and piperidinyl groups that R^{10a}, R^{10b} and the nitrogen atom to which they are attached may together represent may also be substituted with one or more substituents selected from halo, cyano, nitro, lower alkyl (which alkyl groups may itself be optionally substituted or terminated as defined below), OR⁵, C(O)R⁶, C(O)OR⁷, C(O)NR⁸R⁹, NR^{10a}R^{10b}, SO₂NR^{11a}R^{11b} and N(H)SO₂R¹².

For the avoidance of doubt, the nitrogen atom to which R¹⁴ and R¹⁵ are attached is the nitrogen atom that must be present in the relevant Het group.

The term "lower alkyl", when used herein, includes C₁₋₆ alkyl. Alkyl groups which R¹, R², R³, R⁵, R⁶, R⁷, R⁸, R⁹, R^{10a}, R^{10b}, R^{11a}, R^{11b}, R¹², R^{12a}, R¹³ and R¹⁶ may represent, and with which R¹, NR^{10a}R^{10b}, aryl, Het and Het¹ may be substituted, may, when there is a sufficient number of carbon atoms, be linear or branched, be saturated or unsaturated, be cyclic, acyclic or part cyclic/acyclic, be interrupted by oxygen, and/or be substituted by one or more halo atom.

The terms "alkylHet" and "alkylaryl" include C₁₋₆ alkylHet and C₁₋₆ alkylaryl. The alkyl groups (e.g. the C₁₋₆ alkyl groups) of alkylHet and alkylaryl may, when there is a sufficient number of carbon atoms, be linear or branched, be saturated or unsaturated, and/or be interrupted by oxygen. When used in this context, the terms "Het" and "aryl" are as defined hereinbefore.

Halo groups, with which R¹, R³, R¹², R¹³, R¹⁶, aryl, Het, Het¹ and above-mentioned alkyl groups may be substituted or terminated, include fluoro, chloro, bromo and iodo.

The term "pharmaceutically, and veterinarily, acceptable derivative" includes salts and solvates. Salts which may be mentioned include: acid addition salts, for example, salts formed with inorganic acids such as hydrochloric, hydrobromic, sulphuric and phosphoric acid, with carboxylic acids or with organo-sulphonic acids; base addition salts; metal salts formed with bases, for example, the sodium and potassium salts. Pharmaceutically acceptable derivatives also include C₁ to C₄ alkyl ammonium salts.

Preferred compounds of the invention include those wherein:
R¹ represents H, a linear, branched, cyclic, acyclic and/or part cyclic/acyclic lower alkyl group, alkylHet, or alkylaryl;
R² represents a linear or branched, optionally unsaturated lower alkyl group (which alkyl group is optionally interrupted by one or more of O, S or N(R¹²)), C(O)NR¹²R¹³, NR¹²R¹³, N(H)C(O)R¹², OR^{12a}, aryl or Het¹;
R³ represents linear, branched, cyclic and/or acyclic lower alkyl which is optionally substituted or terminated by one or more substituents selected from Het or OR⁵;
R¹² and R¹³ independently represent H, or linear or branched lower alkyl, provided that, in the case where R² represents NR¹²R¹³, R¹² and R¹³ do not both represent H;
R¹⁴ and R¹⁵, together with the nitrogen to which they are attached represent 4-R¹⁶-piperazinyl, in which R¹⁶ is as hereinbefore defined.

More preferred compounds of the invention include those wherein:
R¹ represents H; a linear or part cycliclacyclic C₁-C₆ alkyl group; C₁-C₂ alkylphenyl, the phenyl group of which is optionally substituted by one or more halo atoms; or C₁-C₃ alkylHet, in which Het represents a six-membered heterocyclic group;
R² represents a linear or branched, optionally unsaturated, C₁₋₆ alkyl group (which alkyl group is optionally interrupted by one or more of O or N(R¹²)), C(O)NR¹²R¹³, NR¹²R¹³, N(H)C(O)R¹², OR^{12a}, an optionally substituted phenyl group, or an optionally substituted Het¹ group (e.g. a pyridinyl, benzodioxazolyl, furanyl, tetrahydrofuranyl, imidazolopyridinyl, pyrazolyl, oxadiazolyl pyrimidinyl or pyrazinyl group);
R³ represents linear or branched C₁-C₄ alkyl, which is optionally terminated by one or more substituents selected from pyridinyl or OR⁵, in which R⁵ represents H or C₁-C₃ alkyl;
R¹² and R¹³ independently represent H or linear or branched C₁-C₃ alkyl, provided that, in the case where R² represents NR¹²R¹³, R¹² and R¹³ do not both represent H;
R^{12a} represents C₁₋₃ alkyl;
R¹⁴ and R¹⁵, together with the nitrogen to which they are attached, represent 4-R¹⁶-piperazinyl, in which R¹⁶ represents a linear or branched C₁-C₃ alkyl group which is optionally terminated by OH.

Particularly preferred compounds of the invention include those wherein:
R¹ represents H, a linear or part cyclic C₁-C₅ alkyl group (e.g. methyl, ethyl, propyl or cyclobutylmethyl), CH₂phenyl, CH₂(bromophenyl) (e.g. CH₂(4-bromophenyl)), C₁-C₂ alkylHet, in which Het represents pyridin-2-yl, pyridin-3-yl, pyridin-4-yl, pyrimidin-2-yl or morpolin-4-yl;
R² represents a linear or branched, optionally unsaturated, C₁₋₄ alkyl group (which alkyl group is optionally interrupted by an oxygen atom or an N(R¹²) group), C(O)NR¹²R¹³, NR¹²R¹³, N(H)C(O)R¹², OR^{12a}, phenyl (optionally substituted by one or more substituent (e.g. one or more of C₁₋₃ alkyl, C₁₋₃ alkoxy (which latter two groups are optionally substituted by one or more halo atom and/or optionally interrupted by an oxygen atom), halo and cyano)), pyridin-2-yl, pyridin-3-yl, pyrimidin-5-yl, pyrazin-2-yl (which latter four groups are optionally substituted (e.g. by one or more halo, C₁₋₃ alkyl, C₁₋₃ alkoxy or NR^{10a}R^{10b} groups)), furan-2-yl, furan-3-yl, tetrahydrofuran-2-yl, tetrahydrofuran-3-yl, benzodioxalyl, imidazolo[1,2-a]pyridin-6-yl (which latter six groups are optionally substituted), pyrazol-4-yl or 1,3,4-oxadiazol-2-yl (which latter two groups are optionally substituted (e.g. by one or more C₁₋₃ alkyl groups));
R³ represents C₂-C₄ alkyl optionally terminated with OC₁-C₂ alkyl or pyridin-2-yl;
R^{10a} and R^{10b} independently represent H or C₁₋₂ alkyl or, together with the nitrogen atom to which they are attached, represent azetidinyl;
R¹² and R¹³ independently represent H, methyl or ethyl, provided that, in the case where R² represents NR¹²R¹³, R¹² and R¹³ do not both represent H;
R^{12a} represents methyl or ethyl;
R¹⁴ and R¹⁵, together with the nitrogen to which they are attached represent 4-R¹⁶-piperazinyl, in which R¹⁶ represents methyl or ethyl, the latter of which is optionally terminated with OH.

Most preferred compounds of the invention include the compounds of the

Examples described hereinafter.

According to a further aspect of the invention there is provided a compound of formula IA or IB as hereinbefore defined, provided that:
(a) R² does not represent OR^{12a} or lower alkyl substituted or terminated by Het¹;
(b) Het¹ represents Het;
(c) R^{10a} and R^{10b} do not, together with the nitrogen atom to which they are attached, represent azetidinyl, pyrollidinyl or piperidinyl;
(d) alkyl groups, as defined herein, are not substituted by one or more halo atom.

The compounds of the invention may exhibit tautomerism. All tautomeric forms of the compounds of formulae IA and IB, and mixtures thereof, are included within the scope of the invention.

The compounds of the invention may also contain one or more asymmetric carbon atoms and may therefore exhibit optical and/or diastereoisomerism. Diastereoisomers may be separated using conventional techniques e.g. by fractional crystallisation or chromatography. The various stereoisomers may be isolated by separation of a racemic or other mixture of the compounds using conventional techniques e.g. fractional crystallisation or HPLC. The desired optical isomers may be prepared by reaction of the appropriate optically active starting materials under conditions which will not cause racemisation or epimerisation. Alternatively, the desired optical isomers may be prepared by resolution, either by HPLC of the racemate using a suitable chiral support or, where appropriate, by fractional crystallisation of the diastereoisomeric salts formed by reaction of the racemate with a suitable optically active acid or base. All stereoisomers are included within the scope of the invention.

Also included within the scope of the invention are radiolabelled derivatives of compounds of formulae IA and IB which are suitable for biological studies.

### Preparation

According to a further aspect of the invention there is provided processes for the preparation of compounds of the invention, as illustrated below.

The following processes are illustrative of the general synthetic procedures which may be adopted in order to obtain the compounds of the invention:
1. Compounds of formulae IA and IB may be prepared by cyclisation of corresponding compounds of formulae IIA and IIB, respectively: 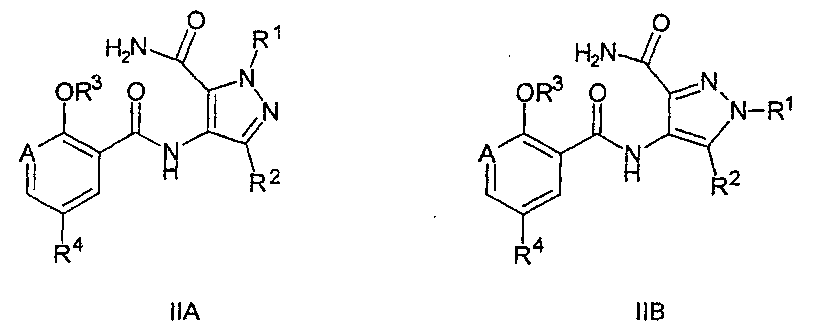 wherein R¹, R², R³, R⁴ and A are as defined previously for compounds of formulae IA and IB.
   This cyclisation may be accomplished under basic, neutral or acidic conditions using known methods for pyrimidone ring formation. Preferably, the cyclisation is performed under basic conditions using an alkali metal salt of an alcohol or amine, such as potassium tert-butoxide or potassium bis(trimethylsilyl) amide, in the presence of a suitable solvent (e.g. an alcohol), for example at elevated (e.g. reflux) temperature (or, if a sealed vessel is employed, at above reflux temperature). The skilled person will appreciate that, when an alcohol is selected as solvent, an appropriate alcohol of formula R³OH, or a sterically hindered alcohol, e.g. iso-propanol or 3-methyl pentan-3-ol, may be used if it is intended to mitigate alkoxide exchange at either the 2-position of the pyridin-3-yl, or the phenyl, substituent. Compounds of formulae IIA and IIB may be prepared by reaction of corresponding compounds of formulae IIIA and IIIB, respectively: 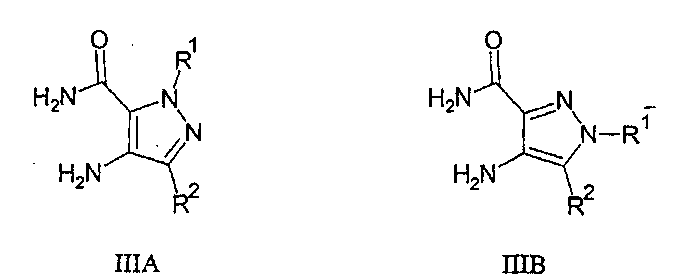 wherein R¹ and R² are as defined previously for compounds of formulae IIA and IIB, with a compound of formula IV or a carboxylic acid derivative thereof: 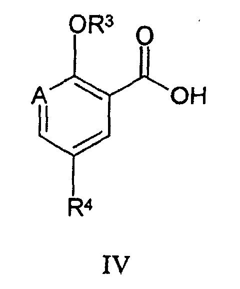 wherein R³, R⁴ and A are as defined previously for compounds of formulae IIA and IIB.
   This coupling reaction may be achieved by conventional amide bond forming techniques which are well known to those skilled in the art. For example, an acyl halide (e.g. chloride) derivative of a compound of formula IV may be reacted with a compound of formula IIIA or IIIB in the presence of an excess of a tertiary amine, such as triethylamine or pyridine, optionally in the presence of a suitable catalyst, such as 4-dimethylaminopyridine, in a suitable solvent such as dichloromethane, at a temperature of about 0°C to room temperature.
   A variety of other amino acid coupling methodologies may be used to couple the compound of formula IIIA or IIIB with the compound of formula IV. For example, the acid of formula IV or a suitable salt thereof (e.g. sodium salt) may be activated with an appropriate activating reagent, e.g. a carbodiimide, such as 1,3-dicyclohexylcarbodiimide or 1-(3-dimethylaminopropyl)-3-ethylcarbodiimide hydrochloride optionally in the presence of 1-hydroxybenzotriazole hydrate and/or a catalyst such as 4-dimethylaminopyridine; a halotrisaminophosphonium salt such as bromotris(pyrrolidino)phosphonium hexafluorophosphate; or a suitable pyridinium salt such as 2-chloro-1-methyl pyridinium chloride. Either type of coupling reaction may be conducted in a suitable solvent such as dichloromethane or tetrahydrofuran, optionally in the presence of a tertiary amine such as N-methylmorpholine or N-ethyldiisopropylamine (for example when either the compound of formula IIIA or IIIB, or the activating agent is presented in the form of an acid addition salt), at from about 0°C to about room temperature. Preferably, from about 1 to 2 molecular equivalents of the activating reagent and from 1 to 3 molecular equivalents of any tertiary amine present may be employed.
   Alternatively, the carboxylic acid function of IV may be activated using an excess of a reagent such as N,N'-carbonyldiimidazole in an appropriate solvent, e.g. ethyl acetate, dichloromethane or butan-2-one, at from about room temperature to about 80°C, followed by reaction of the intermediate imidazolide with a compound of the formula IIIA or IIIB at from about 20°C to about 90°C.
   In a further variation, a compound of formula IA or IB in which A is CH may be formed in a one-pot procedure by coupling a compound of formula IIIA or IIIB with an acyl chloride derivative of a compound of formula IV and by cyclising the resultant intermediate compound of formula IIA or IIB, using the methods as described previously. The one-pot procedure may further involve an *in-situ* coupling and cyclisation reaction to form a compound of formula IA or IB. Preferably, pyridine may serve as an acid scavenger and as the solvent for the *in-situ* coupling and cyclisation reaction.
2. Compounds of formulae IA and IB, in which R² represents C(O)NR¹²R¹³, and R¹² and R¹³ are as defined previously for compounds of formulae IA and IB, may be prepared by reaction of corresponding compounds of formulae IA and IB, in which R² represents C(O)OH (or a carboxylic acid derivative thereof) with a compound of formula HNR¹²R¹³, in which R¹² and R¹³ are as previously defined for compounds of formulae IA and IB.
   This reaction may be accomplished using analogous amide bond forming techniques to those previously described for compounds of formulae IIA and IIB. Alternatively, when R¹² and R¹³ both represent hydrogen and A represents CH, the coupling reaction may be performed by reaction with ammonia in methanol, at 100° C under pressure.
3. Compounds of formulae IA and IB, in which R² represents C(O)OR¹², may be prepared by cyclisation of corresponding compounds of formulae VIA and VIB, respectively: 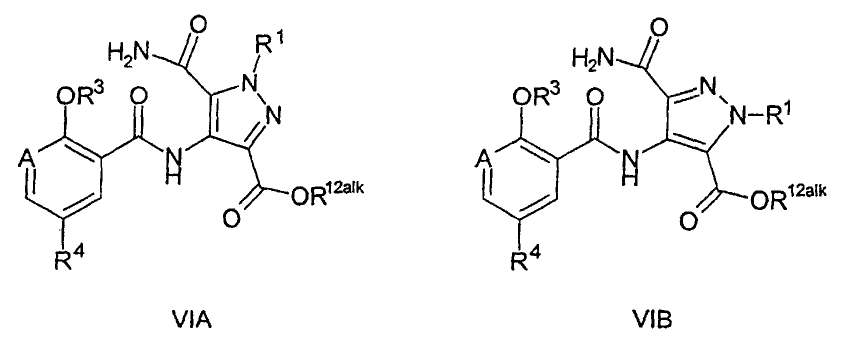 wherein R¹, R³, R⁴ and A are as defined previously for compounds of formulae IA and IB, and R^{12alk} represents an optionally substituted lower alkyl group, as defined hereinbefore, followed by removal of the alkyl group R^{12alk} (if required) by hydrolysis and/or (if required) exchange with a further optionally substituted alkyl group.
   Typically, the cyclisation reaction is accomplished using analogous methods to those previously described for compounds of formulae IIA and IIB.
   Compounds of formulae VIA and VIB may be prepared by reaction of corresponding compounds of formulae VIIA and VIIB, respectively: 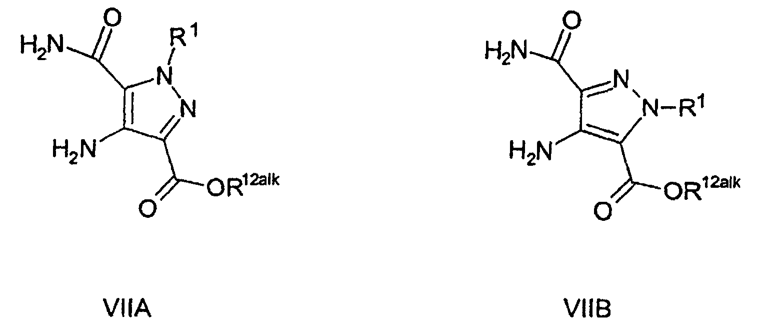 wherein R¹ and R^{12alk} are as defined previously for compounds of formulae VIA and VIB, with a compound of formula IV as defined hereinbefore. The reaction may be accomplished using analogous amide coupling conditions to those described previously in relation to compounds of formulae IIA and IIB.
4. Compounds of formulae IA and IB may alternatively be prepared by reaction of corresponding compounds of formulae VIIIA and VIIIB, respectively: 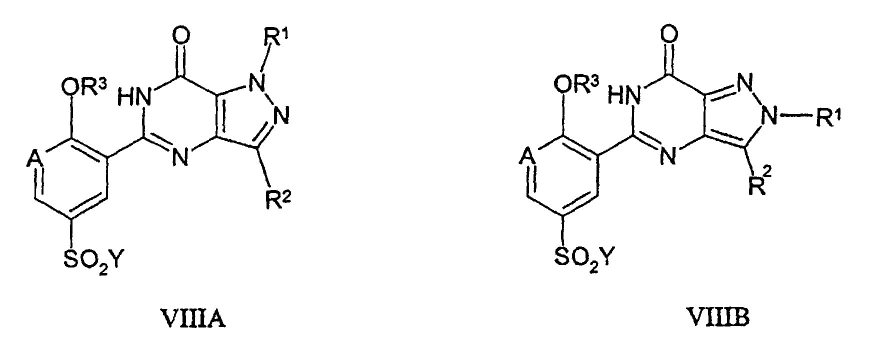 wherein Y is a leaving group, such as halo, preferably chloro, bromo or iodo, and R¹, R², R³ and A are as previously defined for compounds of formulae IA and IB, with a compound of formula IX:

   R¹⁴R¹⁵NH IX

   wherein R¹⁴ and R¹⁵ are as previously defined for compounds of formulae IA and IB.
   This reaction is typically performed at from 0°C to room temperature, in the presence of an appropriate solvent, such as a C₁ to C₃ alcohol or dichloromethane, using an excess of the compound of formula IX and, optionally, in the presence of another suitable base, such as triethylamine.
   Compounds of formula VIIIA and VIIIB, in which A represents N, may be prepared from corresponding compounds of formulae XA and XB, respectively: 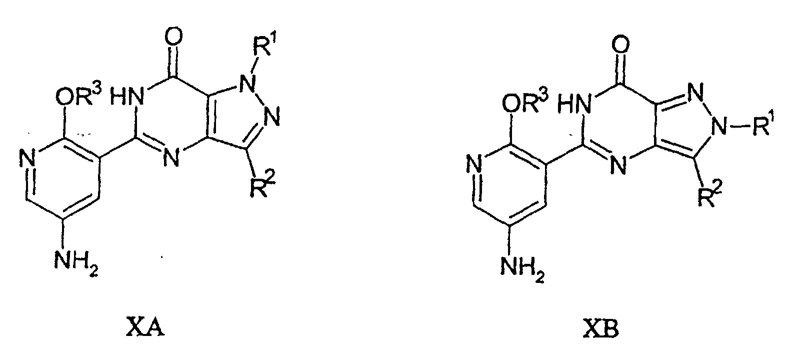 wherein R¹, R² and R³ are as previously defined for compounds of formulae VIIIA and VIIIB, using methods known to those skilled in the art for converting an amino group to an SO₂Y group, in which Y is as previously defined for compounds of formulae VIIIA and VIIIB. For example, compounds of formulae VIIIA and VIIIB in which Y is chloro may be prepared by reacting a corresponding compound of formula XA or XB with about a 1.5 to 2-fold excess of sodium nitrite in a mixture of concentrated hydrochloric acid and glacial acetic acid, at from about -25°C to about 0°C, followed by treatment with excess liquid sulphur dioxide and a solution of about a three-fold excess of cupric chloride in aqueous acetic acid, at from about -30°C (e.g. -15°C) to about room temperature.
   Compounds of formulae XA and XB may be prepared by cyclisation of corresponding compounds of formulae XIA and XIB, respectively: 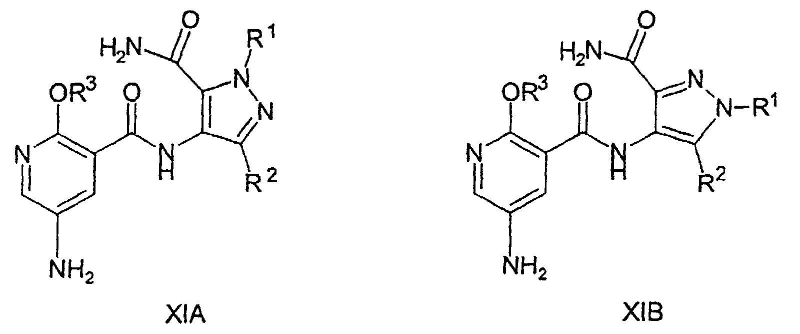 wherein R¹, R² and R³ are as previously defined for compounds of formulae XA and XB. This cyclisation may be carried out using similar techniques to those described hereinbefore for the preparation of compounds of formulae IIA and IIB, but it is preferably base mediated.
   Compounds of formulae XIA and XIB may be prepared by the reduction of corresponding compounds of formulae XIIA and XIIB, respectively: 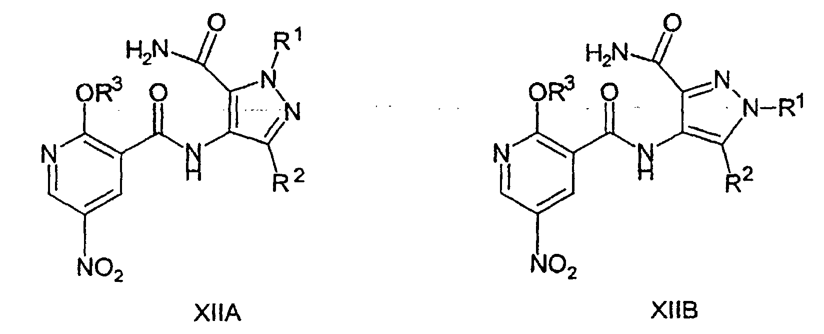 wherein R¹, R² and R³ are as defined previously for compounds of formulae XIA and XIB, by conventional techniques, such as catalytic hydrogenation. Typically, the hydrogenation may be achieved using a Raney nickel catalyst in a suitable solvent such as ethanol at a hydrogen pressure of about 150kPa to 500kPa, especially 345kPa, at from about 40°C to about 50°C. Compounds of formulae XIIA and XIIB may be prepared by reaction of corresponding compounds of formulae IIIA and IIIB as defined hereinbefore, with a compound of formula XIII: 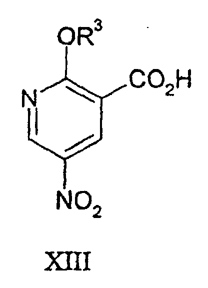 wherein R³ is as previously defined for compounds of formulae XIIA and XIIB. The reaction may be achieved using analogous amide bond forming techniques to those previously described for compounds of formulae IIA and IIB.
   Compounds of formulae XA and XB may alternatively be prepared by reduction of corresponding compounds of formulae XIIIA and XIIIB, respectively: 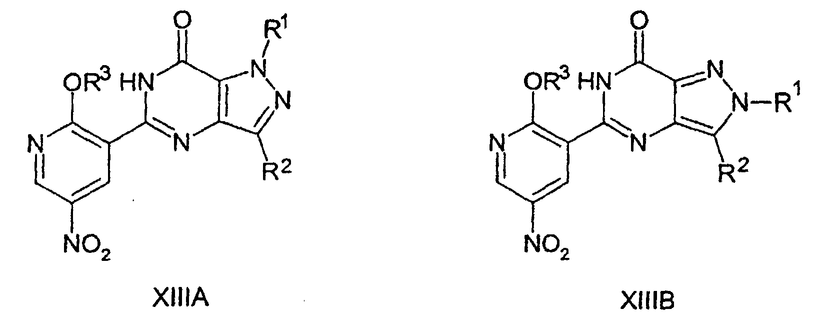 wherein R¹, R² and R³ are as previously defined for compounds of formulae XA and XB. This reduction may be performed under a variety of reaction conditions, for example by catalytic hydrogenation (e.g. using 10% Pd/C in an alcohol, such as ethanol, at 60 psi (415 kPa) H₂ pressure and room temperature) or by transition metal catalysed reduction (e.g. at around room temperature in the presence of iron powder (e.g. 7 eq.) in acetic acid, or TiCl₃ (e.g. 9 eq.) in acetic acid).
   Compounds of formulae XIIIA and XIIIB may be prepared by reaction of a compound of formula XIIIC, 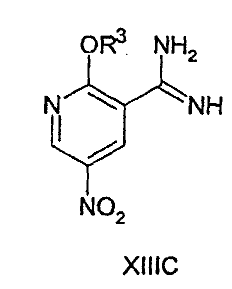 or, preferably, a carboxylic acid addition salt thereof, wherein R³ is as previously defined for compounds of formulae XIIIA and XIIIB, with either:
   (a) a corresponding compound of formula IIIA or formula IIIB, as defined hereinbefore; or
   (b) a corresponding compound of formula XVIIA or formula XVIIB, as defined hereinafter,
   in both cases under conditions such as those described herein. Such reactions may be carried out, for example, using 1.0 to 1.1 equivalents of the amidine compound of formula XIIIC, for example by refluxing in 3-methyl-3-pentanol.
   Compounds of formula XIIIC may be prepared from the corresponding cyanopyridine under conditions well known to those skilled in the art.
   Compounds of formulae XIIIA and XIIIB in which R² represents lower alkyl (which alkyl group is branched and unsaturated at the carbon atom that is attached to the rest of the molecule), NR¹²R¹³, cyano, aryl or Het¹ (which Het¹ group is either aromatic or is unsaturated at the carbon atom that is attached to the rest of the molecule) may alternatively be prepared from corresponding compounds of formulae XIIID or XIIIE, respectively: 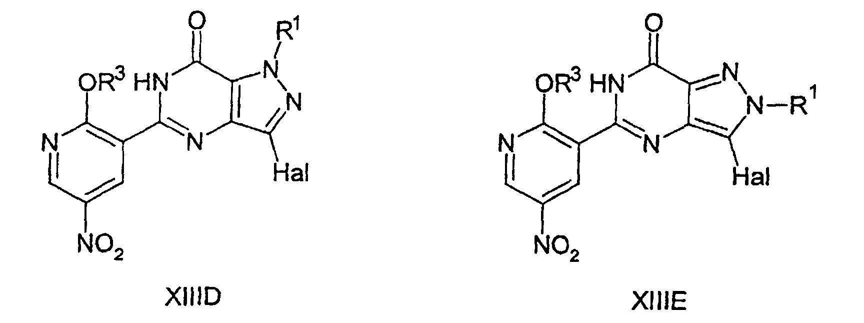 wherein Hal represents Cl, Br or I, preferably I and especially Br, and R¹ and R³ are as previously defined for compounds of formulae XIIIA and XIIIB, for example as described hereinafter for preparation of compounds of formulae IA and IB (see process 6 below). In addition to the process conditions described in process 6 below, suitable coupling conditions include so-called "Suzuki" conditions (e.g. 1.2 eq. of boronic acid, 2 eq. of K₂CO₃ and 0.1 eq. of Pd(PPh₃)₄, refluxing in an approximately 4:1 mixture of dioxane:water, or 2.5 to 3 eq. of CsF, 0.05 to 0.1 eq. of Pd₂(dba)₃ and 0.01 to 0.04 eq of P(o-tol)₃, refluxing in DME); or so-called "Stille" conditions (1.5 eq. of stannane, 10 eq. of LiCl, 0.15 eq. of CuI, and 0.1 eq. of Pd(PPh)₃)₄, refluxing in dioxane, or 5 eq. of stannane, 3.6 eq. of Et₃N, Pd₂(dba) and P(o-tol)₃, refluxing in MeCN).
   Compounds of formula XIIID and XIIIE may be prepared by halogenation of corresponding compounds of formulae XIIIF and XIIIG, respectively: 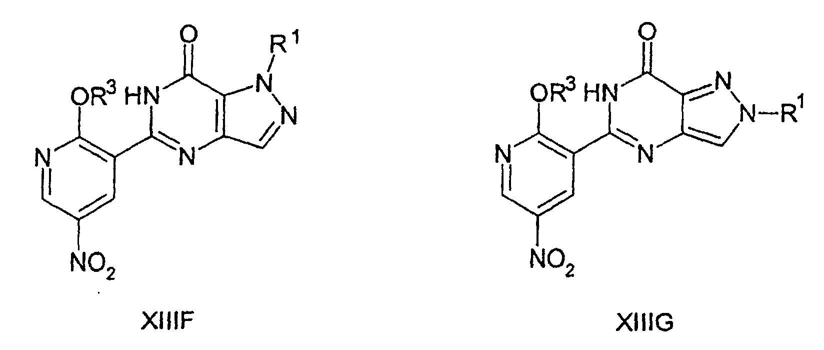 wherein R¹ and R³ are as hereinbefore defined, under conditions known to those skilled in the art (e.g., for bromination, at between room temperature and reflux in the presence of acetic acid as solvent, 1.5 to 2.0 eq. of bromine and e.g. 1.5 to 2.0 eq. of sodium acetate).
   Compounds of formulae VIIIA and VIIIB, in which A is CH, may be prepared from corresponding compounds of formulae XIVA and XIVB, respectively: 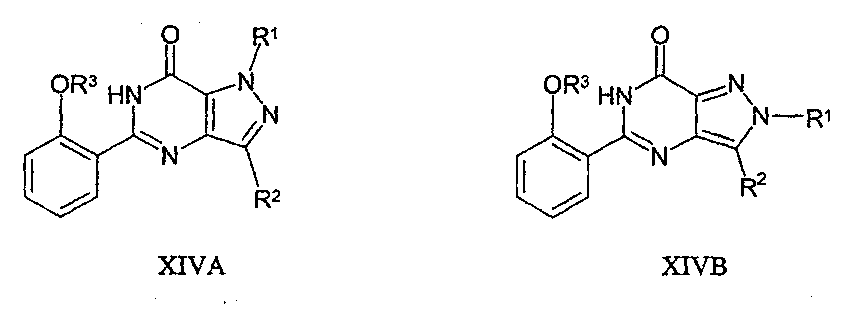 wherein R¹, R² and R³ are as previously defined for compounds of formulae VIIIA and VIIIB, for example using conventional methods for the introduction of a SO₂Y group into an aromatic ring system, such as reaction of a compound of formula XIVA and XIVB with a compound of formula SO₂Y and/or a compound of formula YSO₃H. When Y is chloro, an excess of chlorosulphonic acid, optionally with an excess of thionyl chloride, at from about 0°C to room temperature may be used in an appropriate organic solvent (e.g. dichloromethane).
   Compounds of formulae XIVA and XIVB are available using known techniques. For example, compounds of formulae XIVA and XIVB, in which R¹ represents lower alkyl, alkylHet or alkylaryl, may be prepared by alkylation of corresponding compounds of formulae XVA and XVB, respectively: 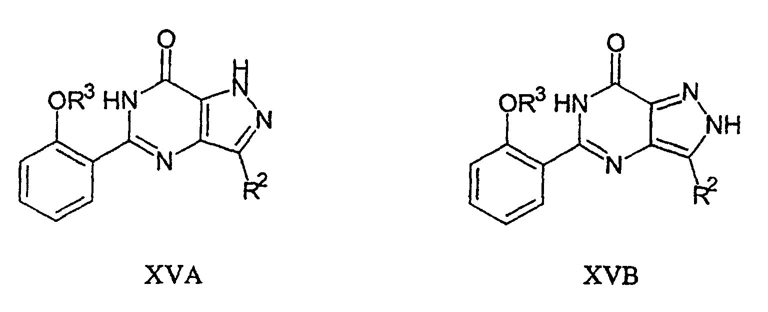 wherein R² and R³ are as previously defined for compounds of formulae XIVA and XIVB, using methods which are well known to those skilled in the art. For example, the reaction may be accomplished by reaction of a compound formula XVA or XVB with a compound of formula R¹L¹, wherein R¹ represents lower alkyl, alkylHet or alkylaryl, and L¹ is a suitable leaving group, using conventional techniques which are well known to those skilled in the art. Preferably, the leaving group is halo (preferably chloro, bromo or iodo) and the alkylation is performed in the presence of an appropriate base (e.g. sodium hydride), in an appropriate solvent (e.g. dimethylformamide), optionally in the presence of sodium iodide or potassium iodide, at from about -70°C to about 100°C.
   Preferably the alkylation is conducted at from about room temperature to about 80°C. Alternatively, compounds of formulae XVA and XVB may be reacted with a compound of formula R¹OH, wherein R¹ represents lower alkyl, alkylHet or alkylaryl, using classical Mitsunobu methodology.
   Compounds of formulae XIVA and XIVB may alternatively be prepared by cyclisation of corresponding compounds of formulae XVIA and XVIB, respectively: 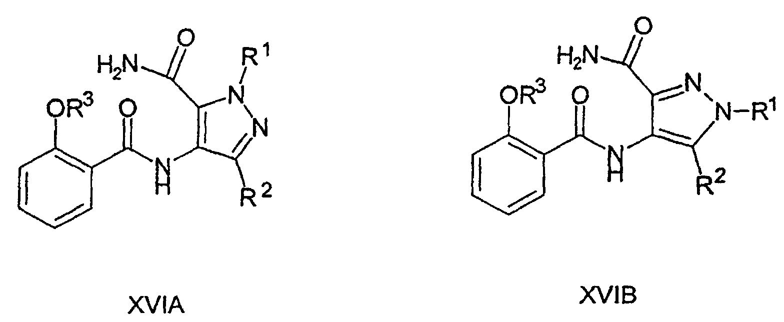 wherein R¹, R² and R³ are as previously defined for compounds of formulae XIVA and XIVB. The cyclisation may be accomplished using analogous conditions to those described previously for compounds of formula IIA and IIB.
   Compounds of formulae XVIA and XVIB may be prepared by coupling corresponding compounds of formulae XVIIA and XVIIB, respectively: 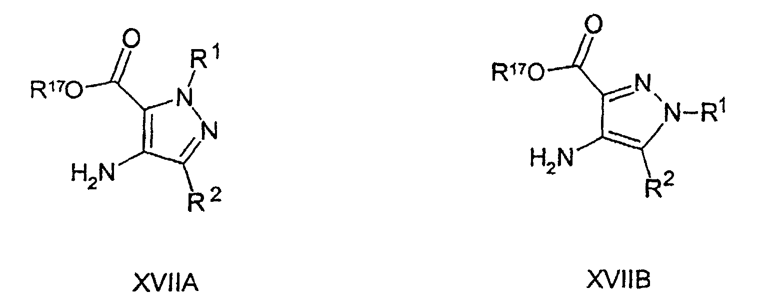 wherein R¹ and R² are as previously defined for compounds of formulae XVIA and XVIB and R¹⁷ represents a lower (e.g. C₁₋₆ alkyl) group, with a compound of formula XVIII or a carboxylic acid derivative thereof: 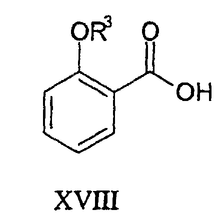 wherein R³ is as previously defined for compounds of formulae XVIA and XVIB, followed by conversion of the C(O)OR¹⁷ group of the resultant amide into C(O)NH₂ using conventional techniques known to those skilled in the art. In a particular embodiment, the *in-situ* conversion of the C(O)OR¹⁷ group of compounds of formulae XVIIA and XVIIB into a C(O)NH₂ group, and the cyclisation of the intermediate formed from the coupling, may be accomplished in a one-pot procedure. Preferably, this one-pot procedure is accomplished with a saturated methanolic ammonia solution, in the presence of base (e.g. potassium *t*-butoxide), under pressure, at elevated temperatures, especially at 100°C.
   Compounds of formulae XIVA and XIVB, in which R² represents C(O)NH₂, may alternatively be prepared by reaction of corresponding compounds of formulae XIXA and XIXB, respectively: 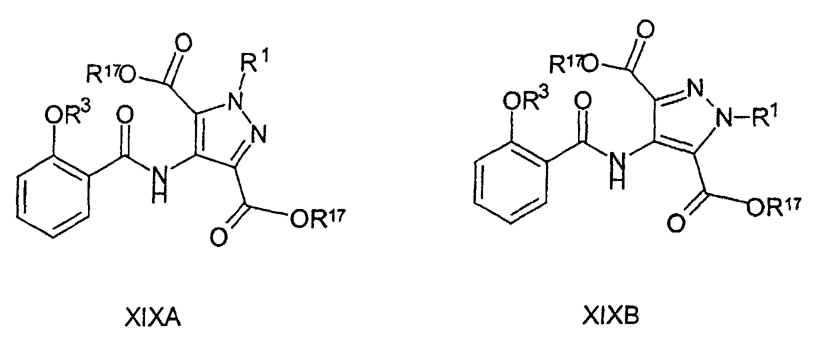 wherein R¹ and R³ are as previously defined for compounds of formulae XIVA and XIVB and R¹⁷ is as previously defined for compounds of formulae XVIIA and XVIIB, with ammonia, followed by cyclisation of the resultant intermediate using similar techniques to those described hereinbefore.
   Preferably, the reaction is accomplished in a saturated methanolic ammonia solution, in a sealed vessel, at elevated temperatures, e.g. 100°C. The cyclisation of the resultant intermediate may be accomplished using analogous techniques to those previously described for preparation of compounds of formulae IA and IB from compounds of formulae IIA and IIB. In a particular embodiment, the *in-situ* conversion of the C(O)OR¹⁷ group, and the cyclisation, may be accomplished in a one-pot procedure.
   Compounds of formula XIXA and XIXB may be prepared by reaction of corresponding compounds of formulae XXA and XXB, respectively: 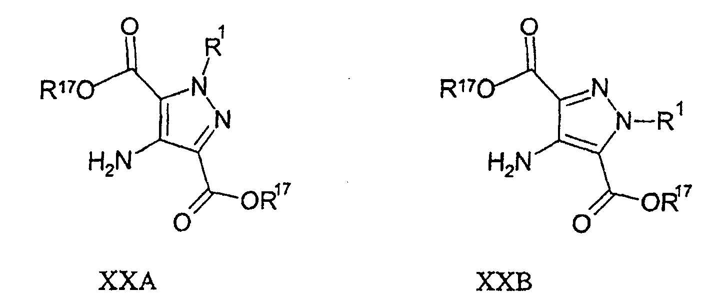 wherein R¹ and R¹⁷ are as previously defined for formulae XIXA and XIXB, with a compound of formula XVIII as defined hereinbefore. The coupling reaction may be performed using analogous conditions to those previously described for compounds of preparation of compounds of formulae IIA and IIB.
   Compounds of formulae XIVA and XIVB, in which R² represents C(O)NR¹²R¹³, may alternatively be prepared by cyclisation of corresponding compounds of formulae XXIA and XXIB, respectively: 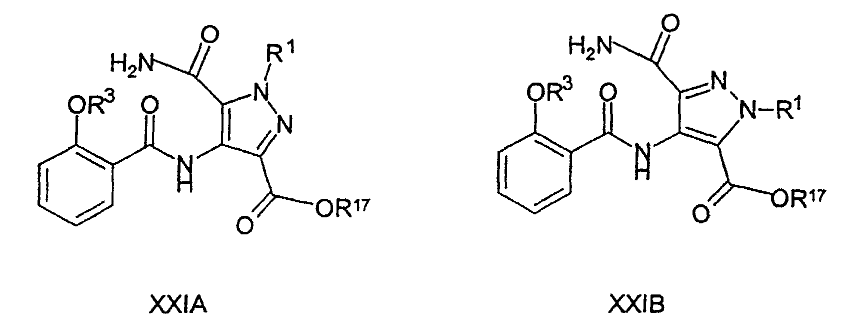 wherein R¹ and R³ are as previously defined for compounds of formulae XIVA and XIVB and R¹⁷ is as previously defined for formulae XVIIA and XVIIB, followed by conversion of the C(O)OR¹⁷ group of the resultant intermediate into an C(O)NR¹²R¹³ group, in which R¹² and R¹³ are as previously defined for compounds of formulae IA and IB.
   The cyclisation may be accomplished using analogous cyclisation techniques to those previously described for formulae IIA and IIB. The conversion of C(O)OR¹⁷ group into C(O)NR¹²R¹³ may be accomplished using techniques which are known to those skilled in the art. Typically, the reaction is accomplished by removal of R¹⁷ and then reacting the resultant acid (or derivative, e.g. alkali metal salt, if formed by the removal reaction) with a compound of formula HNR¹²R¹³, in which R¹² and R¹³ are as previously defined for formulae IA and IB, using analogous amide coupling techniques to those described hereinbefore for compounds of formulae IIA and IIB. It will be appreciated that by an appropriate selection of the protecting group R¹⁷, it may be removed during the reaction of the product formed from the cyclisation of compounds of formulae XXIA and XXIB.
   In a further embodiment, compounds of formulae XIVA and XIVB, in which R² represents C(O)NR¹²R¹³, and R¹² and R¹³ are as defined hereinbefore for compounds of formulae IA and IB, except that they do not represent H, may be prepared from corresponding compounds of formula XIVA and XIVB, in which R² represents C(O)NH₂.
   This conversion may be accomplished using procedures which are known to those skilled in the art. For example, the CONH₂ group may be hydrolysed into the corresponding acid (or acid salt) group, which may then be coupled to a compound of formula HNR¹²R¹³ using analogous amide bond forming techniques to those previously described for compounds of formulae IIA and IIB. Preferably, the hydrolysis is performed under basic conditions e.g. using aqueous sodium hydroxide in ethanol or dioxan, at reflux temperature of the reaction.
5. Compounds of formulae IA and IB in which R¹ represents lower alkyl, alkylHet or alkylaryl may be prepared by alkylation of corresponding compounds of formulae XXIIA and XXIIB, respectively: 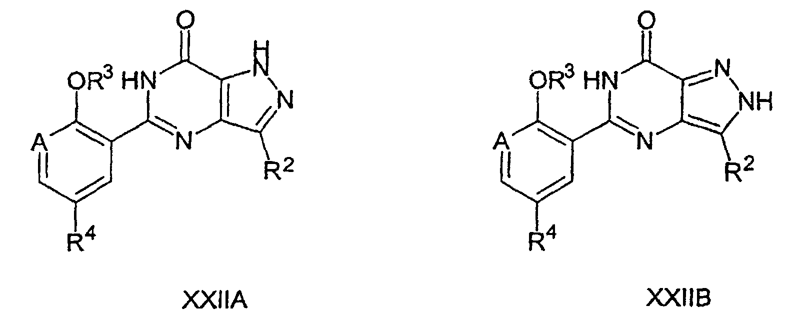 wherein R², R³, R⁴ and A are as previously defined for compounds of formulae IA and IB, for example as described hereinbefore for preparation of compounds of formulae XIVA and XIVB. The skilled person will appreciate that compounds of formulae XXIIA and XXIIB are, respectively, compounds of formulae IA and IB in which R¹ represents H.
6. Compounds of formulae IA and IB, in which R² represents lower alkyl (which alkyl group is branched and unsaturated at the carbon atom that is attached to the rest of the molecule), NR¹²R¹³, cyano, aryl or Het¹ (which Het¹ group is either aromatic or unsaturated at the carbon atom that is attached to the rest of the molecule), may be prepared by cross-coupling of corresponding compounds of formula XXIIIA and XXIIIB: 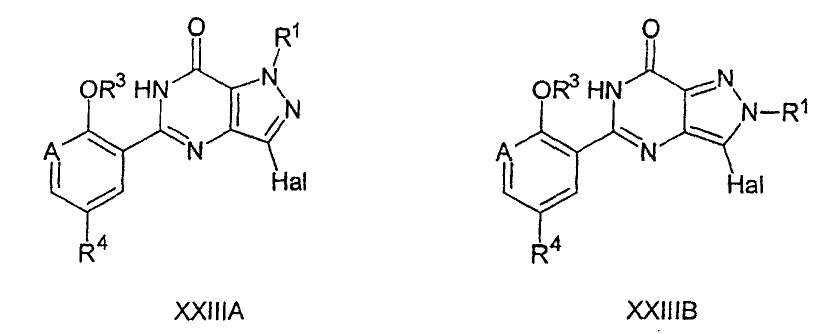 wherein Hal, R¹, R³, R⁴ and A are as hereinbefore defined, using a compound of formula

   R^{2a}M

   wherein R^{2a} represents lower alkyl (which alkyl group is branched and unsaturated at the carbon atom that is attached to M), NR¹²R¹³, cyano, aryl or Het¹ (which Het¹ group is either aromatic or unsaturated at the carbon atom that is attached to M), R¹² and R¹³ are as hereinbefore defined and M represents an optionally substituted metal or boron group, which group is suitable for cross-coupling reactions, for example a trialkylstannane (e.g. tri-*n*-butylstannane), a dialkylborane (e.g. diethylborane), a dialkoxy borane, a dihydroxyborane, lithium, a halomagnesium, a halozinc, copper, a halomercury, in the presence of an appropriate catalyst system (e.g. a palladium or nickel catalyst).
   The cross-coupling reaction is preferably carried out in the presence of a base (e.g. potassium carbonate, cesium fluoride or triethylamine), preferably in excess. Those skilled in the art will appreciate that the type of catalyst that is employed will depend on factors such as the nature of the M group, the substrate that is employed etc.
   Typical procedures that may be employed include those described hereinafter. In a further typical procedure, a compound of formula R^{2a}M may be used, in which M is halozinc. Such a compound may be prepared by reaction of a compound R²Hal, where Hal and R² are as hereinbefore defined, with an alkyllithium (e.g. *n*-butyllithium) at a temperature of between -78°C and room temperature, in a suitable solvent (e.g. THF), and the resultant solution is then treated with Zn(II)Cl₂ (solution in ether) and the resultant solution is treated with a compound of formula XXIIIA or XXIIIB in the presence of a palladium catalyst (e.g. tetrakis(triphenyl)phosphine palladium) in a suitable solvent (e.g. THF). The reaction may be carried out at from room temperature to reflux temperature.
   Suitable coupling conditions also include so-called Suzuki and Stille conditions such as those described hereinbefore in respect of preparation of compounds of formulae XIIIA and XIIIB.
   The skilled person will appreciate that compounds of formulae IA and IB in which R² represents lower alkyl that is branched, but not unsaturated, at the carbon atom that is attached to the rest of the molecule may be prepared by in this way, provided that the corresponding compound of formula IA or IB in which the corresponding R² group is unsaturated is subsequently hydrogenated under conditions known to those skilled in the art.
   Compounds of formulae XXIIIA and XXIIIB may be prepared by cyclisation of corresponding compounds of formulae XXIVA and XXIVB, respectively: 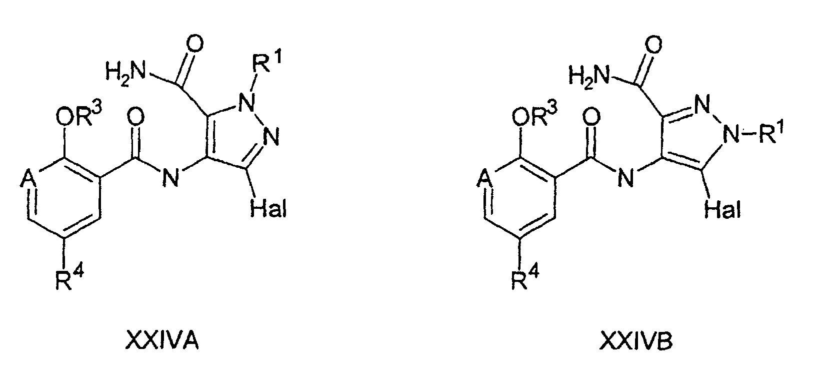 in which R¹, R³, R⁴, A and Hal are as hereinbefore defined, for example under analogous reaction conditions to those described hereinbefore for compounds of formulae IIA and IIB.
   Compounds of formulae XXIVA and XXIVB may be prepared analogously to methods described herein, for example coupling of a compound of formula IV, as hereinbefore defined, to an appropriate 4-amino-3-halopyrazole-5-carboxamide, which pyrazole compound may, in turn, be prepared by halogenation of a corresponding 4-aminopyrazole-5-carboxamide, under conditions which are well known to those skilled in the art.
   Compounds of formulae XXIIIA and XXIIIB may alternatively be prepared from corresponding compounds of formulae XXVA and XXVB, respectively: 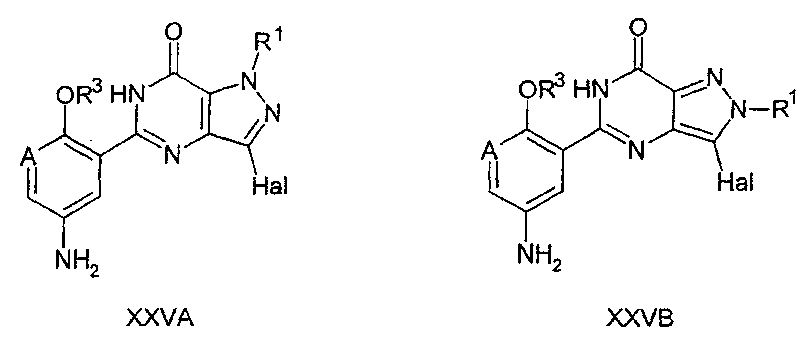 wherein A, Hal, R¹ and R³ are as hereinbefore defined, for example as described hereinbefore for preparation of compounds of formulae IA and IB from compounds of formulae XA and XB (see process 4 above).
   Compounds of formulae XXVA and XXVB may be prepared *via* routine techniques (for example for compounds of formulae XXVA and XXVB in which A represents N, reduction of corresponding nitropyridine compounds of formulae XIIID and XIIIE as defined herein, respectively, for example as described herein).
7. Compounds of formulae IA and IB in which R² represents N(H)C(O)R¹² may be prepared by acylation of a corresponding compound of formula IA or IB in which R² represents NH₂, using a compound of formula XXVI,

   L¹C(O)R¹² XXVI

   in which L¹ and R¹² are as hereinbefore defined under conditions that are known to those skilled in the art.
8. Compounds of formulae IA and IB in which R² represents NR¹²R¹³ in which one of R¹² and R¹³ does not represent H may be prepared by alkylation of a corresponding compound of formula IA or IB in which R² represents NH₂ using an appropriate alkylating agent under conditions that are known to those skilled in the art.
9. Compounds of formulae IA and IB in which R² represents NR¹²R¹³ in which one of R¹² and R¹³ does not represent H may be prepared by reductive amination from a compound of formula IA or IB in which R² represents NH₂, using an appropriate carbonyl compound under conditions that are known to those skilled in the art.
10. Compounds of formulae IA and IB in which R² represents NH₂ may be prepared by reduction of corresponding compounds of formulae XXVIIA or XXVIIB, respectively: 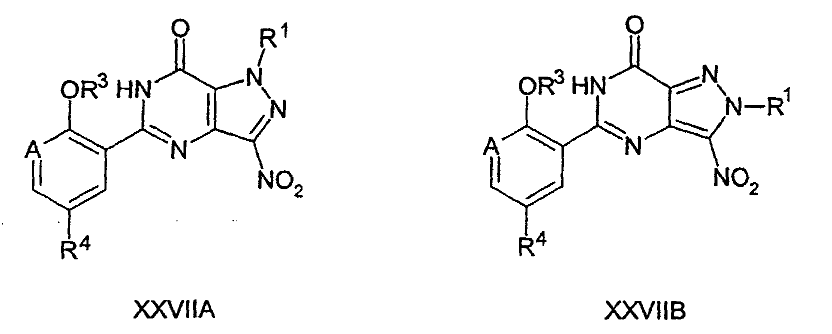 wherein A, R¹, R³ and R⁴ are as hereinbefore defined under conditions that are well known to those skilled in the art.
   Compounds of formulae XXVIIA and XXVIIB may be prepared by nitration of corresponding compounds of formulae XXVIIIA or XXVIIIB, respectively: 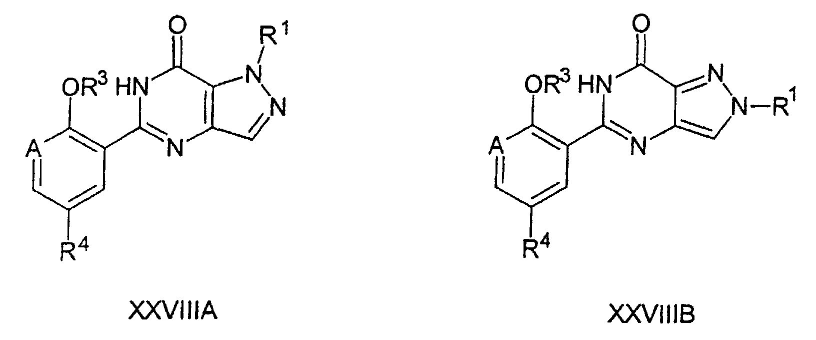 wherein A, R¹, R³ and R⁴ are as hereinbefore defined, using conventional techniques. For example, nitration may be performed at or around room temperature using 1.5 to 3 eq. of ammonium nitrate in the presence of trifluoroacetic anhydride.
   Compounds of formulae XXVIIIA and XXVIIIB may be prepared analogously to methods described herein in respect of the preparation of compounds of formulae IA and IB.
   Compounds of formulae IIIA and IIIB, IV, VIIA and VIIB, IX, XIII, XIIIF and XIIIG, XVA and XVB, XVIIA and XVIIB, XVIII, XXA and XXB, XXIA and XXIB and XXVI, and compounds of formulae HNR¹²R¹³, R^{2a}M, R¹L¹ and R¹OH, and derivatives thereof, when not commercially available or not subsequently described, may be obtained either by analogy with the processes described hereinbefore, or by conventional synthetic procedures, in accordance with standard techniques, from readily available starting materials using appropriate reagents and reaction conditions.

Substituents on aryl and Het/Het¹ groups in the above-mentioned compounds may be introduced, removed and interconverted, using techniques which are well known to those skilled in the art. For example, compounds of formulae IA and IB as described hereinbefore, in which R¹ represents an aryl or alkylaryl group, may be prepared by dehalogenating corresponding compounds of formula IA or IB, in which R¹ represents an aryl or alkylaryl substituted with a halo group, such as a bromo or iodo. The reaction may be performed using methods which are well known to those skilled in the art, for example using a suitable palladium catalyst, such as palladium (0) tetrakis(triphenyl)phosphine, a suitable hydrogen donor (e.g. sodium formate), and a suitable base (e.g. triethylamine), in a suitable solvent (e.g. acetonitrile and/or dimethylsulphoxide).

The skilled person will also appreciate that various standard substituent or functional group interconversions and transformations within certain compounds of formulae IA and IB will provide other compounds of formulae IA and IB. For example, alkoxide exchange at the 2-position of the 5-phenyl and the pyridin-3-yl substituents. Moreover, certain compounds of formulae IA and IB, for example those in which R¹⁴ and R¹⁵, together with the nitrogen to which they are attached, form a 4-R¹⁶-piperazinyl group, in which R¹⁶ does not represent H, may be prepared directly from the corresponding piperazine analogues in which R¹⁶ represents H, using standard procedures (e.g. alkylation).

The compounds of the invention may be isolated from their reaction mixtures using conventional techniques.

It will be appreciated by those skilled in the art that, in the course of carrying out the processes described above, the functional groups of intermediate compounds may need to be protected by protecting groups.

Functional groups which it is desirable to protect include hydroxy, amino and carboxylic acid. Suitable protecting groups for hydroxy include trialkylsilyl and diarylalkylsilyl groups (e.g. *tert*-butyldimethylsilyl, *tert*-butyldiphenylsilyl or trimethylsilyl) and tetrahydropyranyl. Suitable protecting groups for amino include *tert*-butyloxycarbonyl, 9-fluorenylmethoxycarbonyl or benzyloxycarbonyl. Suitable protecting groups for carboxylic acid include C₁₋₆ alkyl or benzyl esters.

The protection and deprotection of functional groups may take place before or after any of the reaction steps described hereinbefore.

Protecting groups may be removed in accordance with techniques which are well known to those skilled in the art.

The use of protecting groups is fully described in "Protective Groups in Organic Chemistry", edited by JWF McOmie, Plenum Press (1973), and "Protective Groups in Organic Synthesis", 2^{nd} edition, TW Greene & PGM Wutz, Wiley-Interscience (1991).

Persons skilled in the art will also appreciate that, in order to obtain compounds of formula I in an alternative, and, on some occasions, more convenient, manner, the individual process steps mentioned hereinbefore may be performed in a different order, and/or the individual reactions may be performed at a different stage in the overall route (i.e. substituents may be added to and/or chemical transformations performed upon, different intermediates to those mentioned hereinbefore in conjunction with a particular reaction). This will depend *inter alia* on factors such as the nature of other functional groups present in a particular substrate, the availability of key intermediates and the protecting group strategy (if any) to be adopted. Clearly, the type of chemistry involved will influence the choice of reagent that is used in the said synthetic steps, the need, and type, of protecting groups that are employed, and the sequence for accomplishing the synthesis.

Pharmaceutically acceptable acid addition salts of the compounds of formulae IA and IB which contain a basic centre may be prepared in a conventional manner. For example, a solution of the free base may be treated with the appropriate acid, either neat or in a suitable solvent, and the resulting salt may then be isolated either by filtration or by evaporation under vacuum of the reaction solvent. Pharmaceutically acceptable base addition salts can be obtained in an analogous manner by treating a solution of a compound of formula IA or IB with the appropriate base. Both types of salt may be formed or interconverted using ion-exchange resin techniques.

It will be appreciated by those skilled in the art that certain protected derivatives of compounds of formula I, which may be made prior to a final deprotection stage, may not possess pharmacological activity as such, but may, in certain instances, be administered orally or parenterally and thereafter metabolised in the body to form compounds of the invention which are pharmacologically active. Such derivatives may therefore be described as "prodrugs". Further, certain compounds of formula I may act as prodrugs of other compounds of formula I.

All protected derivatives, and prodrugs, of compounds of formula I are included within the scope of the invention.

### Medical Use

The compounds of the invention are useful because they possess pharmacological activity in animals, especially mammals, including humans. They are therefore indicated as pharmaceuticals, as well as for use as animal medicaments.

According to a further aspect of the invention there is provided the compounds of the invention for use as pharmaceuticals, and for use as animal medicaments.

In particular, compounds of the invention have been found to be potent and selective inhibitors of cGMP PDEs, such as cGMP PDE5, for example as demonstrated in the tests described below, and are thus useful in the treatment of medical conditions in humans, and in animals, in which cGMP PDEs, such as cGMP PDE5, are indicated, and in which inhibition of cGMP PDEs, such as cGMP PDE5, is desirable.

By the term "treatment", we include both therapeutic (curative) or prophylactic treatment.

Thus, according to a further aspect of the invention there is provided the use of the compounds of the invention in the manufacture of a medicament for the treatment of a medical condition in which a cGMP PDE (e.g. cGMP PDE5) is indicated. There is further provided the use of the compounds of the invention in the manufacture of a medicament for the treatment of a medical condition in which inhibition of a cGMP PDE (e.g. cGMP PDE5) is desirable.

The compounds of the invention are thus expected to be useful for the curative or prophylactic treatment of male erectile dysfunction (MED), female sexual dysfunction (FSD), premature labour, dysmenorrhoea, benign prostatic hyperplasia (BPH), bladder outlet obstruction, incontinence, stable and unstable variant (Prinzmetal) angina, hypertension, pulmonary hypertension, congestive heart failure, atherosclerosis, stroke, peripheral vascular disease, conditions of reduced blood vessel patency (e.g. post transluminal coronary angioplasty (post-PTCA)), chronic asthma, bronchitis, allergic asthma, allergic rhinitis, glaucoma and diseases characterised by disorders of gut motility (e.g. irritable bowel syndrome (IBS)). Other conditions which may be mentioned include pre-eclampsia, Kawasaki's syndrome, nitrate tolerance, multiple sclerosis, peripheral diabetic neuropathy, stroke, Alzheimer's disease, acute respiratory failure, psoriasis, skin necrosis, cancer metastasis, baldness, nutcracker oesophagus, anal fissure and hypoxic vasoconstriction. Particularly preferred conditions include MED and FSD.

### Pharmaceutical Preparations

The compounds of the invention will normally be administered orally or by any parenteral route, in the form of pharmaceutical preparations comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

The compounds of the invention may also be combined with any other drugs useful in the inhibition of cGMP-PDEs, such as cGMP-PDE5.

In human therapy, the compounds of the invention can be administered alone but will generally be administered in admixture with a suitable pharmaceutical excipient diluent or carrier selected with regard to the intended route of administration and standard pharmaceutical practice.

For example, the compounds of the invention can be administered orally, buccally or sublingually in the form of tablets, capsules, ovules, elixirs, solutions or suspensions, which may contain flavouring or colouring agents, for immediate-, delayed- or controlled-release applications. The compounds of invention may also be administered *via* intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds of the invention can also be administered parenterally, for example, intravenously, intra-arterially, intraperitoneally, intrathecally, intraventricularly, intrasternally, intracranially, intramuscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the invention will usually be from 10 to 500 mg/kg (in single or divided doses).

Thus, for example, the tablets or capsules of the compound of the invention may contain from 5 mg to 250 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The compounds of the invention can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A™ or 1,1,1,2,3,3,3-heptafluoropropane (HFA 227EA™), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or "puff" contains from 1 to 50 mg of a compound of the invention for delivery to the patient. The overall daily dose with an aerosol will be in the range of from 1 to 50 mg, which may be administered in a single dose or, more usually, in divided doses throughout the day.

Alternatively, the compounds of the invention can be administered in the form of a suppository or pessary, or they may be applied topically in the form of a lotion, solution, cream, ointment or dusting powder. The compounds of the invention may also be transdermally administered, for example, by the use of a skin patch. They may also be administered by the ocular route, particularly for treating diseases of the eye.

For ophthalmic use, the compounds of the invention can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The skilled person will also appreciate that, in the treatment of certain conditions (including MED and FSD), compounds of the invention may be taken as a single dose on an "as required" basis (i.e. as needed or desired).

Generally, in humans, oral administration of the compounds of the invention is the preferred route, being the most convenient and, for example in MED, in avoiding the well-known disadvantages associated with intracavernosal (i.c.) administration.

A preferred oral dosing regimen in MED for a typical man is from 25 to 250 mg of compound when required.

In circumstances where the recipient suffers from a swallowing disorder or from impairment of drug absorption after oral administration, the drug may be administered parenterally, e.g. sublingually or bucally.

For veterinary use, a compound of the invention is administered as a suitably acceptable formulation in accordance with normal veterinary practice and the veterinary surgeon will determine the dosing regimen and route of administration which will be most appropriate for a particular animal.

Thus, according to a further aspect of the invention there is provided a pharmaceutical formulation including a compound of the invention in admixture with a pharmaceutically or veterinarily acceptable adjuvant, diluent or carrier.

In addition to the fact that compounds of the invention inhibit cyclic guanosine 3',5'-monophosphate phosphodiesterases (cGMP PDEs) and in particular, are potent and selective inhibitors of cGMP PDE5, compounds of the invention may also have the advantage that they may be more efficacious than, be less toxic than, have a broader range of activity than, be more potent than, produce fewer side effects than, be more easily absorbed than, or they may have other useful pharmacological properties over, compounds known in the prior art.

The biological activities of the compounds of the present invention were determined by the following test methods.

### Biological Tests

### Phosphodiesterase (PDE) Inhibitory Activity

*In vitro* PDE inhibitory activities against cyclic guanosine 3',5'-monophosphate (cGMP) and cyclic adenosine 3',5'-monophosphate (cAMP) phosphodiesterases were determined by measurement of their IC₅₀ values (the concentration of compound required for 50% inhibition of enzyme activity).

The required PDE enzymes were isolated from a variety of sources, including human corpus cavernosum, human and rabbit platelets, human cardiac ventricle, human skeletal muscle and bovine retina, essentially by the method of W.J. Thompson and M.M. Appleman (Biochem., 1971, 10, 311). In particular, the cGMP-specific PDE (PDE5) and the cGMP-inhibited cAMP PDE (PDE3) were obtained from human corpus cavernosum tissue, human platelets or rabbit platelets; the cGMP-stimulated PDE (PDE2) was obtained from human corpus cavernosum; the calcium/calmodulin (Ca/CAM)-dependent PDE (PDE1) from human cardiac ventricle; the cAMP-specific PDE (PDE4) from human skeletal muscle; and the photoreceptor PDE (PDE6) from bovine retina.

Assays were performed using a modification of the "batch" method of W.J. Thompson et al. (Biochem., 1979, 18, 5228). Results from these tests show that the compounds of the present invention are potent and selective inhibitors of cGMP-specific PDE5.

### Functional Activity

This was assessed *in vitro* by determining the capacity of a compound of the invention to enhance sodium nitroprusside-induced relaxation of precontracted rabbit corpus cavernosum tissue strips, as described by S.A. Ballard et al. (Brit. J. Pharmacol., 1996, 118 (suppl.), abstract 153P).

### In Vivo Activity

Compounds may be screened in anaesthetised dogs to determine their capacity, after i.v. administration, to enhance the pressure rises in the corpora cavernosa of the penis induced by intracavernosal injection of sodium nitroprusside, using a method based on that described by Trigo-Rocha et al. (Neurourol, and Urodyn., 1994, 13, 71).

### Safety Profile

Compounds of the invention may be tested at varying i.v and p.o. doses in animals such as mouse and dog, observing for any untoward effects.

### Examples and Preparations

The synthesis of the compounds of the invention and of the intermediates for use therein are illustrated by the following Examples and Preparations.

¹H nuclear magnetic resonance (NMR) spectra were recorded using either a Varian Unity 300 or a Varian Inova 400 spectrometer and were in all cases consistent with the proposed structures. Characteristic chemical shifts (δ) are given in parts-per-million downfield from tetramethylsilane using conventional abbreviations for designation of major peaks: eg s, singlet; d, doublet; t, triplet; q, quartet; m, multiplet; br, broad.

Mass spectra (m/z) were recorded using a Fisons Instruments Trio mass spectrometer in the thermospray ionisation mode.

Room temperature includes 20 to 25°C.

### Synthesis of Intermediates

### Preparation 1

### 4-Nitro-1H-pyrazole-3,5-dicarboxylic acid

Fuming sulphuric acid (105ml) was added dropwise over 45 minutes to ice-cooled fuming nitric acid (88ml), so as to maintain the internal temperature below 20°C. Once addition was complete the mixture was warmed to 40°C, pyrazole-3,5-dicarboxylic acid (125g, 0.80mol) added portionwise over 75 minutes, so as to maintain the reaction temperature below 50°C, and the reaction then stirred at 60°C for 18 hours. The cooled mixture was poured onto ice (1kg), and flaked potassium hydroxide carefully added with stirring, until the solution pH was 2. The resulting precipitate was filtered, and triturated with boiling water (500ml), to afford the title compound (123g, 76%) as a white solid.
m.p. 325-327°C.

### Preparation 2

### 4-Nitro-1H-pyrazole-3,5-dicarboxylic acid dimethyl ester

Thionyl chloride (290ml, 3.98mol) was added dropwise over 2 hours, to an ice cooled suspension of the title compound of Preparation 1 (123g, 0.61mol) in dry methanol (1200ml), and the reaction stirred under reflux for 48 hours. The cooled mixture was concentrated under reduced pressure, partitioned between water (500ml) and dichloromethane (500ml), and filtered. The phases were separated, the aqueous layer extracted with dichloromethane (4x250ml), the combined organic solutions dried (Na₂SO₄), and evaporated under reduced pressure to afford the title compound (74.6g, 53%) as a white solid.
Found : C, 36.39; H, 2.98; N, 18.15. C₇H₇N₃O₆ requires C, 36.69; H, 3.08; N, 18.34%.
δ (CDCl₃) : 4.00 (6H, s).
LRMS : m/z 247 (M+18)⁺

### Preparation 3

### 4-Nitro-1-(pyridin-2-yl)methyl-1H-pyrazole-3,5-dicarboxylic acid dimethyl ester

Cesium carbonate (14.22g, 43.6mmol) was added to a solution of the title compound of Preparation 2 (10.0g, 43.6mmol) in dimethylformamide (100ml), and the mixture stirred at room temperature for 30 minutes. 2-(Chloromethyl)pyridine hydrochloride (7.16g, 43.6mmol) was added and the reaction stirred at room temperature for a further 22 hours. The reaction mixture was concentrated under reduced pressure, the residue partitioned between dichloromethane (150ml) and water (70ml), and the layers separated. The aqueous phase was extracted with dichloromethane (2x100ml), the combined organic extracts dried (MgSO₄), and evaporated under reduced pressure. The residual brown solid was purified by column chromatography on silica gel, using an elution gradient of ethyl acetate:pentane (20:80 to 50:50) to afford the title compound, (6.34g, 45%) as a white solid.
δ (CDCl₃) : 3.88 (3H, s), 3.96 (3H, s), 5.93 (2H, s), 7.15 (1H, d), 7.21 (1H, m), 7.66 (1H, m), 8.52 (1H, d).
LRMS : m/z 321 (M+1)⁺

### Preparation 4

### 4-Nitro-1-(pyridin-3-yl)methyl-1H-pyrazole-3,5-dicarboxylic acid dimethyl ester

A mixture of the title compound of Preparation 2 (4.0g, 17mmol), and cesium carbonate (2.86g, 19mmol) in dimethylformamide (100ml) was stirred at room temperature for 45 minutes, 3-(chloromethyl)pyridine hydrochloride (6.26g, 19mmol) added and stirring continued for a further 18 hours. The reaction mixture was concentrated under reduced pressure, and the residue partitioned between ethyl acetate (50ml) and water (50ml). The phases were separated, the aqueous layer extracted with ethyl acetate (2x50ml) and the combined organic extracts dried (Na₂SO₄) and evaporated under reduced pressure. The residual brown oil was purified by column chromatography on silica gel, using an elution gradient of dichloromethane:methanol (100:0 to 98:2) to afford the title compound (2.40g, 45%) as a white solid.
δ (CDCl₃) : 3.92 (3H, s), 3.97 (3H, s), 5.82 (2H, s), 7.29 (1H, m), 7.70 (1H, d), 8.60 (1H, d), 8.69 (1H, s).
LRMS : m/z 321 (M+1)⁺

### Preparation 5

### 4-Nitro-1-(pyridin-4-yl)methyl-1H-pyrazole-3,5-dicarboxylic acid dimethyl ester

Obtained as a white solid (64%) from the title compound of Preparation 2 and 4-(chloromethyl)pyridine hydrochloride using the procedure of Preparation 4.
δ (CDCl₃) : 3.90 (3H, s), 3.98 (3H, s), 5.80 (2H, s), 7.18 (2H, d), 8.62 (2H, d).

### Preparation 6

### 1-Benzyl-4-nitro-1H-pyrazole-3,5-dicarboxylic acid dimethyl ester

A mixture of the title compound of Preparation 2 (3.10g, 13.5mmol), cesium carbonate (2.20g, 6.75mmol) and benzyl bromide (1.6ml, 13.5mmol) in dimethylformamide (40ml) was stirred at room temperature for 72 hours. The reaction mixture was concentrated under reduced pressure and the residue partitioned between water (50ml) and ethyl acetate (50ml). The phases were separated, the aqueous layer extracted with ethyl acetate (2x50ml) and the combined organic extracts dried (Na₂SO₄) and evaporated under reduced pressure to afford the title compound (4.35g, 99%) as a colourless oil.
δ (CDCl₃) : 3.87 (3H, s), 3.96 (3H, s), 5.78 (2H, s), 7.34 (5H, s).

### Preparation 7

### 1-(4-Bromobenzyl)-4-nitro-1H-pyrazole-3,5-dicarboxylic acid dimethyl ester

A mixture of the title compound of Preparation 2 (5.04g, 22.0mmol), cesium carbonate (7.88g, 24.0mmol), and 4-bromobenzyl bromide (5.75g, 24.0mmol) in dimethylformamide (100ml) was stirred for 18 hours at room temperature. The reaction mixture was concentrated under reduced pressure, the residue partitioned between water (50ml) and ethyl acetate (75ml) and the phases separated. The aqueous phase was extracted with dichloromethane (3x50ml), and the combined organic extracts dried (Na₂SO₄) and evaporated under reduced pressure. The residual yellow solid was triturated with ethanol to afford the title compound, (6.60g, 75%).
δ (CDCl₃) : 3.90 (3H, s), 3.97 (3H, s), 5.74 (2H, s), 7.24 (2H, d), 7.48 (2H, d).
LRMS : m/z 415 (M+18)⁺

### Preparation 8

### 1-Methyl-4-nitro-1H-pyrazole-3,5-dicarboxylic acid dimethyl ester

Obtained as an off-white solid after trituration with hexane (93%), from dimethyl sulphate and the title compound of Preparation 2, using the procedure of Preparation 7.
δ (CDCl₃) : 3.95 (6H, 2xs), 4.26 (3H, s).
LRMS : m/z 261 (M+18)⁺

### Preparation 9

### 1-Cyclobutylmethyl-4-nitro-1H-pyrazole-3,5-dicarboxylic acid dimethyl ester

Diethylazodicarboxylate (3.78ml, 24.0mmol) was added dropwise to an ice-cooled solution of cyclobutanemethanol (2.06ml, 21.8mmol), the title compound of Preparation 2 (5.0g, 21.8mmol) and triphenylphosphine (6.30g, 24.0mmol) in tetrahydrofuran (50ml) and the reaction stirred for a further 2 hours at 0°C. The reaction mixture was concentrated under reduced pressure and the residue purified by column chromatography on silica gel, using an elution gradient of ethyl acetate:hexane (15:85 to 20:80) to afford the title compound (6.23g, 96%) as a colourless oil.
δ (CDCl₃) : 1.80-1.95 (4H, m), 2.05 (2H, m), 2.90 (1H, m), 3.96 (6H, 2xs), 4.63 (2H, d).
LRMS : m/z 315 (M+18)⁺

### Preparation 10

### 1-[2-(4-Morpholinyl)ethyl]-4-nitro-1H-pyrazole-3,5-dicarboxylic acid dimethyl ester

Obtained as a solid (15%) from 4-(2-chloroethyl)morpholine hydrochloride and the title compound of Preparation 2, using the procedure of Preparation 3.
δ (CDCl₃) : 2.46 (4H, m), 2.78 (2H, t), 3.61 (4H, m), 3.95 (6H, 2xs), 4.73 (2H, t).
LRMS : m/z 343 (M+1)⁺

### Preparation 11

### 4-Amino-1-(pyridin-2-yl)methyl-1H-pyrazole-3,5-dicarboxylic acid dimethyl ester

A mixture of the title compound of Preparation 3 (1.0g, 3.12mmol) and Raney nickel (800mg) in methanol (50ml) was hydrogenated at 50°C and 345kPa (50psi) for 18 hours, then cooled and filtered. The filtrate was combined with a methanol wash of the filter pad, and concentrated under reduced pressure. The residue was azeotroped with dichloromethane and dried under vacuum to afford the title compound, (895mg, 99%).
δ (DMSOd₆) : 3.74 (3H, s), 3.80 (3H, s), 5.62 (2H, s), 5.74 (2H, s), 6.98 (1H, d), 7.26 (1H, m), 7.74 (1H, m), 8.45 (1H, d).
LRMS : m/z 291 (M+1)⁺

### Preparation 12

### 4-Amino-1-(pyridin-3-yl)methyl-1H-pyrazole-3,5-dicarboxylic acid dimethyl ester

Tin (II) chloride dihydrate (9.30g, 41.2mmol) was added to a suspension of the title compound of Preparation 4 (2.40g, 7.50mmol) in ethanol (20ml) and the reaction stirred at 70°C for 18 hours. The cooled reaction mixture was concentrated under reduced pressure and the residue stirred vigorously in a mixture of ethyl acetate (30ml) and dilute sodium carbonate solution (30ml) for an hour. The phases were separated, the aqueous layer extracted with ethyl acetate (2x25ml), the combined organic solutions dried (Na₂SO₄) and evaporated under reduced pressure, to afford the title compound (1.77g, 80%) as a white solid.
δ (CDCl₃) : 3.86 (3H, s), 3.96 (3H, s), 5.37 (2H, s), 5.70 (2H, s), 7.22 (1H, m), 7.54 (1H, d), 8.52 (1H, d), 8.56 (1H, s).

### Preparation 13

### 4-Amino-1-(pyridin-4-yl)methyl-1H-pyrazole-3,5-dicarboxylic acid dimethyl ester

Obtained (96%) from the title compound of Preparation 5, using the procedure of Preparation 11.
δ (CDCl₃) : 3.84 (3H, s), 3.96 (3H, s), 5.39 (2H, s), 5.70 (2H, s), 7.04 (2H, m), 8.55 (2H, m).

### Preparation 14

### 4-Amino-1-(4-bromobenzyl)-1H-pyrazole-3,5-dicarboxylic acid dimethyl ester

Obtained as a white solid after recrystallisation from isopropyl acetate (74%), from the title compound of Preparation 7, using the procedure of Preparation 12.
δ (CDCl₃) : 3.86 (3H, s), 3.97 (3H, s), 5.36 (2H, s), 5.64 (2H, s), 7.10 (2H, d), 7.42 (2H, d).
LRMS : m/z 369 (M+1)⁺

### Preparation 15

### 4-Amino-1-cyclobutylmethyl-1H-pyrazole-3,5-dicarboxylic acid dimethyl ester

A mixture of the title compound of Preparation 9 (6.23g, 21.0mmol) and 10% palladium on charcoal (800mg) in methanol (150ml) was hydrogenated at 345kPa (50psi) and 50°C for 18 hours. The cooled mixture was filtered, the filter pad washed with methanol (150ml) and the filtrate evaporated under reduced pressure to afford the title compound (5.50g, 98%) as a white solid.
δ (CDCl₃) : 1.78-1.92 (4H, m), 1.99 (2H, m), 2.83 (1H, m), 3.94 (6H, 2x s), 4.55 (2H, d), 5.35 (2H, s).
LRMS : m/z 268 (M+1)⁺

### Preparation 16

### 4-Amino-1-[2-(4-morpholinyl)ethyl]-1H-pyrazole-3,5-dicarboxylic acid dimethyl ester

Obtained as a brown solid (95%) from the title compound of Preparation 10, using the procedure of Preparation 11.
δ (DMSOd₆) : 2.40 (4H, m), 2.62 (2H, t), 3.50 (4H, m), 3.79 (3H, s), 3.81 (3H, s), 4.50 (2H, t), 5.58 (2H, s).

### Preparation 17

### 4-Amino-1H-pyrazole-3,5-dicarboxylic acid dimethyl ester

Obtained as a white solid (91%) from the title compound of Preparation 2, using the procedure of Preparation 11.
δ (DMSOd₆) : 3.80 (6H, s), 5.41 (2H, s), 13.83 (1H, s).
LRMS : m/z 217 (M+1)⁺

### Preparation 18

### Dimethyl 4-(2-n-propoxybenzamido)-1-(pyridin-2-yl)methyl-1H-pyrazole-3,5-dicarboxylate

A solution of the title compound of Preparation 11 (1.56g, 7.84mmol) in dichloromethane (5ml) was added slowly to a solution of 2-n-propoxybenzoyl chloride (1.56g, 7.84mmol) in pyridine (10ml) and the reaction stirred at room temperature for 18 hours. The reaction mixture was concentrated under reduced pressure, azeotroped with toluene and the residual brown oil partitioned between dichloromethane (10ml) and saturated sodium bicarbonate solution (15ml). The phases were separated, the aqueous layer extracted with dichloromethane (3x10ml), and the combined organic extracts washed with aqueous copper (II) sulphate solution (2x10ml), dried (Na₂SO₄) and evaporated under reduced pressure. The residue was purified by column chromatography on silica gel, using an elution gradient of dichloromethane:methanol (100:0 to 98:2) to afford the title compound (1.80g, 51%) as a yellow foam.
δ (CDCl₃) : 1.06 (3H, t), 2.03 (2H, m), 3.78 (3H, s), 3.95 (3H, s), 4.25 (2H, t), 5.85 (2H, s), 6.95-7.08 (3H, m), 7.18 (1H, m), 7.48 (1H, m), 7.62 (1H, m), 8.24 (1H, d), 8.54 (1H, d), 10.69 (1H, s).
LRMS : m/z 453 (M+1)⁺

### Preparations 19 to 23

The compounds of the following tabulated Preparations of the general formula: 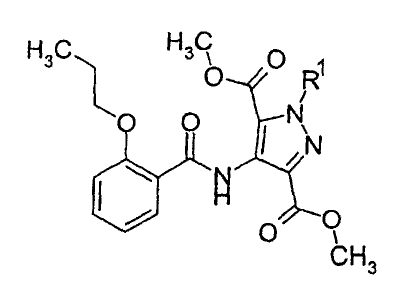 were prepared by the reaction of 2-n-propoxybenzoyl chloride and the corresponding aminopyrazoles, using similar methods to that described in Preparation 18.

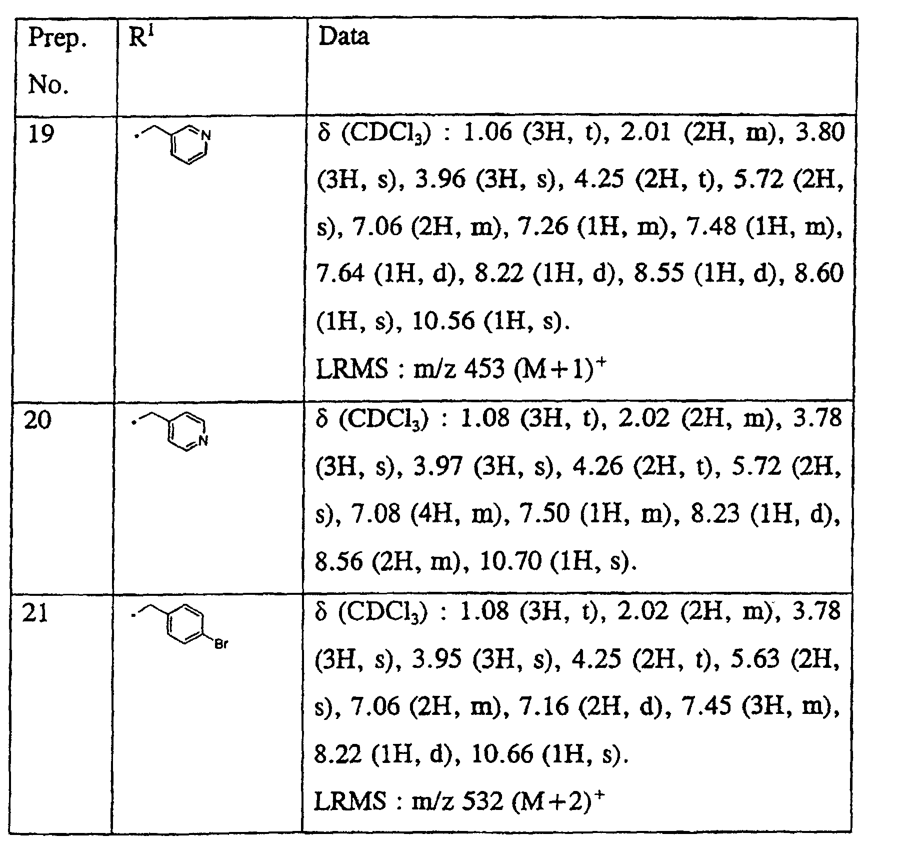

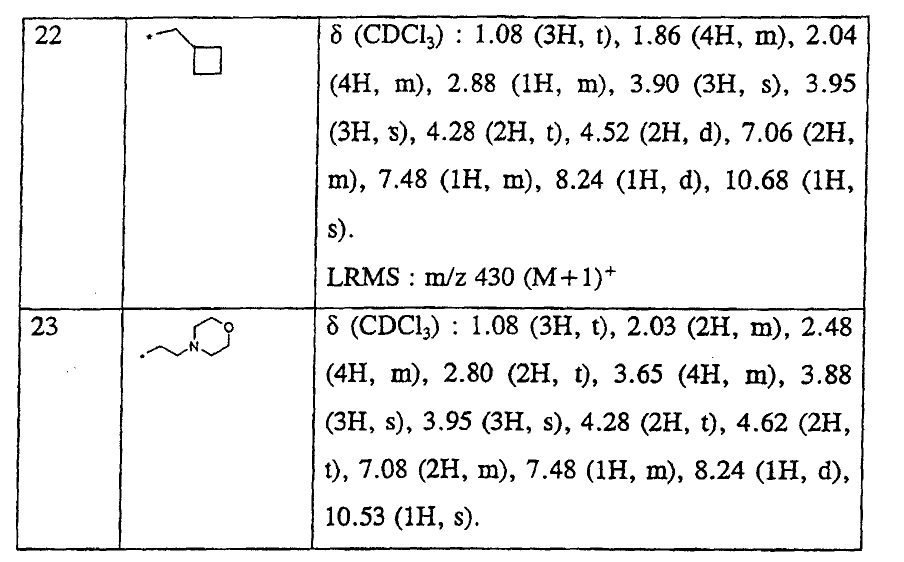

### Preparation 24

### Dimethyl 4-(2-n-propoxybenzamido)-1H-pyrazole-3,5-dicarboxylate

A solution of 2-n-propoxybenzoyl chloride (3.99g, 20.0mmol) in dichloromethane (10ml) was added dropwise to a solution of the title compound of Preparation 17 (4.0g, 20mmol) in pyridine (50ml) and the reaction stirred at room temperature for 18 hours. The reaction mixture was concentrated under reduced pressure, the residue partitioned between water (30ml) and ethyl acetate (100ml) and the layers separated. The organic layer was washed with water (30ml), 1N hydrochloric acid (4x50ml), dried (MgSO₄) and evaporated under reduced pressure. The residue was purified by column chromatography on silica gel, using an elution gradient of ethyl acetate:pentane (50:50 to 100:0) to afford the title compound, (2.04g, 28%) as a white solid.
δ (DMSOd₆) : 0.98 (3H, t), 1.89 (2H, m), 3.78 (3H, s), 3.81 (3H, s), 4.24 (2H, t), 7.10 (1H, m), 7.25 (1H, d), 7.57 (1H, m), 7.99 (1H, d), 10.28 (1H, s), 14.51 (1H, s).
LRMS : m/z 362 (M+1)⁺

### Preparation 25

### 4-(2-n-Propoxybenzamido)-1H-pyrazole-3,5-dicarboxamide

Liquid ammonia (15ml) was added carefully to a cooled (-75°C) solution of the title compound of Preparation 24 (2.01g, 5.56mmol) in methanol (25ml) and the reaction heated at 100°C in a sealed vessel for 18 hours. The cooled reaction mixture was concentrated under reduced pressure and azeotroped with dichloromethane to afford the title compound (1.62g, 93%) as a white solid.
δ (DMSOd₆) : 0.98 (3H, t), 1.90 (2H, m), 4.19 (2H, t), 7.08 (1H, m), 7.22 (1H, d), 7.55 (5H, m), 7.97 (1H, d), 10.56 (1H, s).
LRMS : m/z 332 (M+1)⁺

### Preparation 26

### 3-Carboxamido-5-(2-n-propoxyphenyl)-1-(pyridin-2-yl)methyl-1,6-dihydro-7H-pyrazolo[4,3-d]pyrimidin-7-one

An ice-cooled solution of the title compound of Preparation 18 (3.50g, 7.74mmol) in methanol (300ml) was saturated with ammonia, then heated to 100°C in a sealed vessel for 72 hours. The cooled mixture was concentrated under reduced pressure, and the residue triturated with diethyl ether, then a solution of dichloromethane:methanol (90:10), to give the title compound (1.0g) as a white solid. The filtrate was concentrated under reduced pressure and the residue purified by column chromatography on silica gel, using dichloromethane:methanol (98:2) as eluant to afford a further 780mg, of the title compound.
δ (CDCl₃) : 1.18 (3H, t), 2.00 (2H, m), 4.21 (2H, t), 6.06 (3H, m), 7.06-7.20 (4H, m), 7.54 (1H, m), 7.62 (1H, m), 8.16 (1H, m), 8.43 (1H, d), 8.56 (1H, d).
LRMS : m/z 405 (M+1)⁺

### Preparation 27

### 3-Carboxamido-5-(2-n-propoxyphenyl)-1-(pyridin-3-yl)methyl-1,6-dihydro-7H-pyrazolo[4,3-d]pyrimidin-7-one

An ice-cooled solution of the title compound of Preparation 19 (2.02g, 4.47mmol) in methanol (80ml) was saturated with ammonia and the reaction heated at 100°C in a sealed vessel for 18 hours. The cooled reaction mixture was evaporated under reduced pressure to give a white solid. Potassium t-butoxide (1.40g, 12.43mmol) was added to a suspension of this product in isopropanol (30ml), and the reaction heated under reflux for 8 hours, then cooled. Water (60ml) was added, the mixture neutralised with 2N hydrochloric acid and the resulting precipitate filtered, washed with water and dried under suction to afford the title compound (1.20g, 66%) as a white solid.
δ (CDCl₃) : 1.20 (3H, t), 2.04 (2H, m), 4.22 (2H, t), 5.92 (2H, s), 6.05 (1H, s), 7.10 (1H, d), 7.17 (1H, m), 7.26 (1H, m), 7.54 (1H, m), 7.86 (1H, d), 8.15 (1H, s), 8.40 (1H, d), 8.56 (1H, d), 8.80 (1H, s), 11.49 (1H, s).
LRMS : m/z 405 (M+1)⁺

### Preparation 28

### 3-Carboxamido-5-(2-n-propoxyphenyl)-1-(pyridin-4-yl)methyl-1,6-dihydro-7H-pyrazolo[4,3-d]pyrimidin-7-one

An ice-cooled solution of the title compound of Preparation 20 (1.30g, 2.88mmol) in methanol (100ml) was saturated with ammonia and the reaction heated at 100°C for 24 hours. The cooled reaction mixture was evaporated under reduced pressure and the residue suspended in isopropanol (100ml). Potassium t-butoxide (1.7g, 15.1mmol) was added and the reaction heated under reflux for 5 hours, then cooled. Water (100ml) was added, the mixture neutralised with 2N hydrochloric acid and extracted with dichloromethane (2x100ml). The combined organic extracts were dried (Na₂SO₄), concentrated under reduced pressure and the residue purified by column chromatography on silica gel, using an elution gradient of dichloromethane:methanol (98:2 to 94:6) to afford the title compound (550mg, 47%).
δ (CDCl₃) : 1.19 (3H, t), 2.03 (2H, m), 4.22 (2H, t), 5.88 (2H, s), 6.05 (1H, s), 7.10 (1H, d), 7.17 (1H, m), 7.35 (2H, d), 7.54 (1H, m), 8.16 (1H, s), 8.40 (1H, d), 8.57 (2H, d), 11.50 (1H, s).

### Preparation 29

### 1-(4-Bromobenzyl)-3-carboxamido-5-(2-n-propoxyphenyl)-1,6-dihydro-7H-pyrazolo[4,3-d]pyrimidin-7-one

Obtained (88%) from the title compound of Preparation 21, using a similar procedure to that described in Preparation 28.
δ (DMSOd₆) : 0.94 (3H, t), 1.72 (2H, m), 4.05 (2H, t), 5.78 (2H, s),
7.08 (1H, m), 7.18 (1H, d), 7.26 (2H, d), 7.52 (3H, m), 7.76 (2H, m).

### Preparation 30

### 3-Carboxamido-1-cyclobutylmethyl-5-(2-n-propoxyphenyl)-1,6-dihydro-7H-pyrazolo[4,3-d]pyrimidin-7-one

Obtained as an off-white solid (56%) from the title compound of Preparation 22, using the procedure of Preparation 27.
δ (CDCl₃) : 1.11 (3H, t), 1.93 (4H, m), 2.04 (4H, m), 3.07 (1H, m), 4.24 (2H, t), 4.75 (2H, d), 5.93 (1H, s), 7.10 (1H, d), 7.18 (1H, m), 7.55 (1H, m), 8.17 (1H, s), 8.42 (1H, d), 11.44 (1H, s).
LRMS : m/z 382 (M+1)⁺

### Preparation 31

### 3-Carboxamido-1-[2-(4-morpholinyl)ethyl]-5-(2-n-propoxyphenyl)-1,6-dihydro-7H-pyrazolo[4,3-d]pyrimidin-7-one

Obtained as an orange solid (67%) from the title compound of Preparation 23 using a similar procedure to that described in Preparation 28.
δ (DMSOd₆) : 0.96 (3H, t), 1.73 (2H, m), 2.50 (2H, m), 2.77 (2H, m), 3.32 (2H, m), 3.61 (4H, m), 4.05 (2H, t), 4.84 (2H, t), 7.08 (1H, m), 7.18 (1H, d), 7.52 (1H, m), 7.74 (3H, m), 12.34 (1H, s).
LRMS : m/z 427 (M+1)⁺

### Preparation 32

### 3-Carboxamido-5-(2-n-propoxyphenyl)-2,6-dihydro-7H-pyrazolo[4,3-d]pyrimidin-7-one

A mixture of the title compound of Preparation 25 (1.2g, 3.62mmol) and potassium t-butoxide (1.63g, 14.49mmol) in n-propanol (50ml) was heated under reflux for 18 hours. The cooled reaction mixture was concentrated under reduced pressure, the residue dissolved in water (30ml), washed with ethyl acetate (20ml) and acidified to pH 4 with hydrochloric acid.

The resulting precipitate was filtered, washed with water and dried at 60°C. A mixture of this solid, and N,N'-carbonyldiimidazole (670mg, 4.13mmol) in tetrahydrofuran (50ml) was heated under reflux for 3 hours, then cooled in an ice-bath. The mixture was saturated with ammonia gas, and stirred at room temperature for 18 hours. The resulting precipitate was filtered, washed with ethyl acetate and dried at 60°C to afford the title compound (510mg, 45%) as a beige solid.
δ (DMSOd₆) : 0.96 (3H, t), 1.75 (2H, m), 4.05 (2H, t), 7.08 (1H, m), 7.19 (1H, d), 7.50 (1H, m), 7.67 (1H, s), 7.71 (1H, s), 7.79 (1H, d).
LRMS : m/z 314 (M+1)⁺

### Preparation 33

### 3-N-Methylcarboxamido-5-(2-n-propoxyphenyl)-1-(pyridin-2-yl)methyl-1,6-dihydro-7H-pyrazolo[4,3-d]pyrimidin-7-one

A mixture of the title compound of Preparation 26 (600mg, 1.48mmol) and 2N aqueous sodium hydroxide solution (20ml) in dioxan (10ml) was heated under reflux for 18 hours. The cooled reaction mixture was neutralized with 2N hydrochloric acid, concentrated under reduced pressure and azeotroped with toluene. The residual white solid was suspended in dichloromethane (20ml), N-methylmorpholine (360ml, 3.26mmol), 1-hydroxybenzotriazole hydrate (220mg, 1.63mmol), methylamine hydrochloride (220mg, 3.26mmol) and finally 1-(3-dimethylaminopropyl)-3-ethylcarbodiimide hydrochloride (312mg, 1.63mmol) added and the reaction stirred at room temperature for 18 hours. The reaction mixture was filtered, sodium bicarbonate solution (20ml) added, and the phases separated. The aqueous phase was extracted with dichloromethane (4x20ml), the combined organic extracts washed with brine (20ml), dried and evaporated under reduced pressure. The crude product was purified by column chromatography on silica gel, using an elution gradient of dichloromethane:methanol:0.88 ammonia (100:0:0 to 98:2:1) to afford the title compound (170mg, 30%) as a pale yellow solid.
δ (CDCl₃) : 1.19 (3H, t), 2.01 (2H, m), 3.17 (3H, d), 4.20 (2H, t), 6.05 (2H, s), 7.08 (2H, m), 7.18 (2H, m), 7.54 (1H, m), 7.60 (1H, m), 8.12 (1H, s), 8.42 (1H, d), 8.55 (1H, d), 11.42 (1H, s).
LRMS : m/z 419 (M+1)⁺

### Preparation 34

### 3-N-Methylcarboxamido-5-(2-n-propoxyphenyl)-1-(pyridin-3-yl)methyl-1,6-dihydro-7H-pyrazolo[4,3-d]pyrimidin-7-one

Obtained as a white solid (55%) from the title compound of Preparation 27, using the procedure of Preparation 33.
δ (CDCl₃) : 1.20 (3H, t), 2.04 (2H, m), 3.16 (3H, d), 4.24 (2H, t), 5.90 (2H, s), 7.09 (1H, d), 7.21 (2H, m), 7.55 (1H, m), 7.85 (1H, d), 8.15 (1H, m), 8.39 (1H, d), 8.55 (1H, d), 8.79 (1H, s), 11.43 (1H, s).

### Preparation 35

### 3-N-Methylcarboxamido-5-(2-n-propoxyphenyl)-1-(pyridin-4-yl)methyl-1,6-dihydro-7H-pyrazolo[4,3-d]pyrimidin-7-one

Obtained as a white solid (37%) from the title compound of Preparation 28, using a similar procedure to that described in Preparation 33.
δ (CDCl₃) : 1.18 (3H, t), 2.02 (2H, m), 3.17 (3H, d), 4.21 (2H, t), 5.86 (2H, s), 7.10 (1H, d), 7.20 (1H, m), 7.30 (2H, d), 7.55 (1H, m), 8.16 (1H, s), 8.39 (1H, d), 8.56 (2H, d), 11.48 (1H, s).
LRMS : m/z 419 (M+1)⁺

### Preparation 36

### 1-(4-Bromobenzyl)-3-N-methylcarboxamido-5-(2-n-propoxyphenyl)-1,6-dihydro-7H-pyrazolo[4,3-d]pyrimidin-7-one

A mixture of the title compound of Preparation 29 (2.56g, 5.3mmol) and 6N aqueous sodium hydroxide solution (60ml) in ethanol (30ml) was heated under reflux for 18 hours. The cooled reaction mixture was acidified with hydrochloric acid, the resulting precipitate filtered, washed with water, and dried at 60°C, to give a white solid. A mixture of this product, N-methylmorpholine (1.29ml, 11.7mmol), 1-hydroxybenzotriazole hydrate (950mg, 6.2mmol), methylamine hydrochloride (357mg, 5.3mmol) and finally 1-(3-dimethylaminopropyl)-3-ethylcarbodiimide hydrochloride (1.28mg, 6.7mmol) in dichloromethane (40ml) was stirred at room temperature for 3 hours. The reaction mixture was washed with ammonium chloride solution (10ml), then sodium bicarbonate solution (10ml), dried (Na₂SO₄) and concentrated under reduced pressure. The residue was purified by column chromatography on silica gel, using dichloromethane:methanol:0.88 ammonia (98:2:0.2) as eluant to afford the title compound (1.1g, 42%).
δ (CDCl₃) : 1.19 (3H, t), 2.04 (2H, m), 3.16 (3H, d), 4.22 (2H, t), 5.81 (2H, s), 7.09 (1H, d), 7.19 (1H, m), 7.41 (3H, m), 7.53 (1H, m), 8.15 (1H, d), 8.58 (1H, m), 11.40 (1H, s).
LRMS : m/z 498 (M+2)⁺

### Preparation 37

### 1-Cyclobutyl-3-N-methylcarboxamido-5-(2-n-propoxyphenyl)-1,6-dihydro-7H-pyrazolo[4,3-p]pyrimidin-7-one

Obtained after recrystallisation from ethyl acetate-hexane (51%), from the title compound of Preparation 30, using a similar procedure to that described in Preparation 36.
δ (CDCl₃) : 1.17 (3H, t), 1.86 (4H, m), 1.99 (4H, m), 3.00 (1H, m), 3.12 (3H, d), 4.20 (2H, t), 4.69 (2H, d), 7.06 (1H, d), 7.17 (1H, m), 7.50 (1H, m), 8.11 (1H, m), 8.38 (1H, d), 11.35 (1H, s).
LRMS : m/z 395 (M)⁺

### Preparation 38

### 3-Methoxycarbonyl-4-nitro-1-(pyridin-2-yl)methyl-1H-pyrazole-5-carboxylic acid

Potassium hydroxide solution (6.87ml, 1N, 6.87mmol) was added to a suspension of the title compound of Preparation 3 (2.0g, 6.25mmol) in methanol (50ml) and the reaction stirred at room temperature for 18 hours. The reaction mixture was concentrated under reduced pressure, the residual brown oil dissolved in water (15ml), and washed with diethyl ether (20ml). The aqueous solution was acidified to pH 4 with 2N hydrochloric acid, the resulting precipitate filtered, washed with water and diethyl ether, and dried at 60°C, to afford the title compound (1.37g, 72%).
Found : C, 46.63; H, 3.11; N, 18.00. C₁₂H₃₀N₄O₆ requires C, 47.07; H, 3.29; N, 18.30%.
δ (DMSOd₆) : 3.85 (3H, s), 5.92 (2H, s), 7.34 (2H, m), 7.81 (1H, m), 8.48 (1H, d).

### Preparation 39

### 1-Benzyl-3-methoxycarbonyl-4-nitro-1H-pyrazole-5-carboxylic acid

A methanolic solution of potassium hydroxide (27ml, 2N, 54mmol) was added to a solution of the title compound of Preparation 6 (17.4g, 54.5mmol) in methanol (400ml) and the reaction stirred at room temperature for 40 hours. The reaction mixture was concentrated under reduced pressure, the residue suspended in water (100ml), and acidified to pH 4 using 2N hydrochloric acid. This mixture was evaporated under reduced pressure and recrystallised from dichloromethane-pentane to afford the title compound as a solid.
δ (DMSOd₆) : 3.80 (3H, s), 5.74 (2H, s), 7.23-7.38 (5H, m).

### Preparation 40

### 3-Methoxycarbonyl-1-methyl-4-nitro-1H-pyrazole-5-carboxylic acid

An aqueous solution of potassium hydroxide (6.48ml, 2N, 12.95mmol) was added to a suspension of the title compound of Preparation 8 (3.0g, 12.37mmol) in methanol (60ml) and the reaction stirred at room temperature for 18 hours. The reaction mixture was concentrated under reduced pressure, the residue partitioned between ethyl acetate (30ml) and water (30ml), and the phases separated. The aqueous layer was acidified to pH 4 using 2N hydrochloric acid, extracted with ethyl acetate (4x50ml) and the combined organic solutions dried (Na₂SO₄), and evaporated under reduced pressure to afford the title compound (1.97g, 70%) as a white solid.
δ (DMSOd₆) : 3.83 (3H, s), 4.14 (3H, s).
LRMS : m/z 247 (M+18)⁺

### Preparation 41

### 3-Methoxycarbonyl-1-methyl-4-nitro-1H-pyrazole-5-carboxylic acid chloride

Oxalyl chloride (3.05ml, 34.9mmol) was added dropwise to an ice-cooled suspension of the title compound of Preparation 40 (4.0g, 17.5mmol) and dimethylformamide (1 drop) in dichloromethane (50ml), and the reaction was stirred at room temperature for 18 hours. The reaction mixture was concentrated under reduced pressure, and the residue triturated with hexane to afford the title compound as a beige solid. δ (DMSOd₆) : 3.85 (3H, s), 4.15 (3H, s).

### Preparation 42

### 1-Benzyl-3-methoxycarbonyl-4-nitro-1H-pyrazole-5-carboxamide

Oxalyl chloride (7.8ml, 90mmol) was added dropwise to an ice-cooled solution of the title compound of Preparation 39 (13.7g, 44.9mmol) and dimethylformamide (1 drop) in dichloromethane (100ml), and the reaction stirred at room temperature for 4 hours. The reaction mixture was concentrated under reduced pressure, the residue suspended in dioxan (50ml), and cooled in an ice-bath. 0.88 Ammonia was added dropwise until a pH of 8 had been achieved, the mixture stirred for 30 minutes, then concentrated under reduced pressure. The residue was triturated with water, filtered and dried under suction to afford the title compound (8.2g, 60%) as a white powder.

### Preparation 43

### 3-Methoxycarbonyl-1-methyl-4-nitro-1H-pyrazole-5-carboxamide

Oxalyl chloride (1.1ml, 12.6mmol) was added dropwise to an ice-cooled solution of the title compound of Preparation 40 (1.93g, 8.42mmol) and dimethylformamide (1 drop) in dichloromethane (30ml), and the reaction stirred at room temperature for 18 hours. The reaction mixture was evaporated under reduced pressure, triturated with tetrahydrofuran (100ml), filtered and the filtrate cooled in an ice-bath. Ammonia gas was passed through the solution for 30 minutes, the resulting precipitate filtered, washed with water and dried at 60°C to afford the title compound (1.39g, 72%) as a white solid.
Found : C, 36.97; H, 3.55; N, 24.36. C₇H₈N₄O₅ requires C, 36.85; H, 3.53; N, 24.56%.
δ (DMSOd₆) : 3.88 (3H, s), 3.92 (3H, s), 8.37 (1H, s), 8.50 (1H, s).
LRMS : m/z 246 (M+18)⁺

### Preparation 44

### 5-Methoxycarbonyl-1-methyl-4-nitro-1H-pyrazole-3-carboxamide

Obtained as a white solid after recrystallisation from methanol-ethyl acetate (49%), from 1-methyl-5-(methoxycarbonyl)-4-nitropyrazole-3-carboxylic acid (J.Med.Chem. 1994, 37, 4335) using the procedure of Preparation 43.
Found : C, 36.70; H, 3.42; N, 24.33. C₇H₈N₄O₅ requires C, 36.85; H, 3.53; N, 24.56%.
δ (DMSOd₆) : 3.87 (3H, s), 4.12 (3H, s), 7.77 (1H, s), 8.01 (1H, s).
LRMS : m/z 246 (M+18)⁺

### Preparation 45

### 3-Methoxycarbonyl-4-nitro-1-(pyridin-2-yl)methyl-1H-pyrazole-5-N-methylcarboxamide

A mixture of the title compound of Preparation 38 (1.36g, 4.45mmol), 1-(3-dimethylaminopropyl)-3-ethylcarbodiimide hydrochloride (853mg, 4.45mmol), 1-hydroxybenzotriazole hydrate (682mg, 4.45mmol), methylamine hydrochloride (1.20g, 17.79mmol) and N-ethyldiisopropylamine (3.87ml, 22.24mmol) in dichloromethane (30ml) was stirred at room temperature for 18 hours. The reaction mixture was washed consecutively with water (10ml), 0.5N hydrochloric acid (10ml), 0.5N sodium hydroxide solution (10ml) and water (10ml), then dried (MgSO₄) and evaporated under reduced pressure. The residual orange gum was purified by column chromatography on silica gel, using an elution gradient of dichloromethane:methanol (99:1 to 95:5) to afford the title compound (220mg, 15%) as an orange solid.
δ (CDCl₃) : 3.02 (3H, d), 3.94 (3H, s), 5.67 (2H, s), 7.30 (1H, m), 7.39 (1H, d), 7.78 (1H, m), 8.55 (1H, d), 8.72 (1H, m).
LRMS : m/z 320 (M+1)⁺

### Preparation 46

### 3-Methoxycarbonyl-1-methyl-4-nitro-1H-pyrazole-5-N-methylcarboxamide

Methylamine hydrochloride (2.16g, 32mmol) and triethylamine (5.87ml, 80mmol) were added to an ice-cold solution of the title compound of Preparation 41 (4.55g, 16mmol) in dichloromethane (40ml) and the reaction stirred at room temperature for 18 hours. The reaction mixture was filtered, and the filtrate washed consecutively with water (20ml), 1N hydrochloric acid (20ml), 1N sodium hydroxide solution (3x20ml) and water (20ml). The organic solution was dried (MgSO₄), concentrated under reduced pressure and the residue triturated with diethyl ether to afford the title compound (1.30g, 34%) as a beige solid.
Found : C, 39.54; H, 4.13; N, 22.90. C₈H₁₀N₄O₅ requires C, 39.67; H, 4.16; N, 23.13%.
δ (CDCl₃) : 3.03 (3H, d), 3.96 (3H, s), 4.19 (3H, s), 7.34 (1H, m). LRMS : m/z 243 (M+1)⁺

### Preparation 47

### 4-Nitro-1 -(pyridin-2-yl)methyl-1H-pyrazole-3,5-dicarboxamide

A suspension of the title compound of Preparation 3 (5.0g, 15.6mmol) in methanol (250ml), was saturated with ammonia gas for an hour, and the reaction stirred for a further 90 minutes at room temperature. The reaction mixture was evaporated under reduced pressure, azeotroped with dichloromethane and dried under vacuum to afford the title compound (4.53g, 100%) as a beige solid.
Found : C, 45.35; H, 3.46; N, 28.78. C₁₁H₁₀N₆O₄ requires C, 45.52; H, 3.47; N, 28.96%.
δ (DMSOd₆) : 5.52 (2H, s), 7.29 (1H, d), 7.36 (1H, m), 7.76 (1H, s), 7.81 (1H, m), 8.04 (1H, s), 8.23 (1H, s), 8.55 (2H, m).
LRMS : m/z 291 (M+1)⁺

### Preparation 48

### 5-N-Methylcarboxamido-4-nitro-1-(pyridin-2-yl)methyl-1H-pyrazole-3-carboxamide

An ice-cooled solution of the title compound of Preparation 45 (215mg, 0.67mmol) in methanol (10ml) was saturated with ammonia, and the mixture stirred at room temperature for an hour. The reaction mixture was concentrated under reduced pressure and azeotroped with dichloromethane to afford the title compound (206mg, 100%) as a beige foam.
δ (CDCl₃) : 3.01 (3H, d), 5.60 (2H, s), 5.74 (1H, s), 7.05 (1H, s), 7.34 (1H, m), 7.40 (1H, d), 7.78 (1H, m), 8.55 (1H, d), 8.62 (1H, s).
LRMS : m/z 305 (M+1)⁺

### Preparation 49

### 1-Methyl-5-N-methylcarboxamido-4-nitro-1H-pyrazole-3-carboxamide

Obtained as a solid (99%) from the title compound of Preparation 46, using the procedure of Preparation 48.
Found : C, 36.89; H, 3.91; N, 30.59. C₇H₉N₅O₄ requires C, 37.01; H, 3.99; N, 30.83%.
δ (DMSOd₆) : 2.80 (3H, d), 3.82 (3H, s), 7.74 (1H, s), 8.04 (1H, s), 9.00 (1H, m).
LRMS : m/z 245 (M+18)⁺

### Preparation 50

### 4-Amino-1-benzyl-3-methoxycarbonyl-1H-pyrazole-5-carboxamide

Obtained as a dark brown solid (81%) from the tide compound of Preparation 42 and Raney® nickel using a similar procedure to that described in Preparation 11.
LRMS : m/z 275 (M+1)⁺

### Preparation 51

### 4-Amino-1-methyl-3-methoxycarbonyl-1H-pyrazole-5-carboxamide

Obtained as a white solid (99%) from the title compound of Preparation 43, using the procedure of Preparation 11.
Found : C, 42.18; H, 5.00; N, 27.35. C₇H₁₀N₄O₃ requires C, 42.42; H, 5.09; N, 28.37%.
δ (DMSOd₆) : 3.78 (3H, s), 3.97 (3H, s), 5.18 (2H, s), 7.39 (2H, s).
LRMS : m/z 199 (M+1)⁺

### Preparation 52

### 4-Amino-1-methyl-5-methoxycarbonyl-1H-pyrazole-3-carboxamide

Obtained as a white solid (91%) from the title compound of Preparation 44 and Raney nickel, using the procedure of Preparation 11.
δ (DMSOd₆) : 3.82 (3H, s), 3.98 (3H, s), 5.56 (2H, s), 7.16 (1H, s), 7.34 (1H, s).
LRMS : m/z 199 (M+1)⁺

### Preparation 53

### 4-Amino-1-(pyridin-2-yl)methyl-1H-pyrazole-3,5-dicarboxamide

Obtained as a white solid (90%) from the title compound of Preparation 47 using the procedure of Preparation 11.
δ (DMSOd₆) : 5.28 (2H, s), 5.71 (2H, s), 6.93 (1H, d), 7.19 (1H, s), 7.28 (1H, m), 7.38 (1H, s), 7.46 (2H, s), 7.76 (1H, m), 8.48 (1H, d).
LRMS : m/z 260 (M)⁺

### Preparation 54

### 4-Amino-5-N-methylcarboxamido-1-(pyridin-2-yl)methyl-1H-pyrazole-3-carboxamide

A mixture of the title compound of Preparation 48 (200mg, 0.66mmol) and 10% palladium on charcoal (40mg) in ethanol (10ml) was hydrogenated at 30°C and 207kPa (30psi), for 3 hours, then filtered. The filtrate was combined with an ethanol (30ml) wash of the filter pad, concentrated under reduced pressure and azeotroped with dichloromethane to afford the title compound (135mg, 75%).
δ (CDCl₃) : 3.00 (3H, s), 5.00 (2H, s), 5.26 (2H, s), 5.55 (2H, s), 7.29 (1H, m), 7.38 (1H, d), 7.75 (1H, m), 8.54 (1H, d), 8.97 (1H, s).
LRMS : m/z 275 (M+1)⁺

### Preparation 55

### 4-Amino-1-methyl-5-N-methylcarboxamide-1H-pyrazole-3-carboxamide

Obtained as a white solid (65%) from the title compound of Preparation 49, using the procedure of Preparation 11.
Found : C, 42.52; H, 5.86; N, 34.95. C₇H₁₁N₅O₂ requires C, 42.64; H, 5.62; N, 35.51%.
δ (DMSOd₆) : 2.75 (3H, d), 3.90 (3H, s), 5.13 (2H, s), 7.14 (1H, s), 7.33 (1H, s), 7.60 (1H, m).

### Preparation 56

### 3-Methoxycarbonyl-2-methyl-4-(2-n-propoxybenzamido)-1H-pyrazole-5-carboxamide

A solution of 2-n-propoxybenzoyl chloride (644mg, 3.25mmol) in dichloromethane (5ml) was added dropwise to an ice-cooled solution of the title compound of Preparation 52 (642mg, 3.25mmol) in pyridine (15ml) and the reaction stirred at room temperature for 72 hours. The reaction mixture was concentrated under reduced pressure, the residue partitioned between dichloromethane (30ml) and 1N hydrochloric acid (20ml), and the phases separated. The organic layer was washed with 1N hydrochloric acid (2x20ml), dried (MgSO₄) and evaporated under reduced pressure. The residue was purified by column chromatography on silica gel, using an elution gradient of dichloromethane:methanol (99:1 to 98:2) to afford the title compound (700mg, 60%) as a white solid.
δ (CDCl₃) : 1.04 (3H, t), 2.04 (2H, m), 3.88 (3H, s), 4.08 (3H, s), 4.25 (2H, t), 5.44 (1H, s), 6.70 (1H, s), 7.04 (2H, m), 7.47 (1H, m), 8.21 (1H, d), 10.82 (1H, s).
LRMS : m/z 361 (M+1)⁺

### Preparation 57

### 3-Methoxycarbonyl-1-methyl-4-(2-n-propoxybenzamido)-1H-pyrazole-5-carboxamide

A solution of 2-n-propoxybenzoyl chloride (1.12g, 5.65mmol) in dichloromethane (5ml) was added slowly to an ice-cooled solution of the title compound of Preparation 51 (1.12g, 5.65mmol) in pyridine (20ml) and the reaction stirred at room temperature for 18 hours. The mixture was concentrated under reduced pressure, the residue partitioned between dichloromethane (60ml) and 2N hydrochloric acid (20ml) and the phases separated. The organic layer was washed with 2N hydrochloric acid (20ml), dried (MgSO₄) and evaporated under reduced pressure to afford the title compound (2.0g, 98%) as a white foam.
δ (CDCl₃) : 1.06 (3H, t), 1.99 (2H, m), 3.93 (3H, s), 4.22 (5H, m), 5.72 (1H, s), 7.09 (2H, m), 7.55 (1H, m), 8.28 (2H, m), 10.47 (1H, s).
LRMS : m/z 361 (M+1)⁺

### Preparation 58

### Potassium 2-methyl-7-oxo-5-(2-n-propoxyphenyl)-2,6-dihydro-2H-pyrazolo[4,3-d]pyrimidine-3-carboxylate

A mixture of potassium t-butoxide (498mg, 4.44mmol) and the title compound of Preparation 56 (400mg, 1.11mmol) in n-propanol (20ml) was heated under reflux for 20 hours, then cooled. The resulting precipitate was filtered, washed with diethyl ether and dried at 60°C, to afford the title compound (286mg, 79%) as a white solid.
δ (DMSOd₆) : 0.80 (3H, t), 1.57 (2H, m), 3.85 (2H, t), 4.21 (3H, s), 6.98 (2H, m), 7.21 (1H, m), 7.40 (1H, d).

### Preparation 59

### 1-Methyl-7-oxo-5-(2-D-propoxyphenyl)-1,6-dihydro-1H-pyrazolo[4,3-d]pyrimidine-3-carboxylic acid

Potassium t-butoxide (2.15g, 19.13mmol) was added portionwise to a solution of the title compound of Preparation 57 (1.97g, 5.47mmol) in n-propanol (50ml) and the reaction heated under reflux for 22 hours. The cooled reaction mixture was concentrated under reduced pressure, the residue dissolved in water (20ml) and acidified to pH 4 with 2N hydrochloric acid. The resulting precipitate was filtered, washed with water, and dried at 60°C to afford the title compound (1.64g, 91%) as a white solid.
δ (DMSOd₆) : 0.96 (3H, t), 1.72 (2H, m), 4.03 (2H, t), 4.28 (3H, s), 7.06 (1H, m), 7.18 (1H, d), 7.50 (1H, m), 7.68 (1H, d), 12.20 (1H, s), 12.91 (1H, s).
LRMS : m/z 329 (M+1)⁺

### Preparation 60

### 2-Methyl-7-oxo-5-(2-n-propoxyphenyl)-2,6-dihydro-2H-pyrazolo[4,3-d]pyrimidine-3-N-methylcarboxamide

A mixture of the title compound of Preparation 58 (140mg, 0.38mmol), methylamine hydrochloride (29mg, 0.42mmol), N-ethyldiisopropylamine (220ml, 1.28mmol) and bromo-tris-pyrrolidino-phosphonium hexafluorophosphate (199mg, 0.42mmol) in dichloromethane (10ml) and dimethylformamide (5ml) was stirred at room temperature for 72 hours. The reaction mixture was concentrated under reduced pressure, the residue dissolved in dichloromethane (20ml), and washed with 1N hydrochloric acid (2x10ml), then water (10ml), dried (MgSO₄) and evaporated under reduced pressure. The residue was triturated with diethyl ether to afford the title compound (125mg, 96%) as a white solid.
δ (DMSOd₆) : 0.95 (3H, t), 1.74 (2H, m), 2.89 (3H, d), 4.04 (2H, t), 4.37 (3H, s), 7.08 (1H, m), 7.18 (1H, d), 7.51 (1H, m), 7.80 (1H, d), 8.34 (1H, s), 11.99 (1H, s).
LRMS : m/z 342 (M+1)⁺

### Preparation 61

### 1-Methyl-7-oxo-5-(2-n-propoxyphenyl)-1,6-dihydro-1H-pyrazolo[4,3-d]pyrimidine-3-carboxamide

A mixture of the title compound of Preparation 59 (1.57g, 4.77mmol) and N,N'-carbonyldiimidazole (850mg, 5.24mmol) in tetrahydrofuran (50ml) was heated under reflux for 3 hours, then ice-cooled. This solution was saturated with ammonia gas, and the reaction mixture stirred at room temperature for 18 hours. The resulting precipitate was filtered, washed with ethyl acetate and dried at 60°C to afford the title compound (1.37g, 88%) as a white solid.
δ (DMSOd₆) : 0.97 (3H, t), 1.73 (2H, m), 4.04 (2H, t), 4.25 (3H, s), 7.08 (1H, m), 7.19 (1H, d), 7.51 (1H, m), 7.71 (3H, m), 11.50 (1H, s).

### Preparation 62

### Pyridine-2-amino-5-sulphonic acid

2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the title compound (111.3g, 75%) as a solid.
LRMS : m/z 175 (M+1)⁺

### Preparation 63

### Pyridine-2-amino-3-bromo-5-sulphonic acid

Bromine (99g, 0.62mol) was added dropwise over an hour, to a solution of the title compound of Preparation 62 (108g, 0.62mol) in water (600ml) so as to maintain a steady reflux. Once the addition was complete the reaction was cooled and the resulting mixture filtered. The solid was washed with water and dried under suction to afford the title compound (53.4g, 34%).
δ (DMSOd₆) : 8.08 (1H, s), 8.14 (1H, s).
LRMS : m/z 253 (M)⁺

### Preparation 64

### Pyridine-3-bromo-2-chloro-5-sulphonyl chloride

A solution of sodium nitrite (7.6g, 110mmol) in water (30ml) was added dropwise to an ice-cooled solution of the title compound of Preparation 63 (25.3g, 100mmol) in aqueous hydrochloric acid (115ml, 20%), so as to maintain the temperature below 6°C. The reaction was stirred for 30 minutes at 0°C and a further hour at room temperature. The reaction mixture was evaporated under reduced pressure and the residue dried under vacuum at 70°C for 72 hours. A mixture of this solid, phosphorus pentachloride (30g, 144mmol) and phosphorus oxychloride (1ml) was heated at 125°C for 3 hours, and then cooled. The reaction mixture was poured onto ice (100g) and the resulting solid filtered, and washed with water. The product was dissolved in dichloromethane, dried (MgSO₄), filtered and evaporated under reduced pressure to afford the title compound (26.58g, 91%) as a yellow solid. δ (CDCl₃) : 8.46 (1H, s), 8.92 (1H, s).

### Preparation 65

### Pyridine-3-bromo-5-(4-ethylpiperazin-1-ylsulphonyl)-2-chloride

A solution of 1-ethyl piperazine (11.3ml, 89mmol) and triethylamine (12.5ml, 89mmol) in dichloromethane (150ml) was added dropwise to an ice-cooled solution of the title compound of Preparation 64 (23g, 79mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the title compound (14.5g, 50%) as an orange solid.
δ (CDCl₃) : 1.05 (3H, t), 2.42 (2H, q), 2.55 (4H, m), 3.12 (4H, m), 8.24 (1H, s), 8.67 (1H, s).

### Preparation 66

### 3-Bromo-2-ethoxy-5-(4-ethylpiperazin-1-ylsulphonyl)pyridine

A mixture of the title compound of Preparation 65 (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the title compound (6.41g, 95%) as a brown solid.
Found : C, 41.27; H, 5.33; N, 11.11. C₁₃H₂₀BrN₃O₃S requires C, 41.35; H, 5.28; N, 10.99%.
δ (CDCl₃) : 1.06 (3H, t), 1.48 (2H, m), 2.42 (2H, q), 2.56 (4H, m), 3.09 (4H, m), 4.54 (2H, q), 8.10 (1H, s), 8.46 (1H, s).
LRMS : m/z 380 (M+2)⁺

### Preparation 67

### Pyridine-2-ethoxy-5-(4-ethylpiperazin-1-ylsulphonyl)-3-carboxylic acid ethyl ester

A mixture of the title compound of Preparation 66 (6.40g, 16.92mmol), triethylamine (12ml), and palladium (0) tris(triphenylphosphine) in ethanol (60ml) was heated at 100°C and 200psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the title compound (6.2g, 99%) as an orange oil.
δ (CDCl₃) : 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s).
LRMS : m/z 372 (M+1)⁺

### Preparation 68

### Pyridine-2-ethoxy-5-(4-ethylpiperazin-1-ylsulphonyl)-3-carboxylic acid

A mixture of the title compound of Preparation 67 (4.96g, 13.35mmol) and aqueous sodium hydroxide solution (25ml, 2N) in ethanol (25ml) was stirred at room temperature for 2 hours. The reaction mixture was concentrated under reduced pressure to half its volume, washed with diethyl ether and acidified to pH 5 using 4N hydrochloric acid. The aqueous solution was extracted with dichloromethane (3x30ml), the combined organic extracts dried (MgSO₄) and evaporated under reduced pressure to afford the title compound (4.02g, 88%) as a tan coloured solid.
δ (DMSOd₆) : 1.18 (3H, t), 1.37 (3H, t), 3.08 (2H, q), 3.17-3.35 (8H, m), 4.52 (2H, q), 8.30 (1H, s), 8.70 (1H, s).

### Preparation 69

### Pyridine-2-ethoxy-5-(4-ethylpiperazin-1-ylsulphonyl)-3-carboxylic acid chloride hydrochloride

Oxalyl chloride (0.77ml, 8.85mmol) was added dropwise to an ice-cooled solution of the title compound of Preparation 68 (1.52g, 4.42mmol) and dimethylformamide (2 drops) in dichloromethane (30ml) and the reaction stirred for 18 hours at room temperature. The mixture was concentrated under reduced pressure and the residue triturated with ethyl acetate. The resulting solid was filtered, washed with diethyl ether and dried under suction to afford the title compound (1.68g, 95%).
Found : C, 41.51; H, 5.27; N, 10.32. C₁₄H₂₁Cl₂N₃O₄S;0.10CH₂Cl₂ requires C, 41.73; H, 5.02; N, 10.36%.
δ (CDCl₃) : 1.46 (6H, m), 2.95 (2H, q), 3.11 (2H, m), 3.48 (2H, m), 3.55 (2H, m), 3.92 (2H, m), 4.60 (2H, q), 8.58 (1H, s), 8.66 (1H, s), 13.16 (1H, s).

### Preparation 70

### 2-Ethoxy-5-(4-methylpiperazin-1-ylsulphonyl)benzoic acid chloride hydrochloride

Oxalyl chloride (11.7ml, 134mmol) was added dropwise to an ice cold suspension of 2-ethoxy-5-(4-methylpiperazin-1-ylsulphonyl)benzoic acid (EP 812845) (20.0g, 60.9mmol) and dimethylformamide (2 drops) in dichloromethane (200ml) over 15 minutes, and the reaction mixture stirred at room temperature for 18 hours. The mixture was concentrated under reduced pressure, the residue triturated with ether then ethyl acetate and dried at 40°C for 16 hours, to afford the title compound, (19.6g; 93%).
δ (DMSOd₆) : 1.35 (3H, t), 2.70 (5H, m), 3.12 (2H, m), 3.41 (2H, m), 3.75 (2H, m), 4.21 (2H, q), 7.38 (1H, d), 7.83 (1H, d), 7.94 (1H, s), 11.26 (1H, s).

### Preparation 71

### 1-Benzyl-4-[2-ethoxy-5-(4-methylpiperazin-1-ylsulphonyl)benzamido]-3-methoxycarbonyl-pyrazole-5-carboxamide

A solution of the title compound of Preparation 70 (4.51g, 13.0mmol) in dichloromethane (18ml) was added dropwise to an ice-cooled solution of the title compound of Preparation 50 (3.56g, 13.0mmol) in pyridine (20ml) and dichloromethane (2ml), and the reaction stirred at room temperature for 20 hours. The reaction mixture was concentrated under reduced pressure, azeotroped with toluene and the residual oil partitioned between dichloromethane (50ml) and sodium bicarbonate solution (50ml). The phases were separated, the aqueous layer extracted with dichloromethane (2x50ml), and the combined organic solutions dried (Na₂SO₄) and evaporated under reduced pressure. The residual oil was purified by column chromatography on silica gel, using an elution gradient of dichloromethane:methanol (100:0 to 90:10) and triturated with diethyl ether to afford the title compound (5.08g, 67%) as a white solid.
δ (CDCl₃) : 1.58 (3H, t), 2.20 (3H, s), 2.41 (4H, m), 2.98 (4H, m), 3.88 (3H, s), 4.39 (2H, q), 5.70 (2H, s), 7.13 (1H, d), 7.23 (7H, m), 7.82 (1H, m), 8.56 (1H, s), 10.39 (1H, s).
LRMS : m/z 585 (M+1)⁺

### Preparation 72

### 1-Benzyl-7-oxo-5-[2-ethoxy-5-(4-methylpiperazin-1-ylsulphonyl)phenyl]-1,6-dihydro-1H-pyrazolo[4,3-d]pyrimidine-3-carboxylic acid

A mixture of the title compound of Preparation 71 (5.08g, 8.69mmol) and potassium t-butoxide (3.41g, 30.4mmol) in isopropanol (80ml) was heated under reflux for 10 hours, then cooled. Water (80ml) was added and the mixture acidified to pH 5 using concentrated hydrochloric acid. The resulting precipitate was filtered, washed with water and dried to afford the title compound, (3.95g, 85%) as a white solid.
δ (DMSOd₆) : 1.34 (3H, t), 2.18 (3H, s), 2.40 (4H, m), 2.94 (4H, m), 4.23 (2H, q), 5.84 (2H, s), 7.36 (6H, m), 7.84 (1H, d), 7.94 (1H, s), 12.44 (1H, s).
LRMS : m/z 553 (M+1)⁺

### Preparation 73

### 4-[2-Ethoxy-5-(4-ethylpiperazin-1-ylsulphonyl)pyridin-3-ylcarboxamido]-2-(pyridin-2-yl)methyl-pyrazole-3,5-dicarboxamide

Triethylamine (1.26ml, 9.04mmol) was added dropwise to an ice-cold suspension of the title compounds of Preparations 69 (1.20g, 3.01mmol) and 53 (784mg, 3.01mmol) in dichloromethane (50ml), and the reaction stirred at room temperature for 18 hours. The reaction mixture was diluted with dichloromethane (50ml), washed with water (15ml), and saturated sodium carbonate solution (15ml), dried (MgSO₄) and concentrated under reduced pressure. The residual brown foam was purified by column chromatography on silica gel, using an elution gradient of dichloromethane:methanol (98:2 to 95:5) to afford the title compound (845mg, 49%) as a white solid.
δ (DMSOd₆) : 0.92 (3H, t), 1.49 (3H, t), 2.30 (2H, q), 2.42 (4H, m), 2.95 (4H, m), 4.70 (2H, q), 5.68 (2H, s), 7.17 (1H, d), 7.30 (1H, m), 7.50 (1H, s), 7.66 (2H, s), 7.70 (1H, s), 7.78 (1H, m), 8.50 (2H, m), 8.72 (1H, s), 10.81 (1H, s).
LRMS : m/z 587 (M+2)⁺

### Preparation 74

### 4-[2-Ethoxy-5-(4-ethylpiperazin-1-ylsulphonyl)pyridin-3-ylcarboxamido]-3-N-methylcarboxamido-2-(pyridin-2-yl)methyl-pyrazole-5-carboxamide

Obtained as a yellow foam (59%) from the title compounds of Preparations of 69 and 54, using the procedure of Preparation 73.
δ (CDCl₃) : 1.01 (3H, t), 1.59 (3H, t), 2.40 (2H, q), 2.53 (4H, m), 2.94 (3H, d), 3.08 (4H, m), 4.79 (2H, q), 5.32 (1H, s), 5.66 (2H, s), 6.68 (1H, s), 7.25 (2H, m), 7.70 (1H, m), 8.45 (1H, m), 8.58 (1H, d), 8.66 (1H, s), 8.86 (1H, s), 10.89 (1H, s).
LRMS : m/z 600 (M+1)⁺

### Preparation 75

### 4-[2-Ethoxy-5-(4-ethylpiperazin-1-ylsulphonyl)pyridin-3-ylcarboxamido]-2-methyl-3-N-methylcarboxamido-pyrazole-5-carboxamide

Obtained as a pink foam (26%) from the title compounds of Preparations 69 and 55, using the procedure of Preparation 73.
δ (CDCl₃) : 1.02 (3H, t), 1.57 (3H, t), 2.40 (2H, q), 2.53 (4H, m), 2.93 (3H, d), 3.10 (4H, m), 4.09 (3H, s), 4.78 (2H, q), 5.50 (1H, s), 6.68 (1H, s), 7.96 (1H, s), 8.68 (1H, s), 8.83 (1H, s), 10.75 (1H, s).
LRMS : m/z 523 (M+1)⁺

### Preparation 76

### Ethyl 4-amino-3-(pyridin-2-yl)-1H-pyrazole-5-carboxylate

2-Pyridylacetonitrile (10ml, 94.0mmol) was added dropwise over 20 minutes to an ice-cooled solution of sodium ethoxide (34ml, 2.76M, 94.0mmol) in ethanol (50ml), and the mixture stirred at 0°C for 30 minutes. Ethyldiazoacetate (9.9ml, 94.0mmol) was added dropwise over 15 minutes and the reaction allowed to warm to room temperature and stirred for a further 18 hours. Water (300ml) was added, the mixture neutralised with solid carbon dioxide, and the resulting precipitate filtered and dried to afford the title compound, (13.0g, 60%) as a brown solid.
δ (CDCl₃) : 1.40 (3H, t), 4.40 (2H, q), 5.78 (2H, s), 7.16 (1H, m), 7.72 (1H, m), 8.00 (1H, d), 8.57 (1H, d).
LRMS : m/z 232 (M)⁺

### Preparation 77

### Ethyl 4-amino-3-(pyridin-3-yl)-1H-pyrazole-5-carboxylate

Obtained (64%) from 3-pyridylacetonitrile and ethyldiazoacetate, using a similar procedure to that described in Preparation 76.
δ (CDCl₃) : 1.38 (3H, t), 4.38 (2H, q), 7.38 (1H, m), 8.01 (1H, d), 8.46 (1H, d), 8.84 (1H, s).

### Preparation 78

### Ethyl 4-(2-n-propoxybenzamido)-3-(pyridin-2-yl)-1H-pyrazole-5-carboxylate

A mixture of the title compound of Preparation 76 (2.14g, 10.78mmol) and 2-n-propoxybenzoyl chloride (2.5g, 10.78mmol) in pyridine (25ml) was heated at 60°C for 5 hours, then cooled. The reaction mixture was concentrated under reduced pressure, azeotroped with toluene and the resulting oil partitioned between dichloromethane (50ml) and sodium bicarbonate solution (50ml). The phases were separated, the aqueous layer extracted with dichloromethane (2x50ml) and the combined organic solutions dried (Na₂SO₄) and evaporated under reduced pressure. The residual brown oil was purified by column chromatography on silica gel, using an elution gradient of dichloromethane:methanol (100:0 to 97:3) to afford the title compound, (3.82g, 90%) as a pink foam.
δ (CDCl₃) : 1.07 (3H, t), 1.38 (3H, t), 2.01 (2H, m), 4.26 (2H, t), 4.40 (2H, q), 7.06 (2H, m), 7.24 (2H, m), 7.55 (2H, m), 7.70 (1H, m), 8.26 (1H, d), 8.60 (1H, d), 10.46 (1H, s).
LRMS : m/z 395 (M+1)⁺

### Preparation 79

### Ethyl 4-(2-n-propoxybenzamido)-3-(pyridin-3-yl)-1H-pyrazole-5-carboxylate

Obtained (74%) from the title compound of Preparation 77 and 2-n-propoxybenzoyl chloride using the procedure described in Preparation 78. δ (CDCl₃) : 1.02 (3H, t), 1.28 (3H, t), 1.92 (2H, m), 4.20 (2H, t), 4.36 (2H, q), 7.03 (2H, m), 7.30 (1H, m), 7.47 (1H, m), 8.02 (1H, d), 8.19 (1H, d), 8.56 (1H, d), 8.97 (1H, s), 10.00 (1H, s), 11.72 (1H, s).

### Preparation 80

### 4-(2-n-Propoxybenzamido)-3-(pyridin-2-yl)-1H-pyrazole-5-carboxamide

An ice-cooled solution of the title compound of Preparation 78 (3.10g, 7.87mmol) in methanol (100ml) was saturated with ammonia gas, and the reaction mixture heated at 100°C for 36 hours in a sealed vessel, then cooled. The mixture was evaporated under reduced pressure and the residue purified by column chromatography on silica gel, using an elution gradient of dichloromethane:methanol (100:0 to 90:10). The product was triturated with diethyl ether to afford the title compound (1.50g, 52%) as a pink solid.
δ (DMSOd₆) : 0.96 (3H, t), 1.86 (2H, m), 4.20 (2H, t), 7.05 (1H, m), 7.22 (1H, d), 7.30 (2H, m), 7.52 (3H, m), 7.86 (2H, m), 8.60 (1H, s), 10.38 (1H, s), 13.76 (1H, s).
LRMS : m/z 366(M+1)⁺

### Preparation 81

### 2-Ethoxypyridine-3-carboxylic acid

A solution of potassium t-butoxide (44.9g, 0.40mol) in absolute ethanol (300ml) was added slowly to a solution of 2-chloronicotinic acid (30g, 0.19mol) in ethanol (100ml), and the reaction heated in a sealed vessel at 170°C for 20 hours. On cooling, the reaction mixture was concentrated under reduced pressure, the residue dissolved in water (200ml) and acidified to pH 3 with aqueous hydrochloric acid. The aqueous solution was extracted with dichloromethane (4x200ml), the organic phases combined, dried (Na₂SO₄) and concentrated under reduced pressure to afford the title compound (27.4g, 41%) as a white solid.
δ (CDCl₃) : 1.53 (3H, t), 4.69 (2H, q), 7.13 (1H, m), 8.37 (1H, d), 8.48 (1H, d).

### Preparation 82

### 2-Ethoxypyridine-3-carboxylic acid ethyl ester

A suspension of the title compound of Preparation 81 (16.4g, 98mmol), and cesium carbonate (32g, 98mmol) in dimethylformamide (240ml) was stirred at room temperature for 2 hours. Ethyl iodide (7.85ml, 98mmol) was added and the reaction stirred for a further 24 hours. The reaction mixture was concentrated under reduced pressure and the residue partitioned between aqueous sodium carbonate solution (100ml) and ethyl acetate (100ml). The phases were separated and the aqueous phase extracted with ethyl acetate (2x100ml). The combined organic phases were washed with brine, dried (Na₂SO₄) and evaporated under reduced pressure to afford the title compound (18.0g, 94%) as a pale yellow oil.
δ (CDCl₃) : 1.41 (6H, m), 4.36, (2H, q), 4.48 (2H, q), 6.90 (1H, m), 8.12 (1H, d), 8.28 (1H, d).

### Preparation 83

### 2-Ethoxy-5-nitropyridine-3-carboxylic acid ethyl ester

Ammonium nitrate (5.36g, 66mmol) was added portionwise to an ice-cooled solution of the title compound of Preparation 82 (4.66g, 22.3mmol) in trifluoroacetic anhydride (50ml) and the reaction stirred for 18 hours at room temperature. The reaction mixture was carefully poured into ice water (200ml) and the resulting suspension stirred for an hour. The precipitate was filtered off, washed with water and dried under suction to afford the title compound (3.29g, 61%).
δ (CDCl₃) : 1.41 (3H, t), 1.48 (3H, t), 4.41 (2H, q), 4.62 (2H, q), 8.89 (1H, s), 9.16 (1H, s).

### Preparation 84

### 2-Ethoxy-5-nitropyridine-3-carboxylic acid

Aqueous sodium hydroxide solution (4ml, 5N, 20mmol) was added dropwise to a solution of the title compound of Preparation 83 (5.1g, 20mmol) in ethanol (100ml) and the reaction stirred at room temperature for 18 hours. The reaction mixture was concentrated under reduced pressure, the residue suspended in water (50ml) and acidified to pH 3 with hydrochloric acid. This aqueous solution was extracted with ethyl acetate (3x100ml), the combined organic layers washed with brine (100ml), dried (Na₂SO₄) and evaporated under reduced pressure to give a beige solid. The crude product was recrystallised from ethyl acetate/hexane to afford the title compound (3.32g, 78%) as beige crystals.
δ (CDCl₃) : 1.55 (3H, t), 4.78 (2H, q), 9.17 (1H, s), 9.23 (1H, s).

### Preparation 85

### 4-Amino-3-(pyridin-3-yl)-1H-pyrazole-5-carboxamide

The title compound of Preparation 77 (2.9g, 12.5mmol) was dissolved in saturated methanolic ammonia solution (50ml) and the reaction heated at 100°C for 18 hours in a sealed vessel. The cooled mixture was evaporated under reduced pressure to afford the title compound (2.53g, 99%) as a brown solid.
δ (DMSOd₆) : 3.28 (2H, s), 5.08 (2H, s), 7.43 (1H, m), 8.09 (1H, d), 8.46 (1H, d), 8.95 (1H, s).
LRMS : m/z 204 (M+1)⁺

### Preparation 86

### 4-[2-Ethoxy-5-nitropyridin-3-ylcarboxamido]-3-(pyridin-3-yl)-1H-pyrazole-5-carboxamide

A mixture of the title compounds of Preparations 84 (2.37g, 11.2mmol) and 85 (2.5g, 12.3mmol), N-ethyldiisopropylamine (3.87ml, 22.4mmol), 1-hydroxybenzotriazole hydrate (1.66g, 12.3mmol) and 1-(3-dimethylaminopropyl)-3-ethylcarbodiimide hydrochloride (2.36g, 12.3mmol) in tetrahydrofuran (60ml) was stirred at room temperature for 48 hours. The mixture was concentrated under reduced pressure, suspended in ethyl acetate (100ml), washed with brine (25ml), 2N hydrochloric acid (25ml), dried (MgSO₄) and concentrated under reduced pressure. The residue was purified by column chromatography on silica gel, using dichloromethane:methanol (97.5:2.5) as eluant to afford the title compound, (1.82g, 41%) as a yellow solid.
δ (DMSOd₆) : 1.42 (3H, t), 4.62 (2H, q), 7.42 (2H, m), 7.63 (1H, s), 7.95 (1H, s), 8.51 (1H, d), 8.66 (1H, s), 8.80 (1H, s), 9.18 (1H, d), 10.48 (1H, s), 13.78 (1H, s).
LRMS : m/z 398 (M+1)⁺

### Preparation 87

### 4-[5-Amino-2-ethoxypyridin-3-ylcarboxamido]-3-(pyridin-3-yl)-1H-pyrazole-5-carboxamide

A mixture of the title compound of Preparation 86 (1.8g, 4.53mmol) and Raney® nickel (800mg) in ethanol (100ml) was hydrogenated at 345kPa (50psi) and 50°C for 18 hours. The cooled mixture was filtered through Arbocel®, the filter pad washed well with ethanol (100ml) and the filtrate evaporated under reduced pressure to afford the title compound (1.65g, 99%) as a white solid.
LRMS : m/z 368 (M+1)⁺

### Preparation 88

### 2-n-Propoxyphenyl-3-(pyridin-2-yl)-1,6-dihydro-7H-pyrazolo[4,3-d]pyrimidin-7-one

Potassium t-butoxide (1.60g, 14.44mmol) was added to a suspension of the title compound of Preparation 80 (1.50g, 4.11mmol) in isopropanol (40ml) and the mixture heated under reflux for 5 hours, then cooled. Water (50ml) was added, the mixture neutralised using solid carbon dioxide and the resulting precipitate filtered and dried, to afford the title compound (1.26g, 88%) as a pale yellow solid.
δ (DMSOd₆) : 0.96 (3H, t), 1.75 (2H, m), 4.06 (2H, t), 7.10 (1H, m), 7.19 (1H, d), 7.39 (1H, m), 7.50 (1H, m), 7.82 (1H, d), 7.97 (1H, m), 8.48 (1H, d), 8.67 (1H, d), 11.82 (1H, br s).
LRMS : m/z 348 (M+1)⁺

### Preparation 89

### 2-n-Propoxyphenyl-3-(pyridin-3-yl)-1,6-dihydro-7H-pyrazolo[4,3-d]pyrimidin-7-one

An ice-cooled solution of the tide compound of Preparation 79 (2.5g, 6.33mmol) in methanol (100ml) was saturated with ammonia and the reaction heated at 100°C for 18 hours, then cooled and evaporated under reduced pressure. A mixture of this product, and potassium t-butoxide (1.9g, 17.0mmol) in isopropanol (40ml) was heated under reflux for 6 hours, then cooled. Water (20ml) was added, the mixture neutralised using solid carbon dioxide and the resulting precipitate filtered and dried to afford the title compound, (950mg, 43%). δ (CDCl₃) : 1.12 (3H, t), 1.99 (2H, m), 4.18 (2H, t), 7.02 (1H, d), 7.12 (1H, m), 7.40 (2H, m), 8.55 (1H, d), 8.64 (1H, d), 9.71 (1H, s), 11.32 (1H, s).

### Preparation 90

### 5-[5-Amino-2-ethoxypyridin-3-yl]-3-(pyridin-3-yl)-1,6-dihydro-7H-pyrazolo[4,3-d]pyrimidin-7-one

A mixture of the title compound of Preparation 87 (1.45g, 3.95mmol) and potassium t-butoxide (2.66g, 23.7mmol) in ethanol (70ml) was heated under reflux for 72 hours, then cooled. The reaction mixture was evaporated under reduced pressure and the residue purified by column chromatography on silica gel, using dichloromethane:methanol (97.5:2.5) as eluant to afford the title compound (1.0g, 73%) as a yellow solid.
δ (DMSOd₆) : 1.27 (3H, t), 4.27 (2H, q), 5.02 (2H, s), 7.49 (1H, m), 7.59 (1H, s), 7.65 (1H, s), 8.58 (2H, m), 9.46 (1H, s), 11.98 (1H, s), 14.48 (1H, s).
LRMS : m/z 350 (M+1)⁺

### Preparation 91

### 5-(2-n-Propoxyphenyl)-3-(pyridin-2-yl)-2-(pyridin-2-yl)methyl-2,6-dihydro-7H-pyrazolo[4,3-d]pyrimidin-7-one

Sodium hydride (63mg, 60%, 1.59mmol) was added to a solution of the title compound of Preparation 88 (500mg, 1.44mmol) in dimethylformamide (10ml), and the mixture stirred at room temperature for 45 minutes. A solution of 2-(chloromethyl)pyridine (obtained from 284mg, 1.73mmol of the hydrochloride) in dimethylformamide (5ml), was added dropwise and the reaction mixture stirred at room temperature for 18 hours. Water (2ml) was added, the mixture partitioned between ethyl acetate (25ml) and sodium bicarbonate solution (25ml) and the phases separated. The aqueous layer was extracted with ethyl acetate (2x25ml), the combined organic solutions dried (Na₂SO₄) and evaporated under reduced pressure. The residual pink solid was purified by column chromatography on silica gel, using an elution gradient of dichloromethane:methanol (100:0 to 97:3) and repeated using dichloromethane:methanol:0.88 ammonia (100:0:0 to 98:2:1) to afford the title compound (230mg, 36%) as a white solid.
δ (CDCl₃) : 1.14 (3H, t), 1.99 (2H, m), 4.18 (2H, t), 6.10 (2H, s), 6.99 (1H, d), 7.04 (1H, d), 7.12 (1H, m), 7.18 (1H, m), 7.26 (1H, m), 7.46 (1H, m), 7.56 (1H, m), 7.83 (1H, m), 8.54 (1H, d), 8.60 (1H, d), 8.69 (1H, d), 8.77 (1H, d), 11.40 (1H, s).
LRMS : m/z 440 (M+2)⁺

### Preparation 92

### 5-(2-n-Propoxyphenyl)-3-(pyridin-3-yl)-2-(pyridin-2-yl)methyl-2,6-dihydro-7H-pyrazolo[4,3-d]pyrimidin-7-one

Obtained (60%) from the title compound of Preparation 89 and 2-(chloromethyl)pyridine, using a similar procedure to that described in Preparation 91.
δ (CDCl₃) : 1.20 (3H, t), 2.04 (2H, m), 4.22 (2H, t), 6.05 (2H, s), 7.08 (1H, d), 7.19 (3H, m), 7.48 (2H, m), 7.64 (1H, m), 8.61 (3H, m), 8.74 (1H, d), 9.79 (1H, s), 11.48 (1H, s).
LRMS : m/z 439 (M+1)⁺

### Preparation 93

### 5-[2-Ethoxy-5-(4-ethylpiperazin-1-ylsulphonyl)pyridin-3-yl]-3-(pyridin-3-yl)-1H-1,6-dihydro-7H-pyrazolo[4,3-d]pyrimidin-7-one

Sodium nitrite (240mg, 3.43mmol) was added portionwise to a cooled (-20°C) solution of the title compound of Preparation 90 (750mg, 2.15mmol) in concentrated hydrochloric acid (30ml) and acetic acid (15ml), and the mixture allowed to warm to 0°C over 2 hours. The mixture was re-cooled to -20°C, liquid sulphur dioxide (9ml) and copper (II) chloride (900mg, 6.64mmol) in water (2ml) and acetic acid (10ml) were added, and the reaction mixture allowed to warm to room temperature and stirred for a further 2 hours. The mixture was poured into ice and this aqueous solution extracted with dichloromethane (3x50ml), the combined organic extracts dried (MgSO₄) and evaporated under reduced pressure to give a yellow solid. A mixture of this intermediate sulphonyl chloride, N-ethylpiperazine (1.05g, 9.16mmol) and N-ethyldiisopropylamine (1.58ml, 9.16mmol) in ethanol (10ml) was stirred at room temperature for 18 hours. The mixture was concentrated under reduced pressure and the residue partitioned between dichloromethane (20ml) and water (10ml) and the layers separated. The organic phase was extracted with aqueous citric acid solution (2x20ml), and these combined extracts neutralised using 1N sodium hydroxide solution. This aqueous solution was re-extracted with dichloromethane:methanol (90:10) (3x30ml), the combined organic extracts dried (MgSO₄) and evaporated under reduced pressure. The product was purified by column chromatography on silica gel, using dichloromethane:methanol (97.5:2.5) as eluant to afford the title compound (320mg, 29%) as a white solid.
δ (DMSOd₆) : 0.90 (3H, t), 1.30 (3H, t), 2.25 (2H, q), 2.39 (4H, m), 2.95 (4H, m), 4.48 (2H, q), 7.46 (1H, m), 8.28 (1H, s), 8.52 (2H, m), 8.62 (1H, s), 9.41 (1H, s), 12.44 (1H, s), 14.57 (1H, s).
LRMS : m/z 511 (M+1)⁺

### Preparation 94

### 2-Methyl-pyrimidine-N-oxide

A freshly prepared solution of sodium (11.5g, 0.50mol) in ethanol (170ml) was added dropwise over an hour to a suspension of hydroxylamine hydrochloride (34.75g, 0.50mol) and phenolphthalein (50mg) in ethanol (200ml) so as to maintain a colourless solution, and the reaction stirred at room temperature for 3 hours. Acetonitrile (26ml, 0.50mol) was added, and the reaction stirred for a further 2 hours at room temperature, and then at 45°C for 48 hours. The reaction mixture was filtered, and concentrated under reduced pressure to a volume of 100ml.

The solution was cooled to 0°C and the resulting precipitate filtered and dried under suction to give white crystals (9.9g). Boron trifluoride diethyl ether complex (9.5ml, 75mmol) followed by 1,1,3,3-tetramethoxypropane (11.5ml, 70mmol) were added to a solution of dimethylformamide (100ml) in toluene (100ml). 1-Hydroxyimmo-2-ethylamine (5.0g, 67.5mmol) was added and the reaction heated under reflux for 45 minutes, then cooled. The mixture was concentrated under reduced pressure and the residual brown oil partitioned between dichloromethane:methanol (80:20) (100ml) and aqueous sodium carbonate solution (100ml). The phases were separated, the aqueous layer extracted with dichloromethane:methanol (80:20) (10x50ml) and the combined organic extracts dried (MgSO₄) and concentrated under reduced pressure. The residue was purified by column chromatography on silica gel, using dichloromethane:methanol (98:2) as eluant to afford the title compound (2.5g, 34%) as an orange solid. δ (CDCl₃) : 2.74 (3H, s), 7.19 (1H, m), 8.16 (1H, d), 8.39 (1H, d).

### Preparation 95

### 2-(Chloromethyl)pyrimidine

A mixture of the title compound of Preparation 94 (2.5g, 22.7mmol) in phosphorous oxychloride (18ml, 193mmol) was heated under reflux for 2 hours, then cooled. The mixture was poured into ice and neutralised using solid sodium carbonate over 3 hours. The aqueous solution was extracted with dichloromethane (3x100ml), the combined organic extracts dried (MgSO₄) and concentrated under reduced pressure. The residual brown oil was purified by column chromatography on silica gel, using an elution gradient of dichloromethane:methanol (100:0 to 97:3) to afford the title compound (510mg, 17%). δ (CDCl₃) : 4.72 (2H, s), 7.22 (1H, m), 8.75 (2H, d).
LRMS : m/z 129 (M+1)⁺

### Preparation 96

### 4-Nitro-1H-pyrazole-5-carboxamide

Oxalyl chloride (33.3ml, 0.4mol) was added dropwise over 15 minutes to an ice-cold suspension of 4-nitro-1H-pyrazole-5-carboxylic acid (40.0g, 0.25mol) and dimethylformamide (3 drops) in dichloromethane (400ml). The mixture was allowed to warm to room temperature and stirred for 24 hours. Additional oxalyl chloride (16.7ml, 0.2mol) was added and the reaction stirred for a further 24 hours. The reaction mixture was filtered, the filtrate evaporated under reduced pressure and redissolved in tetrahydrofuran (400ml). This solution was cooled in an ice-bath, ammonia bubbled through for an hour, and the mixture purged with nitrogen for 30 minutes. The reaction mixture was concentrated under reduced pressure, the residue triturated with water, and the solid filtered and dried under vacuum to afford the title compound (34.7g, 86%) as a white solid. δ (DMSOd₆) : 7.60-8.10 (3H, m), 8.68 (1H, s).

### Preparation 97

### 2-Methyl-4-nitro-pyrazole-5-carboxamide

A mixture of the title compound of preparation 96 (35.5g, 0.22mol), cesium carbonate (79.7g, 0.24mol) and methyl iodide (34.7g, 0.24mol) in dimethylformamide (200ml) was stirred at room temperature for 4 days. The reaction mixture was concentrated under reduced pressure and the residue azeotroped with xylene. The resulting brown gum was triturated with hot ethyl acetate (6x400ml) and hot methanol/dichloromethane (4x500ml), the resulting suspensions filtered and the combined filtrates evaporated under reduced pressure. The residual brown solid was purified by column chromatography on silica gel, using an elution gradient of ethyl acetate:hexane (30:70 to 100:0) to afford the title compound, (11.5g, 31%) as a solid. δ (CDCl₃) : 4.03 (3H, s), 5.88 (1H, s), 7.80 (1H, s), 8.25 (1H, s).

### Preparation 98

### 4-Amino-2-methyl-pyrazole-5-carboxamide

A mixture of the title compound of preparation 97 (5.0g, 30.0mmol) and 10% palladium on charcoal (500mg) in methanol (200ml) was hydrogenated at 30psi (207 kPa) and 50°C for 18 hours. The cooled mixture was filtered through Arbocel® , the filter pad washed with methanol, and the combined filtrate evaporated under reduced pressure to afford the title compound, (4.2g, 100%) as a pink solid. δ (DMSOd₆) : 3.72 (3H, s), 4.60 (2H, s), 6.88 (1H, s), 7.05 (2H, m).

### Preparation 99

### 4-Amino-3-bromo-2-methyl-pyrazole-5-carboxamide

Bromine (92ml, 1.8mmol) was added to a solution of the title compound of preparation 98 (250mg, 1.8mmol) in acetic acid (10ml), and the reaction stirred for an hour at room temperature. The mixture was concentrated under reduced pressure, and the residue azeotroped with toluene. The crude product was purified by column chromatography on silica gel, using ethyl acetate:methanol:0.88 ammonia (90:10:1) as eluant, to afford the title compound (250mg, 64%). δ (DMSOd₆) : 3.76 (3H, s), 4.64 (2H, s), 7.14 (1H, s), 7.27 (1H, s)

### Preparation 100

### 3-Bromo-4-[2-ethoxy-5-(4-ethylpiperazin-1-ylsulphonyl)pyridin-3-ylcarboxamido]-2-methyl-pyrazole-5-carboxamide

A mixture of the title compounds of preparation 99 (250mg, 1.1mmol), and 68 (429mg, 1.25mmol), N-ethyldiisopropylamine (294mg, 2.3mmol) and 2-chloro-1-methylpyridinium iodide (363mg, 1.4mmol) in dichloromethane (10ml), was stirred at room temperature for 18 hours. The mixture was concentrated under reduced pressure and the residue partitioned between water (20ml) and ethyl acetate (20ml), and the phases separated. The aqueous phase was extracted with ethyl acetate (2x20ml), and the combined organic solutions dried (Na₂SO₄), and evaporated under reduced pressure. The residual brown gum was purified by column chromatography on silica gel, using an elution gradient of methanol:ethyl acetate (5:95 to 7:93) to afford the title compound, (310mg, 50%).
δ (CDCl₃) : 1.12 (3H, t), 1.60 (3H, t), 2.41 (2H, q), 2.55 (4H, m), 3.14 (4H, m), 3.96 (3H, s), 4.78 (2H, q), 5.34 (1H, s), 6.62 (1H, s), 8.67 (1H, s), 8.86 (1H, s), 10.39 (1H, s).
LRMS : m/z 546 (M+2)⁺

### Preparation 101

### 3-Bromo-5-[2-ethoxy-5-(4-ethylpiperazin-1-ylsulphonyl)pyridin-3-yl]-2-methyl-2,6-dihydro-7H-pyrazolo[4,3-d]pyrimidin-7-one

A mixture of the title compound of preparation 100 (815mg, 1.2mmol), and potassium bis(trimethylsilyl)amide (439mg, 2.2mmol) in 3-methyl-3-pentanol (60ml) was stirred at 125°C in a sealed vessel for 20 hours. The cooled reaction was evaporated under reduced pressure and the residue purified by column chromatography on silica gel, using an elution gradient of ethyl acetate:diethylamine (95:5 to 90:10) to afford the title compound, (360mg, 46%).
δ (CDCl₃) : 1.02 (3H, t), 1.60 (3H, t), 2.41 (2H, q), 2.58 (4H, m), 3.16 (4H, m), 4.18 (3H, s), 4.76 (2H, q), 8.65 (1H, s), 9.13 (1H, s), 10.77 (1H, s).
LRMS : m/z 528 (M+2)⁺

### Preparation 102

### 2-(2-Methoxyethoxy)pyridine-3-carboxylic acid

Potassium *t*-butoxide (45.0g, 0.40mol) was added portion-wise to ice-cold 2-methoxyethanol (175ml), and the resulting solution added to a suspension of 2-chloronicotinic acid (30.0g, 0.19mol) in 2-methoxyethanol (175ml). The reaction mixture was heated under reflux for 26 hours, then cooled and concentrated under reduced pressure. The residue was diluted with water (200ml), the pH of the solution adjusted to 5 using concentrated hydrochloric acid, and extracted with dichloromethane (3x). The combined organic extracts were washed with brine, dried (MgSO₄), evaporated under reduced pressure and azeotroped with toluene, to afford the title compound, (30.54g, 81%).
δ (CDCl₃) : 3.42 (3H, s), 3.80 (2H, t), 4.72 (2H, t), 7.14 (1H, m), 8.36 (1H, m), 8.45 (1H, m).
LRMS : m/z 198 (M+1)⁺

### Preparation 103

### 2-(2-Methoxyethoxy)-5-nitropyridine-3-carboxylic acid

Ammonium nitrate (21.8g, 273.0mmol) was added portion-wise to an ice-cooled solution of the title compound of preparation 102 (30.5g, 155.0mmol) in trifluoroacetic anhydride (110ml), and once addition was complete, the reaction was allowed to warm to 10°C, and initiation occurred. The reaction mixture was re-cooled using an ice-bath, and then stirred at room temperature for an hour. Tlc analysis showed starting material remaining, so the reaction was cooled in an ice-bath, additional ammonium nitrate (12.4g, 155.0mmol) was added portionwise, and the reaction stirred at room temperature for a further hour. The reaction was poured onto ice (300g), the resulting precipitate filtered, washed with water and dried under vacuum to afford the title compound, (25g, 67%).
δ (CDCl₃) : 3.44 (3H, s), 3.82 (2H, t), 4.61 (2H, t), 9.16 (1H, s), 9.21 (1H, s).
LRMS : m/z 243 (M+1)⁺

### Preparation 104

### 2-Ethoxy-5-nitropyridine-3-carboxamide

N,N-Dimethylformamide (2 drops) was added to an ice-cold solution of the title compound of preparation 84 (3.0g, 13.9mmol) and oxalyl chloride (5ml, 57.0mmol) in dichloromethane (30ml), and the reaction then stirred at room temperature for 2 hours. The reaction mixture was concentrated under reduced pressure and azeotroped with dichloromethane. The residue was dissolved in dichloromethane (30ml), the solution cooled in an ice-bath, 0.88 ammonia (5ml) added, and the reaction stirred for 15 minutes. The mixture was partitioned between dichloromethane and water and the layers separated. The organic phase was washed with aqueous saturated sodium bicarbonate solution, brine, then dried (MgSO₄) and evaporated under reduced pressure. The residual yellow solid was triturated with diethyl ether, filtered and dried to afford the title compound (2.4g, 83%).
δ (CDCl₃) : 1.56 (3H, t), 4.74 (2H, q), 6.14 (1H, br, s), 7.66 (1H, br, s), 9.18 (1H, d), 9.29 (1H, d).
LRMS: m/z 229 (M+18)⁺

### Preparation 105

### 2-(2-Methoxyethoxy)-5-nitropyridine-3-carboxamide

The title compound was obtained as a pale yellow solid (84%) from the title compound of preparation 103, following the procedure described in preparation 104.
δ (CDCl₃) : 3.43 (3H, s), 3.80 (2H, t), 4.78 (2H, t), 6.12 (1H, br, s), 7.80 (1H, br, s), 9.15 (1H, d), 9.25 (1H, d).
LRMS : m/z 264 (M+23)⁺

### Preparation 106

### 2-Ethoxy-5-nitropyridine-3-carbonitrile

Trifluoroacetic anhydride (3.46g, 16.5mmol) in dioxan (5ml) was added to an ice-cold solution of the title compound of preparation 104 (2.32g, 11.0mmol) and pyridine (2.17g, 27.5mmol) in dioxan (15ml), and the solution stirred at room temperature for 3 hours. The mixture was concentrated under reduced pressure and the residue partitioned between ethyl acetate and water. The layers were separated and the organic phase washed consecutively with hydrochloric acid (2N, 2x), aqueous saturated sodium bicarbonate solution, then brine. The solution was dried (MgSO₄) and evaporated under reduced pressure. The residue was purified by column chromatography on silica gel using an elution gradient of pentane:ethyl acetate (100:0 to 95:5) to afford the title compound (1.73g, 81%).
δ (CDCl₃) : 1.50 (3H, t), 4.63 (2H, q), 8.66 (1H, d), 9.20 (1H, d).

### Preparation 107

### 2-(2-Methoxyethoxy)-5-nitropyridine-3-carbonitrile

The title compound was prepared from the title compound of preparation 105, following a similar procedure to that described in preparation 106. The crude product was purified by trituration, and filtration from diethyl ether to give the desired product, (18.27g, 97%) as a solid.
δ (CDCl₃) : 3.42 (3H, s), 3.81 (2H, t), 4.76 (2H, t), 8.68 (1H, d), 9.20 (1H, d).

### Preparation 108

### 2-Ethoxy-5-nitropyridine-3-carboximidamide acetate

The title compound of preparation 106 (11.0g, 57.0mmol) was added "in one portion" to a cooled (-10°C) solution of ethanol saturated with HCl gas (100ml), and the reaction stirred at this temperature for 8 hours. The reaction was evaporated under reduced pressure, the residue triturated with diethyl ether, and the precipitate filtered off. The solid was partitioned between ethyl acetate and aqueous saturated sodium bicarbonate solution, and the layers separated. The organic phase was washed with aqueous saturated sodium bicarbonate solution, brine, then dried (MgSO₄), and evaporated under reduced pressure to give a white solid, 4.25g. Ammonium acetate (3.61g, 46.9mmol) was added to a solution of this intermediate imidate (8.62g) in ethanol (80ml), and the reaction heated under reflux for an hour. Tlc analysis showed starting material remaining, so additional ammonium acetate (0.5g, 6.5mmol) was added, and the reaction heated under reflux for a further 30 minutes. The cooled reaction mixture was evaporated under reduced pressure and the residue triturated with diethyl ether. The resulting solid was filtered off, and dried under vacuum to afford the title compound (8.26g).
δ (DMSOd₆) : 1.38 (3H, t), 1.77 (3H, s), 4.54 (2H, q), 8.74 (1H, d), 9.20 (1H, d).
LRMS : m/z 211 (M+1)⁺

### Preparation 109

### 2-(2-Methoxyethoxy)-5-nitropyridine-3-carboximidamide formate

The title compound was obtained as a pale brown solid (53%) from the title compound of preparation 107 and ammonium formate, in 2-methoxyethanol, following a similar procedure to that described in preparation 108.
δ (DMSOd₆) : 3.29 (3H, s), 3.73 (2H, t), 4.60 (2H, t), 8.40 (1H, s), 8.81 (1H, d), 9.24 (1H, d).
LRMS: m/z 241 (M+1)⁺

### Preparation 110

### Ethyl 2-ethoxy-1-methyl-pyrazole-4-carboxylate

Diethyl azodicarboxylate (1.3ml, 7.5mmol) was added dropwise to a solution of ethyl 2-hydroxy-1-methyl-pyrazole-4-carboxylate (Chem. Pharm. Bull; 1983; 31; 1228), (880mg, 5.0mmol), and triphenylphosphine (2.03g, 7.5mmol) in ethanol (0.3ml, 5.0mmol) and tetrahydrofuran (100ml), and the reaction stirred at room temperature for 4 hours. The mixture was concentrated under reduced pressure, the residue partitioned between dichloromethane and water, and the layers separated. The aqueous phase was extracted with dichloromethane, the combined organic solutions washed consecutively with water, 2N aqueous sodium hydroxide, water and finally brine. The solution was then dried (Na₂SO₄), and evaporated under reduced pressure. The crude product was purified by column chromatography on silica gel using dichloromethane as eluant to afford the title compound (768mg, 79%), as a crystalline solid.
δ (CDCl₃) : 1.39 (6H, m), 3.68 (3H, s), 4.10 (2H, q), 4.38 (2H, q), 6.00 (1H, s).
LRMS : m/z 199 (M+1)⁺

### Preparation 111

### Ethyl 2-ethoxy-1-methyl-3-nitro-pyrazole-4-carboxylate

Fuming nitric acid (0.43ml) was added dropwise to ice-cooled concentrated sulphuric acid (2.6ml), and the resulting solution warmed to 40°C. The title compound of preparation 110 (433mg, 2.18mmol) was added portion-wise, and the reaction mixture stirred for a further 50 minutes. The reaction was poured carefully onto ice, and the resulting mixture extracted with dichloromethane. The combined organic extracts were washed with water, and brine, then dried (Na₂SO₄), and evaporated under reduced pressure to afford the title compound, (301mg, 57%) as an orange oil.
δ (CDCl₃) : 1.40 (3H, t), 1.48 (3H, t), 3.78 (3H, s), 4.39-4.55 (4H, m).
LRMS : m/z 244 (M+1)⁺

### Preparation 112

### Ethyl 3-amino-2-ethoxy-1-methyl-pyrazole-4-carboxylate

A mixture of the title compound of preparation 111 (301mg, 1.24mmol) and 10% palladium on charcoal (60mg) in ethanol (12ml) was hydrogenated at 60 psi (414 kPa), and room temperature for 3 hours. The reaction mixture was filtered through Arbocel®, and the filtrate evaporated under reduced pressure. The residue was purified by column chromatography on silica gel using an elution gradient of dichloromethane:methanol (100:0 to 99:1) to afford the title compound, (140mg, 53%).
δ (CDCl₃) : 1.38 (6H, m), 3.72 (5H, s), 4.17 (2H, q), 4.39 (2H, q).
LRMS : m/z 214 (M+1)⁺

### Preparation 113

### 5-(2-Ethoxy-5-nitropyridin-3-yl)-2-methyl-2,6-dihydro-7H-pyrazolo[4,3-d]pyrimidin-7-one

A mixture of the title compounds of preparations 98 (3.85g, 27.5mmol) and 108 (8.26g, 30.6mmol) in 3-methyl-3-pentanol (80ml) were heated under reflux for 2½ hours, then cooled. The reaction mixture was partitioned between dichloromethane and hydrochloric acid (2N), and the resulting precipitate filtered, washed with water and diethyl ether, and dried. The filtrate was separated, and the organic layer washed with hydrochloric acid (2N), saturated aqueous sodium bicarbonate solution, brine, then dried (MgSO₄) and evaporated under reduced pressure. The residue was triturated with diethyl ether, and the resulting solid filtered and dried. The isolated solids were combined to provide the title compound (6.9g, 79%).
δ (DMSOd₆) : 1.35 (3H, t), 4.10 (3H, s), 4.54 (2H, q), 8.39 (1H, s), 8.70 (1H, d), 9.19 (1H, d), 11.92 (1H, s).
LRMS : m/z 317 (M+1)⁺
Found: C, 49.36; H, 3.82; N, 26.57. C₁₃H₁₂N₆O₄ requires C, 49.18; H, 3.77; N, 26.53%.

### Preparation 114

### 5-[2-(2-Methoxyethoxy)-5-nitropyridin-3-yl]-2-methyl-2,6-dihydro-7H-pyrazolo[4,3-d]pyrimidin-7-one

The title compound was obtained as a yellow solid from the title compounds of preparations 98 and 109, following a similar procedure to that described in preparation 113.
δ (DMSOd₆) : 3.23 (3H, s), 3.70 (2H, t), 4.10 (3H, s), 4.60 (2H, t), 8.40 (1H, s), 8.77 (1H, d), 9.18 (1H, d).

### Preparation 115

### 3-Ethoxy-5-(2-ethoxy-5-nitropyridin-3-yl)-2-methyl-2,6-dihydro-7H-pyrazolo[4,3-d]pyrimidin-7-one

A solution of the title compounds of preparations 108 (177mg, 0.66mmol) and 112 (140mg, 0.66mmol) in 3-methyl-3-pentanol (5ml) was heated at 130°C for 3 hours. The cooled reaction was partitioned between water and dichloromethane, and the layers separated. The aqueous phase was extracted with dichloromethane, the combined organic solutions washed with brine, then dried (Na₂SO₄) and evaporated under reduced pressure. The crude product was purified by column chromatography on silica gel using dichloromethane:methanol (99.5:0.5) as eluant to afford the title compound, (55mg, 18%) as a yellow solid.
δ (CDCl₃) : 1.58 (6H, m), 3.91 (3H, s), 4.80 (2H, q), 4.95 (2H, q), 9.15 (1H, d), 9.39 (1H, d), 10.54 (1H, s).
LRMS : m/z 383 (M+23)⁺

### Preparation 116

### 3-Bromo-5-(2-ethoxy-5-nitropyridin-3-yl)-2-methyl-2,6-dihydro-7H-pyrazolo[4,3-d]pyrimidin-7-one

A mixture of the title compound of preparation 113 (6.9g, 21.8mmol), bromine (1.35ml, 26.2mmol), and sodium acetate (2.7g, 32.7mmol) in acetic acid (100ml) was heated under reflux for 7 hours, then allowed to cool. Additional bromine (0.35ml, 6.8mmol) was added and the reaction stirred at room temperature for a further 18 hours. The reaction mixture was concentrated under reduced pressure and azeotroped with toluene.

The residue was partitioned between dichloromethane and water and the resulting precipitate filtered off, washed with dichloromethane, water, then diethyl ether and dried. The filtrate was separated, and the organic layer washed with aqueous saturated sodium bicarbonate solution, and brine, then dried (MgSO₄) and evaporated under reduced pressure to give a yellow solid. The isolated solids were combined, suspended in ethyl acetate, and stirred for 30 minutes. The resulting precipitate was filtered off, and dried to afford the title compound (7.66g, 89%).
δ (DMSOd₆) : 1.35 (3H, t), 4.10 (3H, s), 4.54 (2H, q), 8.70 (1H, d), 9.20 (1H, d), 12.16 (1H, s).
LRMS : m/z 394, 396 (M+1)⁺
Found: C, 39.51: H, 2.80; N, 21.27. C₁₃H₁₁BrN₆O₄ requires C, 39.63; H, 2.73; N, 21.36%.

### Preparation 117

### 3-Bromo-5-[2-(2-methoxyethoxy)-5-nitropyridin-3-yl]-2-methyl-2,6-dihydro-7H-pyrazolo[4,3-d]pyrimidin-7-one

Bromine (500µl, 10.0mmol) was added dropwise to a suspension of the title compound of preparation 114 (2.64g, 7.62mmol) and sodium acetate (1.25g, 15.2mmol) in acetic acid (45ml), and the reaction heated at 50°C for 3 hours. Additional bromine (300µl, 6.0mmol) was added dropwise, and the reaction stirred for a further 3 hours at 50°C. The cooled reaction mixture was evaporated under reduced pressure, the residue triturated with water, and the resulting precipitate filtered. The solid was washed with water and diethyl ether, then dried under vacuum, to afford the title compound, (2.05g, 63%).
δ (DMSOd₆) : 3.25 (3H, s), 3.70 (2H, t), 4.10 (3H, s), 4.60 (2H, t), 8.78 (1H, d), 9.20 (1H, d), 12.18 (1H, s).
LRMS: m/z 443 (M+18)⁺

### Preparation 118

### 5-(5-Amino-2-ethoxypyridin-3-yl)-2-methyl-2,6-dihydro-7H-pyrazolo[4,3-d]pyrimidin-7-one

Iron powder (3.7g, 66.3mmol) was added to a solution of the title compound of preparation 113 (2.91g, 9.2mmol) in acetic acid (40ml) and water (4ml), and the reaction stirred at room temperature for 18 hours. The mixture was filtered through Celite®, the filtrate concentrated under reduced pressure, and the residue partitioned between water and dichloromethane. The resulting precipitate was filtered off, washed well with methanol and dried under vacuum. The filtrate was separated, the organic phase was dried (MgSO₄), and evaporated under reduced pressure to give, when combined with the previously isolated solid, the title compound (1.05g, 40%).
δ (CDCl₃) : 1.50 (3H, t), 3.57 (2H, br, s), 4.18 (3H, s), 4.58 (2H, q), 7.80 (1H, d), 7.84 (1H, s), 8.16 (1H, d), 11.15 (1H, s).
LRMS : m/z 304 (M+18)⁺

### Preparation 119

### 5-[5-Amino-2-(2-methoxyethoxy)pyridin-3-yl]-3-bromo-2-methyl-2,6-dihydro-7H-pyrazolo[4,3-d]pyrimidin-7-one

Titanium trichloride (35ml, 15%w/v solution, 33.6mmol) was added dropwise to a solution of the title compound of preparation 117 (2.04g, 4.8mmol) in acetic acid (35ml), and the reaction stirred at room temperature for an hour. Tlc analysis showed starting material remaining, so additional titanium trichloride (2x5ml, 35% w/v solution, 10.4mmol) was added and the reaction stirred for a further 2 hours. The mixture was concentrated under reduced pressure and the residue partitioned between dichloromethane and saturated aqueous sodium bicarbonate solution. The resulting suspension was filtered through Celite®, and the filtrate separated. The aqueous phase was saturated with sodium chloride, and extracted with dichloromethane. The combined organic solutions were washed with brine, dried (MgSO₄) and evaporated under reduced pressure to afford the title compound, (1.88g, 99%).
δ (CDCl₃) : 3.36 (3H, s), 3.62 (2H, t), 3.90 (2H, s), 3.98 (3H, s), 4.42 (2H, t), 7.60 (1H, s), 7.98 (1H, s), 11.17 (1H, s).

### Preparation 120

### 5-(5-Amino-2-ethoxypyridin-3-yl)-3-ethoxy-2-methyl-2,6-dihydro-7H-pyrazolo[4,3-d]pyrimidin-7-one

A mixture of the title compound of preparation 115 (55mg, 0.15mmol) and 10% palladium on charcoal (7.5mg) in ethanol (22ml) was hydrogenated at 60psi (414 kPa) and room temperature for 2 hours. The reaction mixture was filtered through Arbocel®, and the filtrate evaporated under reduced pressure. The crude product was purified by column chromatography on silica gel using dichloromethane:methanol (93.4:6.6) as eluant to afford the title compound, (40mg, 80%).
δ (CDCl₃) : 1.50 (6H, m), 3.86 (3H, s), 4.55 (2H, q), 4.87 (2H, q), 7.77 (1H, d), 8.02 (1H, d), 11.00 (1H, s).
LRMS : m/z 331 (M+1)⁺

### Preparation 121

### 5-[2-Ethoxy-5-(4-ethylpiperazin-1-ylsulphonyl)pyridin-3-yl]-2-methyl-2,6-dihydro-7H-pyrazolo[4,3-d]pyrimidin-7-one

Sodium nitrite (380mg, 5.5mmol) was added to a cooled (-10°C) solution of the title compound of preparation 118 (1.05g, 3.7mmol) in acetic acid (16ml) and concentrated hydrochloric acid (16ml), and the solution stirred at 0°C for 2 hours. The solution was re-cooled to -30°C, liquid sulphur dioxide (11ml) added, followed by a solution of copper (II) chloride (1.5g, 11.1mmol) in water (5ml). The reaction mixture was stirred at 0°C for 30 minutes and at room temperature for an additional 2 hours. The reaction was poured onto ice, and this aqueous mixture extracted with dichloromethane. The combined organic extracts were dried (MgSO₄), and evaporated under reduced pressure. A solution of this intermediate sulphonyl chloride in dichloromethane (5ml) was cooled in ice. N-Ethylpiperazine (0.7ml, 5.55mmol) was added and the reaction stirred at room temperature for 20 hours, then evaporated under reduced pressure. The residue was suspended in aqueous saturated sodium bicarbonate solution, and extracted with ethyl acetate. The combined organic extracts were evaporated under reduced pressure to afford the title compound, (530mg, 32%).
δ (CDCl₃) : 1.02 (3H, t), 1.58 (3H, t), 2.40 (2H, q), 2.58 (4H, m), 3.14 (4H, m), 4.19 (3H, s), 4.77 (2H, q), 7.91 (1H, s), 8.62 (1H, d), 9.07 (1H, d), 10.70 (1H, br, s).
LRMS : m/z 448 (M+1)⁺

### Preparation 122

### 3-Bromo-5-[5-(4-ethylpiperazin-1-ylsulphonyl)-2-(2-methoxyethoxy)pyridin-3-yl]-2-methyl-2,6-dihydro-7H-pyrazolo[4,3-d]pyrimidin-7-one

Sodium nitrite (462mg, 6.70mmol) was added to a cooled (-10°C) solution of the title compound of preparation 119 (1.89g, 4.78mmol) in acetic acid (10ml) and concentrated hydrochloric acid (10ml) and the solution allowed to warm to 0°C over an hour. The solution was re-cooled to -15°C, liquid sulphur dioxide (15ml) and a solution of copper (II) chloride (1.92g, 14.3mmol) in water (3ml) added, and the reaction then allowed to warm to room temperature over 2 hours. The mixture was extracted with dichloromethane, the combined organic extracts dried (MgSO₄), concentrated under reduced pressure and azeotroped with toluene. The intermediate sulphonyl chloride was dissolved in dichloromethane (20ml), triethylamine (1.45g, 14.3mmol) and N-ethylpiperazine (1.1g, 9.6mmol) were added, and the reaction was stirred at room temperature for an hour. The mixture was washed with aqueous sodium bicarbonate solution, and brine, then dried (MgSO₄) and evaporated under reduced pressure. The crude product was purified by column chromatography on silica gel using an elution gradient of dichloromethane:methanol (100:0 to 97:3) to afford the title compound, (1.0g, 38%).
δ (CDCl₃) : 1.02 (3H, t), 2.40 (2H, q), 2.57 (4H, m), 3.17 (4H, m), 3.58 (3H, s), 3.85 (2H, t), 4.17 (3H, s), 4.78 (2H, t), 8.62 (1H, d), 9.03 (1H, d), 10.95 (1H, s).

### Preparation 123

### 5-[2-Ethoxy-5-(4-ethylpiperazin-1-ylsulphonyl)pyridin-3-yl]-2-methyl-3-nitro-2,6-dihydro-7H-pyrazolo[4,3-d]pyrimidin-7-one

Ammonium nitrate (115mg, 1.45mmol) was added to a solution of the compound of preparation 121 (430mg, 0.96mmol) in trifluoroacetic anhydride (20ml), and the reaction stirred at room temperature for 18 hours. Tlc analysis showed starting material remaining, so additional ammonium nitrate (115mg, 1.45mmol) was added, and the reaction stirred for a further 3 hours. The reaction mixture was carefully diluted with water, then basified to pH 8 using sodium carbonate, and extracted with ethyl acetate. The combined organic extracts were dried (MgSO₄), and evaporated under reduced pressure. The residue was re-partitioned between dichloromethane and hydrochloric acid (2N), and the phases separated. The aqueous phase was basified to pH 8 using sodium carbonate, and this aqueous solution re-extracted with dichloromethane. These combined organic extracts were dried (MgSO₄) and evaporated under reduced pressure. The crude product was purified by column chromatography on silica gel using an elution gradient of dichloromethane:methanol (100:0 to 90:10) to afford the title compound, (460mg, 97%).
δ (CDCl₃) : 1.02 (3H, t), 1.60 (3H, t), 2.42 (2H, q), 2.58 (4H, m), 3.19 (4H, m), 4.55 (3H, s), 4.80 (2H, q), 8.74 (1H, d), 9.22 (1H, d), 11.08 (1H, s).
LRMS : m/z 493 (M+1)⁺

### Preparation 124

### 5-[2-Ethoxy-5-nitropyridin-3-yl]-2-methyl-3-(4-trifluoromethoxyphenyl)-2,6-dihydro-7H-pyrazolo[4,3-d]pyrimidin-7-one

A mixture of the title compound of preparation 116 (250mg, 0.64mmol), 4-trifluoromethoxybenzyl boronic acid (157mg, 0.76mmol) and potassium carbonate (176mg, 1.27mmol) in dioxan (8ml) and water (2ml) was de-gassed and placed under an atmosphere of nitrogen. Tetrakis(triphenylphosphine)palladium (0) (75mg, 0.065mmol) was added, and the reaction heated under reflux for 3 hours. The cooled mixture was partitioned between water and dichloromethane and the phases separated. The aqueous layer was extracted with dichloromethane (2x), and the combined organic solutions washed with brine, dried (MgSO₄), and evaporated under reduced pressure. The residue was purified by medium pressure column chromatography on silica gel using an elution gradient of dichloromethane:methanol (99.8:0.2 to 99.4:0.6) to afford the title compound, (168mg, 55%) as a yellow solid.
δ (CDCl₃) : 1.61 (3H, t), 4.20 (3H, s), 4.81 (2H, q), 7.48 (2H, d), 7.75 (2H, d), 9.16 (1H, d), 9.42 (1H, d), 10.72 (1H, s).
LRMS : m/z 499 (M+23)⁺

### Preparations 125 to 133

The compounds of the general structure: 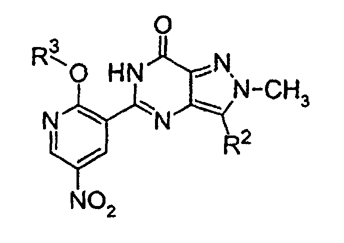 were prepared from the corresponding bromides and boronic acids, following a similar procedure to that described in preparation 124.

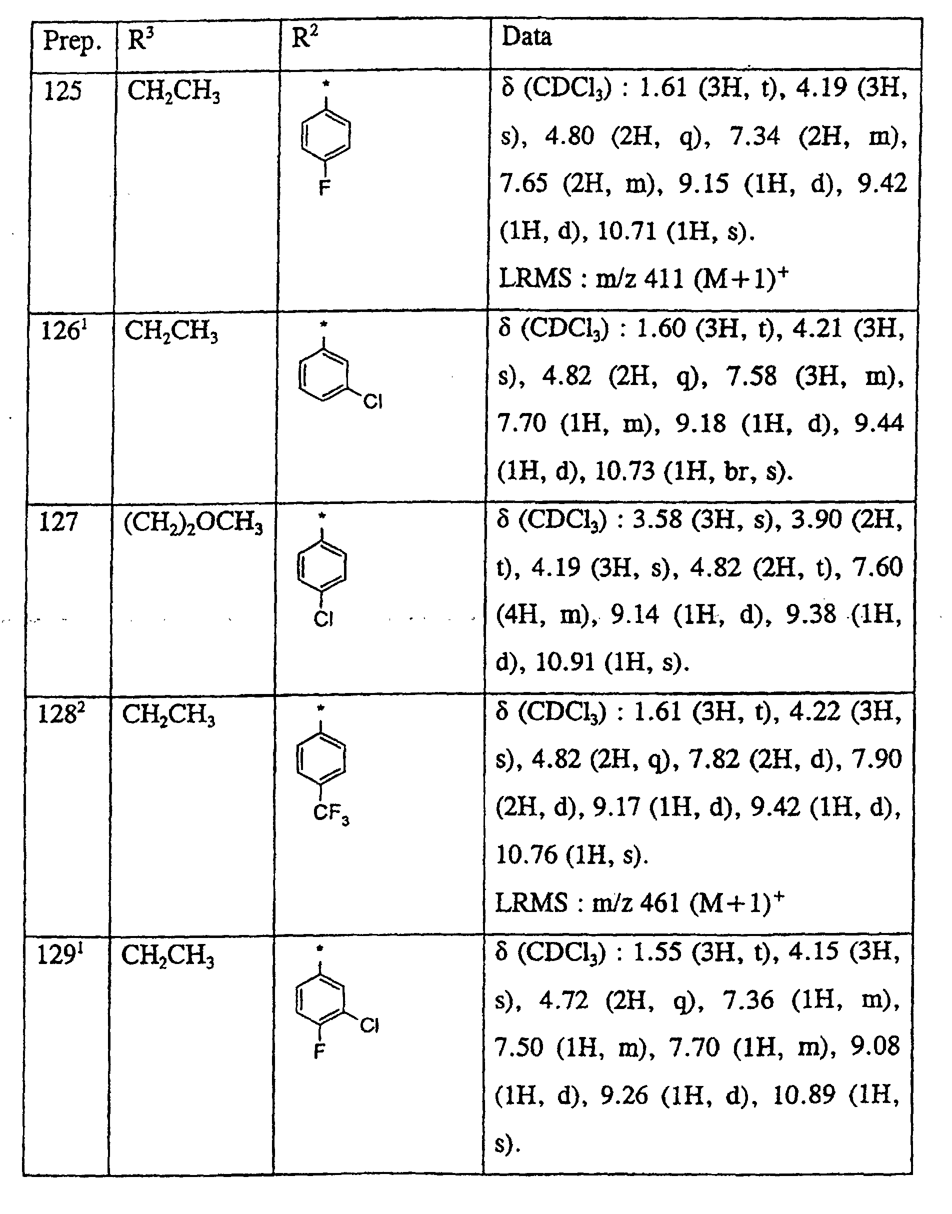

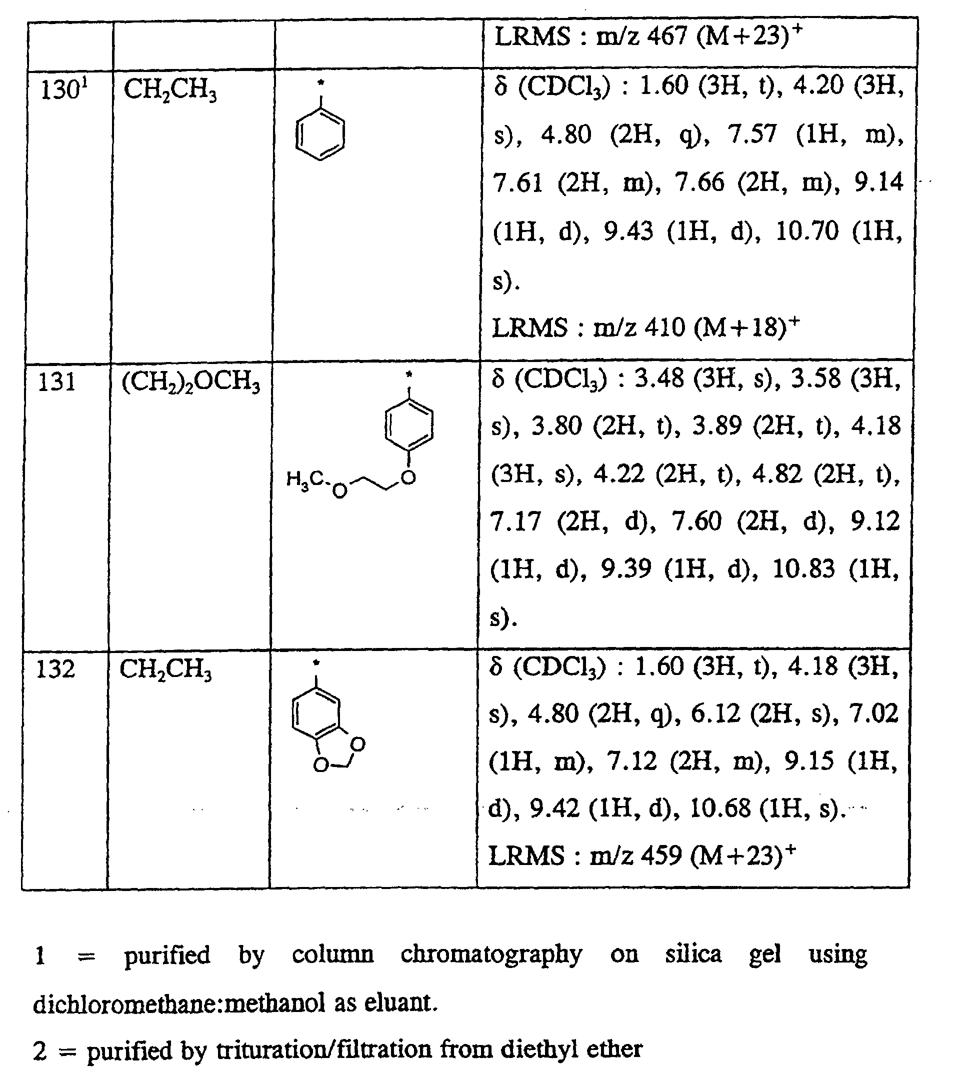

### Preparation 133

### 5-[5-Amino-2-ethoxypyridin-3-yl]-3-(4-fluorophenyl)-2-methyl-2,6-dihydro-7H-pyrazolo[4,3-d]pyrimidin-7-one

10% Palladium on charcoal (20mg) was added to a solution of the title compound of preparation 125 (86mg, 0.21mmol) in ethanol (50ml) and water (1ml), and the mixture hydrogenated at 60 psi (414 kPa) and 50°C for 3 hours. The reaction mixture was filtered through Arbocel®, the filtrate evaporated under reduced pressure, and azeotroped with dichloromethane. The residue was triturated with diethyl ether, filtered and dried to afford the title compound, (63mg, 79%).
δ (CDCl₃) : 1.40 (3H, t), 3.75 (2H, br, s), 4.06 (3H, s), 4.45 (2H, q), 7.19 (2H, m), 7.58 (2H, m), 7.64 (1H, d), 7.99 (1H, d), 11.14 (1H, s).
LRMS : m/z 381 (M+1)⁺

### Preparation 134

### 5-[5-Amino-2-ethoxypyridin-3-yl]-2-methyl-3-(4-trifluoromethylphenyl)-2,6-dihydro-7H-pyrazolo[4,3-d]pyrimidin-7-one

The title compound was prepared from the title compound of preparation 128, following the procedure described in preparation 133. The crude product was purified by column chromatography on silica gel using an elution gradient of dichloromethane:methanol (98:2 to 95:5) to afford the desired product (90mg, 44%).
δ (CDCl₃) : 1.56 (3H, t), 3.58 (2H, br, s), 4.21 (3H, s), 4.59 (2H, q), 7.79 (1H, d), 7.83 (4H, m), 8.12 (1H, d), 11.28 (1H, s).
LRMS : m/z 432 (M+2)⁺

### Preparation 135

### 5-[5-Amino-2-ethoxypyridin-3-yl]-2-methyl-3-phenyl-2,6-dihydro-7H-pyrazolo[4,3-d]pyrimidin-7-one

The title compound was obtained as a solid (75%) from the title compound of preparation 130, following a similar procedure to that described in preparation 133.
δ (CDCl₃) : 1.54 (3H, t), 3.56 (2H, br, s), 4.18 (3H, s), 4.58 (2H, q), 7.53 (1H, m), 7.60 (2H, m), 7.67 (2H, m), 7.77 (1H, d), 8.15 (1H, d), 11.22 (1H, s).
LRMS : m/z 363 (M+1)⁺

### Preparation 136

### 5-[5-Amino-2-ethoxypyridin-3-yl]-3-(3-chlorophenyl)-2-methyl-2,6-dihydro-7H-pyrazolo[4,3-d]pyrimidin-7-one

Acetic acid (5ml) was added to a mixture of the title compound of preparation 126 (250mg, 0.59mmol) and iron powder (328mg, 5.86mmol) in water (300µl) and the reaction stirred at room temperature for 2 hours. The reaction mixture was filtered through Celite®, and the filtrate evaporated under reduced pressure. The crude product was purified by column chromatography on silica gel using dichloromethane:methanol (95:5) as eluant to afford the title compound, (203mg, 87%) as a brown solid.
δ (CDCl₃) : 1.56 (3H, t), 4.20 (3H, s), 4.60 (2H, q), 7.55 (3H, m), 7.74 (2H, m), 8.14 (1H, m), 11.38 (1H, s).
LRMS : m/z 397 (M+1)⁺

### Preparation 137

### 5-[5-Amino-2-ethoxypyridin-3-yl]-3-(3-chloro-4-fluorophenyl)-2-methyl-2,6-dihydro-7H-pyrazolo[4,3-d]pyrimidin-7-one

A suspension of the title compound of preparation 129 (125mg, 0.28mmol) in ethanol (4ml) was added to a mixture of iron powder (47mg, 0.84mmol) and ammonium chloride (75mg, 1.40mmol) in water (1.5ml), and the reaction heated under reflux for 4 hours. The hot solution was filtered through Arbocel®, and washed through well with hot ethanol.

The filtrate was evaporated under reduced pressure to give the title compound as a yellow solid (35mg, 30%). The Arbocel® filter pad was suspended in a solution of dichloromethane:ethanol (1:1), the mixture stirred for a minute, and the supernatant decanted off. This was repeated several times and the combined solutions filtered, and the filtrate evaporated under reduced pressure to afford an additional (46.4mg, 40%) of the title compound.
δ (CDCl₃) : 1.56 (3H, m), 3.57 (2H, br, s), 4.18 (3H, s), 4.58 (2H, q), 7.38 (1H, m), 7.57 (1H, m), 7.78 (2H, m), 8.12 (1H, d), 11.27 (1H, s).
LRMS : m/z 415 (M+1)⁺

### Preparation 138

### 5-[5-Amino-2-ethoxypyridin-3-yl]-2-methyl-3-(4-trifluoromethoxyphenyl)-2,6-dihydro-7H-pyrazolo[4,3-d]pyrimidin-7-one

The title compound was obtained as a yellow solid (86%) from the title compound of preparation 124, following a similar procedure to that described in preparation 137.
δ (CDCl₃) : 1.54 (3H, t), 3.59 (2H, br, s), 4.19 (3H, s), 4.58 (2H, q), 7.42 (2H, d), 7.74 (2H, d), 7.79 (1H, d), 8.14 (1H, d), 11.25 (1H, d).
LRMS : m/z 469 (M+23)⁺

### Preparation 139

### 5-[5-Amino-2-ethoxypyridin-3-yl]-3-(1,3-benzodioxol-5-yl)-2-methyl-2,6-dihydro-7H-pyrazolo[4,3-d]pyrimidin-7-one

The title compound was obtained as a yellow solid (65%) from the title compound of preparation 132, following a similar procedure to that described in preparation 138.
δ (CDCl₃) : 1.55 (3H, m), 3.55 (2H, s), 4.16 (3H, s), 4.58 (2H, q), 6.09 (2H, s), 7.01 (1H, d), 7.14 (2H, m), 7.78 (1H, m), 8.16 (1H, m), 11.20 (1H, s).
LRMS : m/z 407 (M+1)⁺

### Preparation 140

### 5-[5-Amino-2-ethoxypyridin-3-yl]-3-[4-(2-methoxyethoxy)phenyl]-2-methyl-2,6-dihydro-7H-pyrazolo[4,3-d]pyrimidin-7-one

Titanium trichloride (2.9ml, 15% w/v aqueous solution, 3.0mmol) was added to a solution of the title compound of preparation 131 (200mg, 0.40mmol) in acetic acid (4ml) and the reaction stirred at room temperature for an hour. Tlc analysis showed starting material remaining, so additional titanium trichloride (1ml, 15% w/v aqueous solution, 0.97mmol) was added and the reaction stirred for a further 30 minutes. The reaction mixture was concentrated under reduced pressure and the residue partitioned between dichloromethane and saturated aqueous sodium bicarbonate solution. The phases were separated and the aqueous layer filtered to remove titanium residues and this filtrate extracted with dichloromethane (2x). The combined organic solutions were washed with brine, dried (MgSO₄), and evaporated under reduced pressure to afford the title compound (87mg, 46%). The filtered titanium residues were triturated with a dichloromethane:methanol (95:5) solution, this solution decanted off and evaporated under reduced pressure to provide an additional (57mg, 30%) of the title compound.
δ (CDCl₃) : 3.49 (3H, s), 3.55 (5H, s), 3.80 (4H, m), 4.16 (3H, s), 4.22 (2H, t), 4.61 (2H, t), 7.14 (2H, d), 7.59 (2H, d), 7.75 (1H, d), 8.05 (1H, d), 11.23 (1H, s).
LRMS : m/z 467 (M+1)⁺

### Preparation 141

### 5-[5-Amino-2-ethoxypyridin-3-yl]-3-(4-chlorophenyl)-2-methyl-2,6-dihydro-7H-pyrazolo[4,3-d]pyrimidin-7-one

The title compound was obtained (60%) from the title compound of preparation 127, following a similar procedure to that described in preparation 140.
δ (CDCl₃) : 3.57 (3H, s), 3.82 (2H, t), 4.18 (3H, s), 4.61 (2H, t), 7.58 (2H, d), 7.62 (2H, d), 7.77 (1H, d), 8.04 (1H, d), 11.30 (1H, s).
LRMS : m/z 427 (M+1)⁺

### Preparation 142

### 1-Iodo-4-(2-methoxyethoxy)benzene

Triphenylphosphine (2.8g, 10.7mmol) was added to an ice-cold solution of 4-iodophenol (2.2g, 10.0mmol) and 2-methoxyethanol (0.79ml, 10.0mmol) in tetrahydrofuran (10ml). A solution of diethyl azodicarboxylate (1.88ml, 11.5mmol) in tetrahydrofuran (10ml) was then added dropwise, and the reaction stirred at room temperature for 18 hours. The mixture was evaporated under reduced pressure, the residue partitioned between dichloromethane and hydrochloric acid (2N) and the phases separated. The organic layer was washed with brine, dried (MgSO₄) and evaporated under reduced pressure. The residue was triturated with diethyl ether, the resulting suspension filtered and the filtrate concentrated under reduced pressure. The crude product was purified by column chromatography on silica gel using pentane:dichloromethane (75:25) as eluant to give the title compound, (2.0g, 71 %) as an oil.
δ (CDCl₃) : 3.42 (3H, s), 3.75 (2H, t), 4.08 (2H, t), 6.70 (2H, d), 7.56 (2H, d).

### Preparation 143

### 2-Amino-5-iodopyridine

A mixture of 2-aminopyridine (7.0g, 74.4mmol), periodic acid (14.9mmol), iodine (7.59g, 30.0mmol) and concentrated sulphuric acid (1.4ml) in water (9ml) and acetic acid (45ml) was heated at 80°C for 4 hours, and at room temperature for a further 18 hours. The reaction was poured into 10% aqueous sodium thiosulphate solution (200ml), and the mixture extracted with diethyl ether. The combined organic extracts were washed with aqueous sodium hydroxide solution (2N), brine, then dried (K₂CO₃) and evaporated under reduced pressure. The crude product was purified by medium pressure column chromatography on silica gel using an elution gradient of pentane:ethyl acetate (84:16 to 75:25) to give the title compound.
δ (DMSOd₆) : 6.78 (1H, d), 8.02 (1H, d), 8.20 (1H, s).

### Preparation 144

### 2-(Azetidin-1-yl)-5-bromopyridine hydrochloride

Azetidine hydrochloride (3.0g, 32.1mmol) was added to a solution of sodium (0.73g, 31.7mmol) in ethanol (25ml) and the solution stirred vigorously for an hour. 2,5-Dibromopyridine (5.0g, 21.1mmol) was then added and the reaction mixture heated at 100°C for 10 hours in a sealed vessel, and then at 120°C for a further 10 hours. The cooled mixture was concentrated under reduced pressure and the residue was partitioned between water and ethyl acetate. The layers were separated, the aqueous phase was extracted with ethyl acetate (3x), and the combined organic extracts washed with brine, dried (MgSO₄), and evaporated under reduced pressure. The crude product was purified by medium pressure column chromatography on silica gel, using dichloromethane:pentane (66:34) as eluant to give the title compound, (900mg, 17%) as white crystals.
δ (CDCl₃) : 2.39 (2H, m), 4.00 (4H, m), 6.17 (1H, d), 7.48 (1H, dd), 8.16 (1H, d).
LRMS : m/z 213, 215 (M+1)⁺

### Preparation 145

### 4-Bromo-1 -ethylpyrazole

A mixture of 4-bromopyrazole (4.25g, 28.9mmol), cesium carbonate (18.8g, 57.8mmol) and ethyl bromide (3.24ml, 43.3mmol) in acetonitrile (40ml) was stirred at room temperature under a nitrogen atmosphere for 72 hours. The reaction mixture was concentrated under reduced pressure at room temperature, and the residue triturated with diethyl ether. The suspension was filtered, the solid washed well with diethyl ether, and the combined filtrates evaporated under reduced pressure at room temperature, to afford the title compound (3.2g, 63%).
δ (CDCl₃) : 1.43 (3H, t), 4.15 (2H, q), 7.39 (1H, s), 7.42 (1H, s).
LRMS : m/z 175, 177 (M+1)⁺

### Preparation 146

### 4-(2-Methoxyethoxy)phenyl boronic acid

*n*-Butyllithium (5.17ml, 1.6M in hexanes, 8.27mmol) was added dropwise to a cooled (-78°C) solution of the title compound from preparation 142 (2.0g, 7.19mmol) in tetrahydrofuran (10ml), and the solution stirred for 10 minutes. Triisopropyl borate (2.4ml, 10.4mmol) was added dropwise and the reaction allowed to warm to room temperature over 3 hours. Hydrochloric acid (2N) was added and the mixture extracted with diethyl ether (4x). The combined organic extracts were washed with brine, dried (MgSO₄) and evaporated under reduced pressure. The residue was triturated with diethyl ether:pentane (1:1), the resulting solid filtered off and the filtrate evaporated under reduced pressure. The isolated solid was redissolved in diethyl ether, treated with charcoal, this suspension filtered and the filtrate evaporated under reduced pressure to give the title compound, (256mg, 18%) as a yellow solid. The remaining crude product was purified by column chromatography on silica gel using pentane:diethyl ether (50:50) as eluant to afford additional title compound, (150mg, 10%).
δ (DMSOd₆) : 3.27 (3H, s), 3.62 (2H, t), 4.08 (2H, t), 6.85 (2H, d), 7.70 (2H, d).

### Preparation 147

### 6-(Methylamino)pyridin-3-yl boronic acid hydrochloride

*n*-Butyllithium (10.8ml, 1.6M in hexanes, 17.3mmol) was added dropwise to a cooled (-70°C) solution of 5-bromo-2-methylaminopyridine (J. Org. Chem. 1983; 48; 1064) (1.5g, 8.02mmol) in tetrahydrofyran (20ml), and the solution stirred for 30 minutes. A solution of triisopropyl borate (2.77ml, 12.0mmol) in tetrahydrofuran (4ml) was added dropwise, and the reaction then allowed to warm to room temperature over 2 hours. Additional triisopropyl borate (1.85ml, 8.02mmol) was added and the mixture stirred for a further hour. The reaction was quenched by the addition of hydrochloric acid (2N), and the mixture then evaporated under reduced pressure. The residue was suspended in water, washed with diethyl ether, and the aqueous solution evaporated under reduced pressure. The residue was purified by reverse phase column chromatography on polystyrene gel, using an elution gradient of water:methanol (100:0 to 80:20) to give the title compound, (140mg, 9%) as a white solid.
δ (DMSOd₆) : 2.95 (3H, d), 7.00 (1H, d), 8.03 (1H, d), 8.21 (1H, s), 8.41 (2H, s).
LRMS : m/z 152 (M+1)⁺

### Preparation 148

### 6-(Dimethylamino)pyridin-3-yl boronic acid dihydrochloride

*n*-Butyllithium (5.3ml, 1.6M in hexanes, 8.5mmol) was added dropwise to a cooled (-70°C) solution of 5-bromo-2-(dimethylamino)pyridine (J. Org. Chem. 1983; 48; 1064) (1.5g, 7.46mmol) in tetrahydrofyran (20ml), and the solution stirred for 30 minutes. A solution of triisopropyl borate (2.57ml, 11.2mmol) in tetrahydrofuran (4ml) was added dropwise, and the reaction then allowed to warm to room temperature over 3 hours. The reaction was quenched by the addition of hydrochloric acid (2N), and the mixture then evaporated under reduced pressure. The residue was crystallised from methanol:diethyl ether to afford the title compound, (800mg, 45%) as an off-white solid.
δ (DMSOd₆) : 3.20 (6H, s), 7.18 (1H, d), 8.18 (2H, m).

### Preparation 149

### 5-Methyl-2-(tri-n-butylstannyl)pyridine

*n*-Butyllithium (12.8ml, 2.5M in hexanes, 32.6mmol) was added dropwise to a cooled (-78°C) solution of 2-bromo-5-methylpyridine (5.0g, 29.1mmol), and the solution stirred for an hour. Tri-n-butyltin chloride (9.5ml, 34.9mmol) was then added and the reaction allowed to warm to room temperature, and stirred for 18 hours. The reaction mixture was evaporated under reduced pressure and the residue purified by column chromatography on silica gel using an elution gradient of pentane:ethyl acetate (100:0 to 90:10) to give the title compound, (6.5g, 58%) as a yellow oil.
δ (CDCl₃) : 0.78-1.68 (m, 27H), 2.25 (3H, s), 7.24 (2H, m), 8.58 (1H, m).

### Preparation 150

### 2-Ethyl-5-(tri-n-butylstannyl)pyridine

*n*-Butyllithium (2ml, 1.6M in hexanes, 3.22mmol) was added dropwise to an ice-cooled solution of diisopropylamine (0.45ml, 3.22mmol) in tetrahydrofuran (6ml) under a nitrogen atmosphere, and the solution stirred for an hour. Tri-*n*-butyltin hydride (0.79ml, 2.96mmol) was added and the solution stirred for a further 2 hours, and then cooled to -78°C. A solution of 5-bromo-2-ethylpyridine (WO 97/01552) (500mg, 2.69mmol) in tetrahydrofruan (4ml) was then added dropwise, and once addition was complete, the reaction was allowed to warm to room temperature, and stirred for 18 hours. The reaction mixture was concentrated under reduced pressure and the residue partitioned between dichloromethane and aqueous ammonium chloride solution. The layers were separated, the organic phase dried (MgSO₄) and evaporated under reduced pressure. The crude product was purified by column chromatography on silica gel using an elution gradient of pentane:ethyl acetate (100:0 to 90:10) to afford the title compound, (210mg, 19%) as a yellow oil.
LRMS : m/z 397 (M+1)⁺

### Preparation 151

### 2-(Tri-n-butylstannyl)pyrazine

*n*-Butyllithium (30.0ml, 1.6M in hexanes, 48.0mmol) was added dropwise to a cooled (-40°C) solution of diisopropylamine (7ml, 50.1mmol) in tetrahydrofuran (30ml), so as to maintain the temperature below -30°C. Once addition was complete, the solution was allowed to warm to room temperature for 2 minutes, then re-cooled to -70°C. Tri-n-butyltin hydride (12ml, 45.8mmol) was added dropwise over 10 minutes, and once addition was complete, the reaction was stirred at -60°C for 2 hours. A solution of 2-chloropyrazine (5.0g, 43.7mmol) in tetrahydrofuran (5ml) was added, and the reaction allowed to warm to room temperature. Aqueous ammonium chloride solution was added to quench the reaction, followed by dilution with ethyl acetate. The resulting suspension was filtered through Celite®, and the filtrate separated. The organic phase was washed with brine, dried (MgSO₄), and evaporated under reduced pressure. The residual oil was purified by column chromatography on silica gel using an elution gradient of pentane:ethyl acetate (100:0 to 90:10) to afford the title compound, (800mg, 5%) as a yellow oil.
δ (CDCl₃) : 0.88 (3H, t), 1.18 (6H, m), 1.22-1.40 (6H, m), 1.58 (6H, m), 8.38 (1H, d), 8.55 (1H, d), 8.70 (1H, s).

### Preparation 152

### 2-Chloro-5-(tri-n-butylstannyl)pyrimidine

*n*-Butyllithium (31.0ml, 1.6M in hexanes, 49.0mmol) was added dropwise to a cooled (-78°C) solution of diisopropylamine (6.9ml, 49.0mmol) in tetrahydrofuran (35ml), and the resulting solution stirred for 30 minutes. Tri-n-butyltin hydride (13.4ml, 49.0mmol) was then added, and the reaction stirred for 2 hours at -78°C. A solution of 5-bromo-2-chloropyrimidine (J. Chem. Soc. Chem. Comm. 1996; 2719), (8.0g, 41.0mmol) in tetrahydrofuran (10ml) was added, and the reaction allowed to warm to room temperature, and stirred for 14 hours. The reaction mixture was concentrated under reduced pressure, and the residue triturated with a solution of ethyl acetate:hexane (50:50). The resulting suspension was filtered through silica gel and the filtrate concentrated under reduced pressure. The crude product was purified by column chromatography on silica gel using an elution gradient of pentane:ethyl acetate (100:0 to 95:5) to afford the title compound, (1.1g, 5.5%) as a yellow oil.
δ (CDCl₃) : 0.89 (9H, m), 1.15 (6H, m), 1.34 (6H, m), 1.52 (6H, m), 8.56 (2H; s).
LRMS : m/z 402, 404 (M+1)⁺

### Preparation 153

### 5-(Tri-n-butylstannyl)-2-pyrimidinylamine

A solution of the title compound from preparation 152 (435mg, 1.08mmol) in saturated methanolic ammonia (10ml) was heated at 50°C for 48 hours in a sealed vessel. The reaction was concentrated under reduced pressure, re-suspended in dichloromethane and the resulting precipitate filtered off, and the filtrate concentrated under reduced pressure. The crude product was purified by column chromatography on silica gel using pentane:ethyl acetate (97:3) as eluant to afford the title compound, (182mg, 44%) as a yellow solid.
δ (CDCl₃) : 0.80-1.72 (27H, m), 5.04 (2H, s), 8.24 (2H, m).
LRMS : m/z 386 (M+1)⁺

### Preparation 154

### 6-(Trimethylstannyl)imidazo[1,2-a]pyridine

A solution of 6-bromo-imidazo[1,2-a]pyridine (Chem. Pharm. Bull. 39; 6; 1991; 1556) (500mg, 2.55mmol) and hexamethylditin (919mg, 2.81mmol) in dioxan (8ml) was de-gassed and placed under an atmosphere of nitrogen. Tetrakis(triphenylphosphine)palladium (0) (147mg, 0.13mmol) was added, and the reaction heated under reflux for 5 hours. The cooled mixture was partitioned between 10% aqueous potassium fluoride solution and ethyl acetate and the layers separated. The organic phase was dried (MgSO₄) and evaporated under reduced pressure. The crude product was purified by column chromatography on silica gel using dichloromethane:methanol (90:10) as eluant to afford the title compound, (620mg, 87%) as an oil.
δ (CDCl₃) : 0.32 (9H, s), 7.18 (1H, d), 7.48-7.62 (3H, m), 8.07 (1H, s).
LRMS: m/z 281 (M+1)⁺

### Preparation 155

### 1-Ethyl-4-(tri-n-butylstannyl)pyrazole

*t*-Butyllithium (14.0ml, 1.7M in pentane, 23.8mmol) was added dropwise to a cooled (-78°C) solution of the title compound of preparation 145 (2.0g, 11.4mmol) in tetrahydrofuran (30ml) and diethyl ether (30ml), under a nitrogen atmosphere. The solution was stirred for 90 minutes, then tri-*n*-butyltin chloride (3.7ml, 13.7mmol) was added, and the reaction allowed to warm to room temperature, and stirred for 18 hours. The reaction mixture was concentrated under reduced pressure and the residue partitioned between water and dichloromethane. The layers were separated, and the organic phase dried (MgSO₄) and evaporated under reduced pressure. The crude product was purified by column chromatography on silica gel using an elution gradient of pentane:ethyl acetate (100:0 to 90:10) to afford the title compound (1.7g, 39%).
δ (CDCl₃) : 0.88 (9H, t), 0.99 (6H, t), 1.32 (6H, m), 1.50 (9H, m), 4.19 (2H, q), 7.24 (1H, s), 7.41 (1H, s).
LRMS : m/z 387 (M+2)⁺

### Preparation 156

### 4-[2-Ethoxy-5-(4-ethylpiperazin-1-ylsulphonyl)pyridin-3-ylcarboxamido]-3-methoxycarbonyl-2-methyl-pyrazole-5-carboxamide

The title compound of preparation 52 (9.0g, 45.0mmol) was added to a suspension of the title compound of preparation 69 (19.9g, 50.0mmol) in dichloromethane (200ml), and the mixture cooled in an ice-bath. Triethylamine (21ml, 150.0mmol) was added dropwise over 30 minutes, and once addition was complete, the reaction was stirred at room temperature for 20 hours. The reaction mixture was washed with aqueous saturated sodium bicarbonate solution, and water, then dried (Na₂SO₄) and evaporated under reduced pressure. The residual solid was triturated with ethanol, filtered and dried to afford the title compound (16.0g, 68%).
δ (CDCl₃) : 1.01 (3H, t), 1.60 (3H, t), 2.40 (2H, q), 2.52 (4H, m), 3.08 (4H, m), 3.92 (3H, s), 4.08 (3H, s), 4.80 (2H, q), 5.38 (1H, s), 6.67 (1H, s), 8.65 (1H, d), 8.82 (1H, d), 11.01 (1H, s).
LRMS : m/z 525 (M+2)⁺

### Preparation 157

### 5-[2-Ethoxy-5-(4-ethylpiperazin-1-ylsulphonyl)pyridin-3-yl]-2-methyl-7-oxo-2,6-dihydro-[4,3-d]pyrimidine-3-carboxylic acid

A mixture of the title compound from preparation 156 (16.0g, 30.4mmol) and potassium bis(trimethylsilyl)amide (25.0g, 125.2mmol) in ethanol (900ml) was heated at 110°C in a sealed vessel for 18 hours. The cooled mixture was diluted with sufficient water to obtain a solution, then acidified to pH 3 using hydrochloric acid. The resulting precipitate was filtered slowly, and dried. The solid was suspended in water (200ml), and basified to pH 12 using 0.88 ammonia solution. The mixture was heated to reflux, then cooled in ice and the resulting precipitate filtered and dried to afford the title compound (10.1g, 68%).
δ (DMSOd₆) : 0.96 (3H, t), 1.35 (3H, t), 2.40 (2H, q), 2.50 (4H, m), 2.99 (4H, m), 4.30 (3H, s), 4.48 (2H, q), 8.24 (1H, s), 8.63 (1H, s), 12.11 (1H, br, s).
LRMS : m/z 492 (M+1)⁺

### Preparation 158

### 5-[2-Ethoxy-5-(4-ethylpiperazin-1-ylsulphonyl)pyridin-3-yl]-2-methyl-7-oxo-2,6-dihydro-[4,3-d]pyrimidine-3-carboxylic acid chloride hydrochloride

Oxalyl chloride (230µl, 2.6mmol) was added to a suspension of the title compound of preparation 157 (500mg, 1.02mmol) and N,N-dimethylformamide (20µl) in dichloromethane (40ml) and the reaction stirred at room temperature for 2½ hours. The reaction mixture was evaporated under reduced pressure, azeotroped with toluene and dried under vacuum to afford the title compound, (450mg), as a pale yellow solid.
δ (DMSOd₆) : 1.20 (3H, t), 1.35 (3H, t), 2.94 (2H, m), 3.10 (4H, m), 3.52 (2H, m), 3.82 (2H, m), 4.35 (3H, s), 4.52 (2H, q), 8.38 (1H, s), 8.78 (1H, s), 11.00 (1H, s).

### Preparation 159

### 3-Amino-5-[2-ethoxy-5-(4-ethylpiperazin-1-ylsulphonyl)pyridin-3-yl]-2-methyl-2,6-dihydro-7H-pyrazolo[4,3-d]pyrimidin-7-one

A mixture of the title compound of preparation 123 (55mg, 0.11mmol) and 10% palladium on charcoal (6mg) in ethanol (5ml), was hydrogenated at 50 psi (345 kPa) and room temperature for 4 hours. The mixture was filtered through Arbocel®, and the filtrate evaporated under reduced pressure. The crude product was purified by column chromatography on silica gel using an elution gradient of dichloromethane:methanol (100:0 to 90:10) to afford the title compound, (28mg, 55%).
δ (CDCl₃) : 1.02 (3H, t), 1.58 (3H, t), 2.41 (2H, q), 2.57 (4H, m), 3.13 (4H, m), 3.95 (3H, s), 4.21 (2H, s), 4.75 (2H, q), 8.60 (1H, d), 9.00 (1H, d), 10.60 (1H, s).
LRMS : m/z 463 (M+1)⁺

### Synthesis of the Compounds of Formulae IA and IB

### Example 1

### 5-[5-(4-Methylpiperazin-1-ylsulphonyl)-2-n-propoxyphenyl]-7-oxo-1-(pyridin-2-yl)methyl-1,6-dihydro-1H-pyrazolo[4,3-d]pyrimidine-3-carboxamide

Thionyl chloride (64µl, 0.87mmol) and chlorosulphonic acid (387µl, 5.82mmol) were added to an ice cooled flask containing the title compound of Preparation 26 (235mg, 0.58mmol), and the reaction stirred at room temperature for 18 hours. Ice (1g) was carefully added with stirring, then N-methylpiperazine (2ml, 18.0mmol) followed by sufficient ethanol to obtain a solution. The mixture was stirred for 3 hours at room temperature and evaporated under reduced pressure. The residue was partitioned between dichloromethane (5ml) and saturated sodium bicarbonate solution (10ml), and the phases separated. The aqueous layer was extracted with dichloromethane (3x10ml), the combined organic solutions dried (Na₂SO₄) and evaporated under reduced pressure. The residual yellow oil was purified by column chromatography on silica gel, using an elution gradient of dichloromethane:methanol (100:0 to 97:3) to afford the title compound (160mg, 50%) as a pale yellow solid.
δ (DMSOd₆) : 0.92 (3H, t), 1.72 (2H, m), 2.15 (3H, s), 2.37 (4H, m), 2.92 (4H, m), 4.14 (2H, t), 5.96 (2H, s), 7.23 (1H, d), 7.31 (1H, m), 7.40 (1H, d), 7.70 (1H, s), 7.79 (2H, m), 7.85 (1H, d), 7.93 (1H, s), 8.48 (1H, d), 12.55 (1H, s).
LRMS : m/z 567 (M+1)⁺

### Example 2

### 5-[5-(4-Methylpiperazin-1-ylsulphonyl)-2-n-propoxyphenyl]-7-oxo-1-(pyridin-2-yl)methyl-1,6-dihydro-1H-pyrazolo[4,3-d]pyrimidine-3-N-methylcarboxamide

Thionyl chloride (26µl, 0.36mmol) and chlorosulphonic acid (160µl, 2.39mmol) were added to an ice cooled flask containing the title compound of Preparation 33 (100mg, 0.24mmol) and the reaction stirred at room temperature for 18 hours. Ice (1g) was carefully added, then N-methylpiperazine (3ml, 27.0mmol) followed by enough ethanol to ensure solution, and the mixture stirred at room temperature for 4 hours. The reaction mixture was concentrated under reduced pressure, the residue partitioned between dichloromethane (5ml) and saturated sodium bicarbonate solution (10ml), and the phases separated. The aqueous layer was extracted with dichloromethane (3x10ml), the combined organic solutions washed with water (10ml), dried (Na₂SO₄) and evaporated under reduced pressure. The residue was triturated with diethyl ether, to afford the title compound (95mg, 68%) as a white solid.
Found : C, 55.05; H, 5.51; N, 18.77. C₂₇H₃₂N₈O₅S;0.50H₂O requires C, 55.00; H, 5.64; N, 19.00%.
δ (CDCl₃) : 1.18 (3H, t), 2.04 (2H, m), 2.29 (3H, s), 2.52 (4H, m), 3.12 (7H, m), 4.30 (2H, t), 6.04 (2H, s), 7.08-7.23 (3H, m), 7.61 (1H, m), 7.90 (1H, d), 8.10 (1H, m), 8.53 (1H, d), 8.75 (1H, s), 11.00 (1H, s).
LRMS : m/z 581 (M+1)⁺

### Examples 3 to 14

The compounds of the following tabulated examples of the general formula: 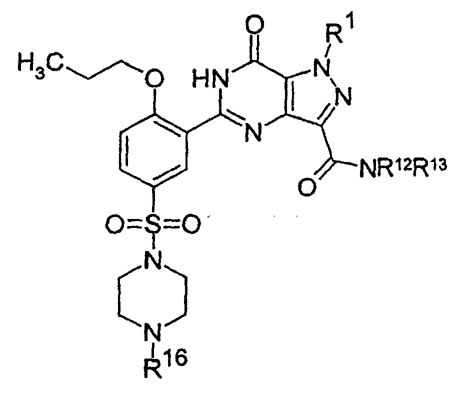 were prepared by the reaction of the corresponding pyrazolo[4,3-d]pyrimidinone with N-alkyl piperazine using similar methods to those described in either Example 1 (method a) or 2 (method b).

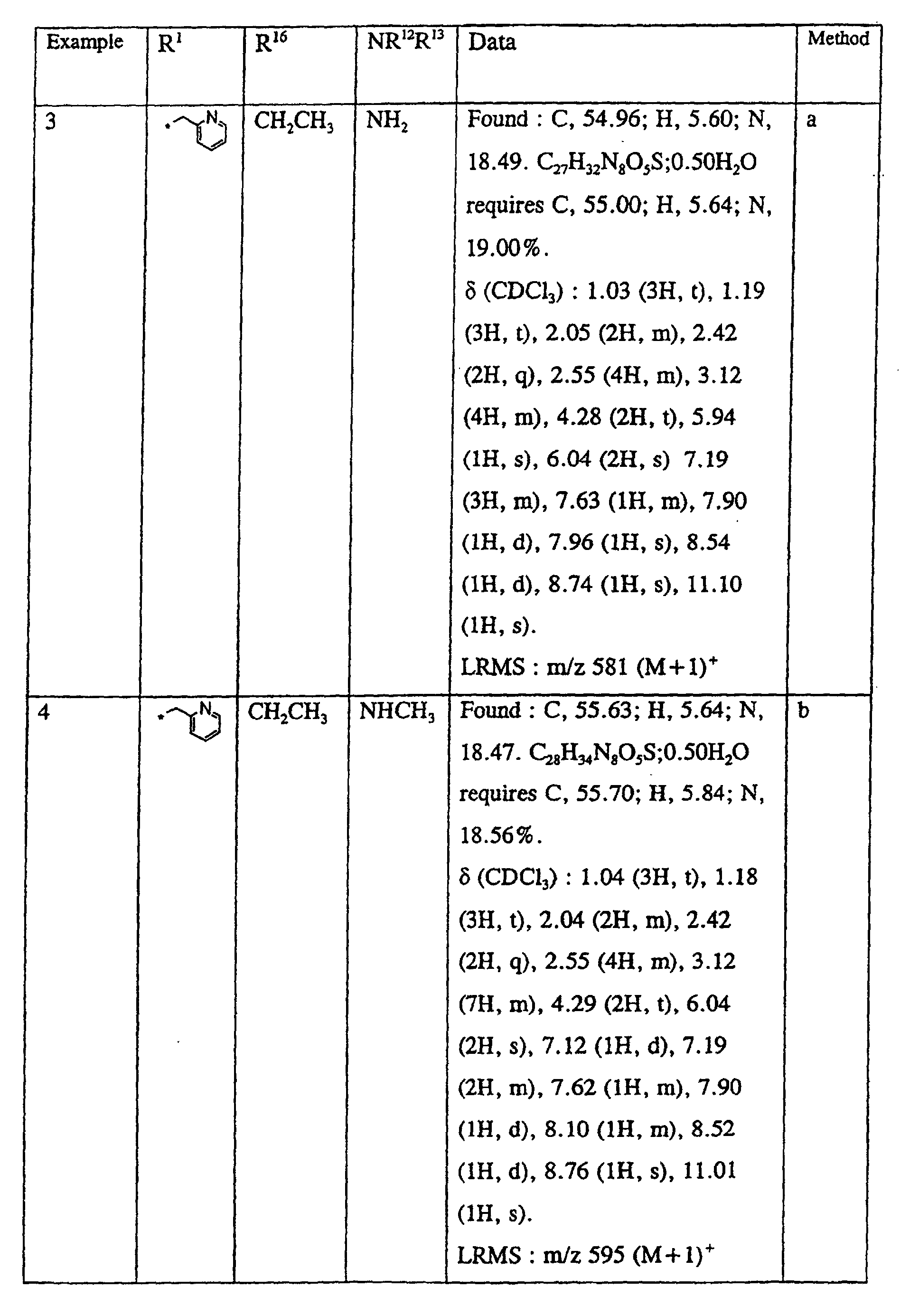

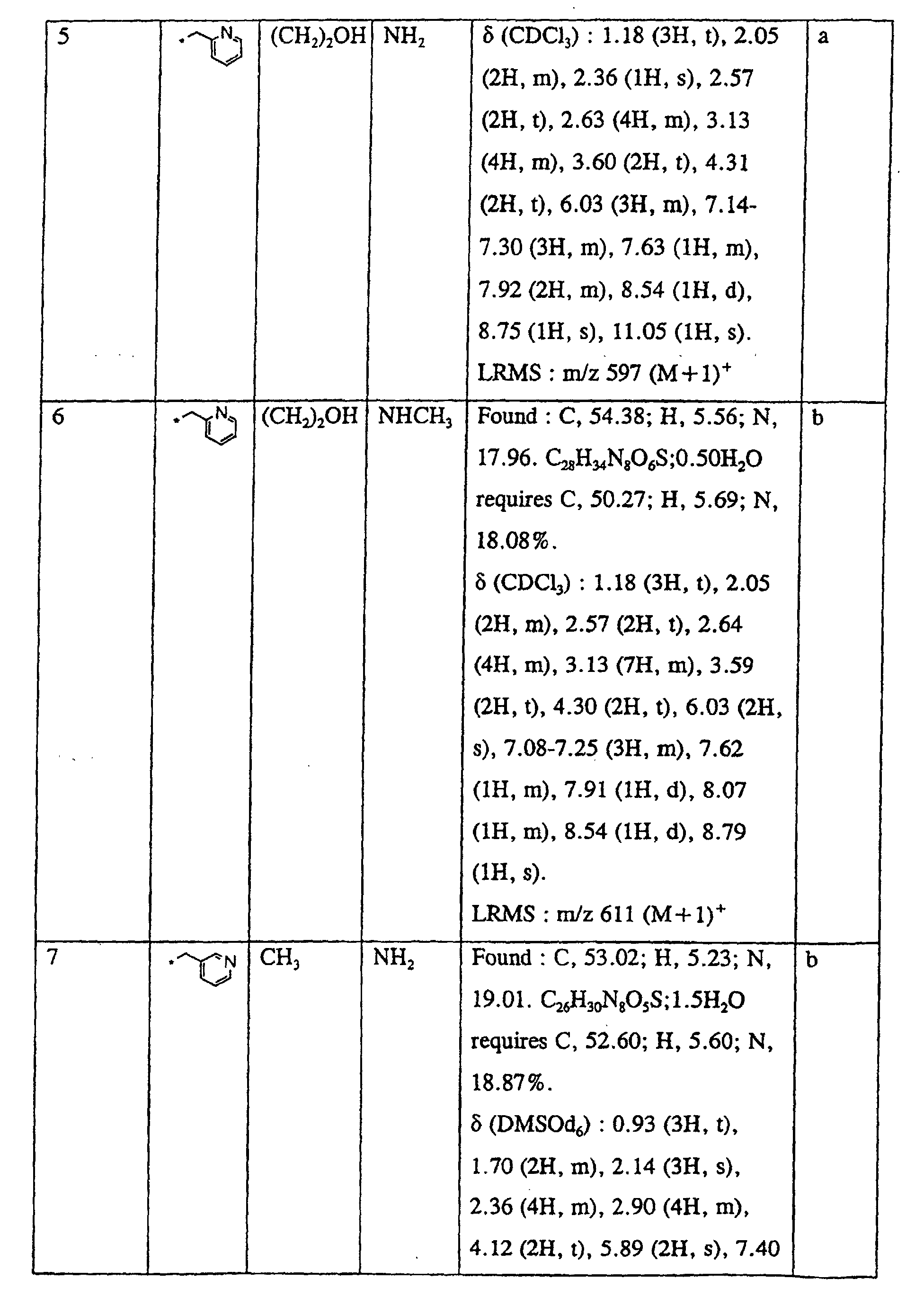

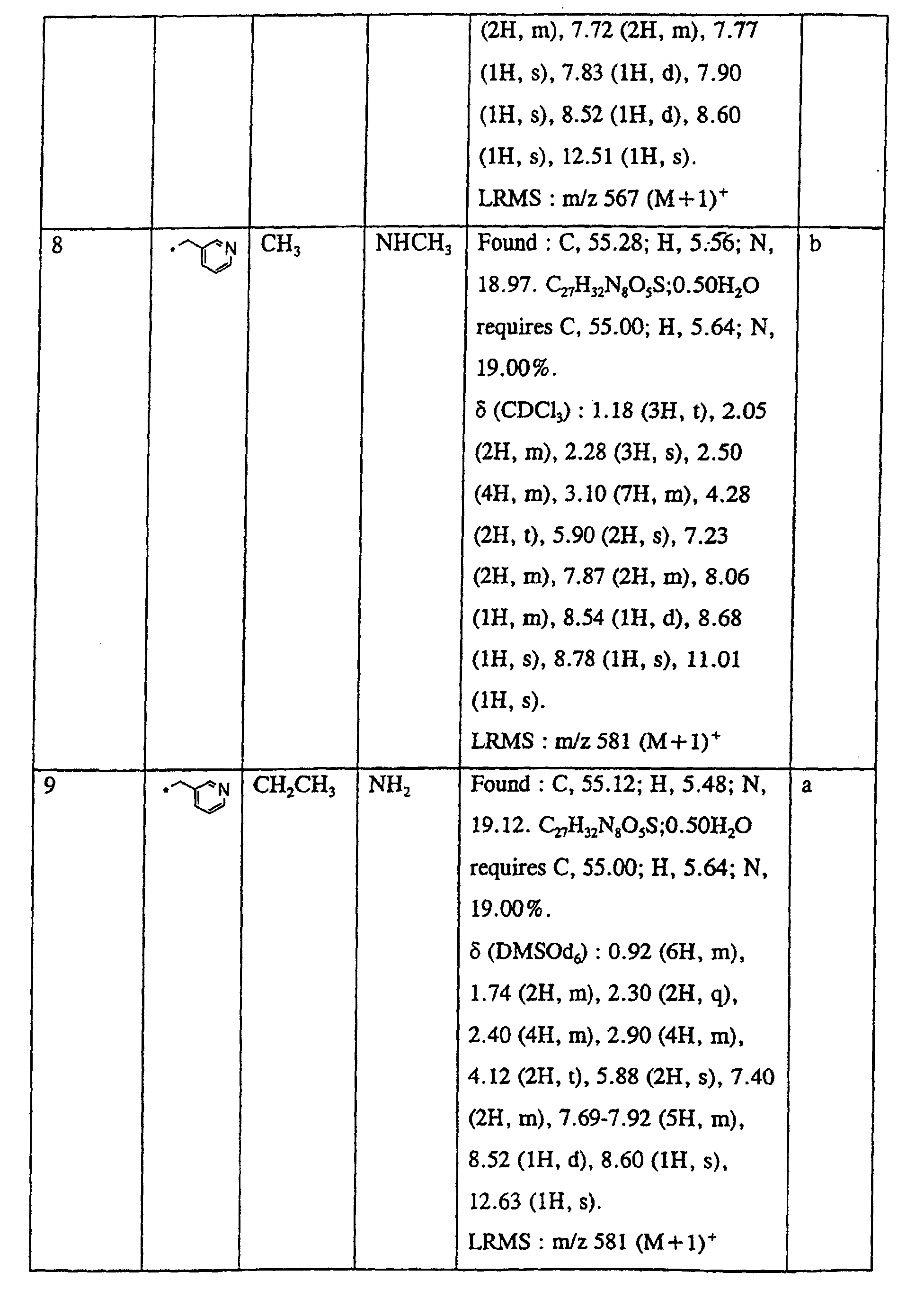

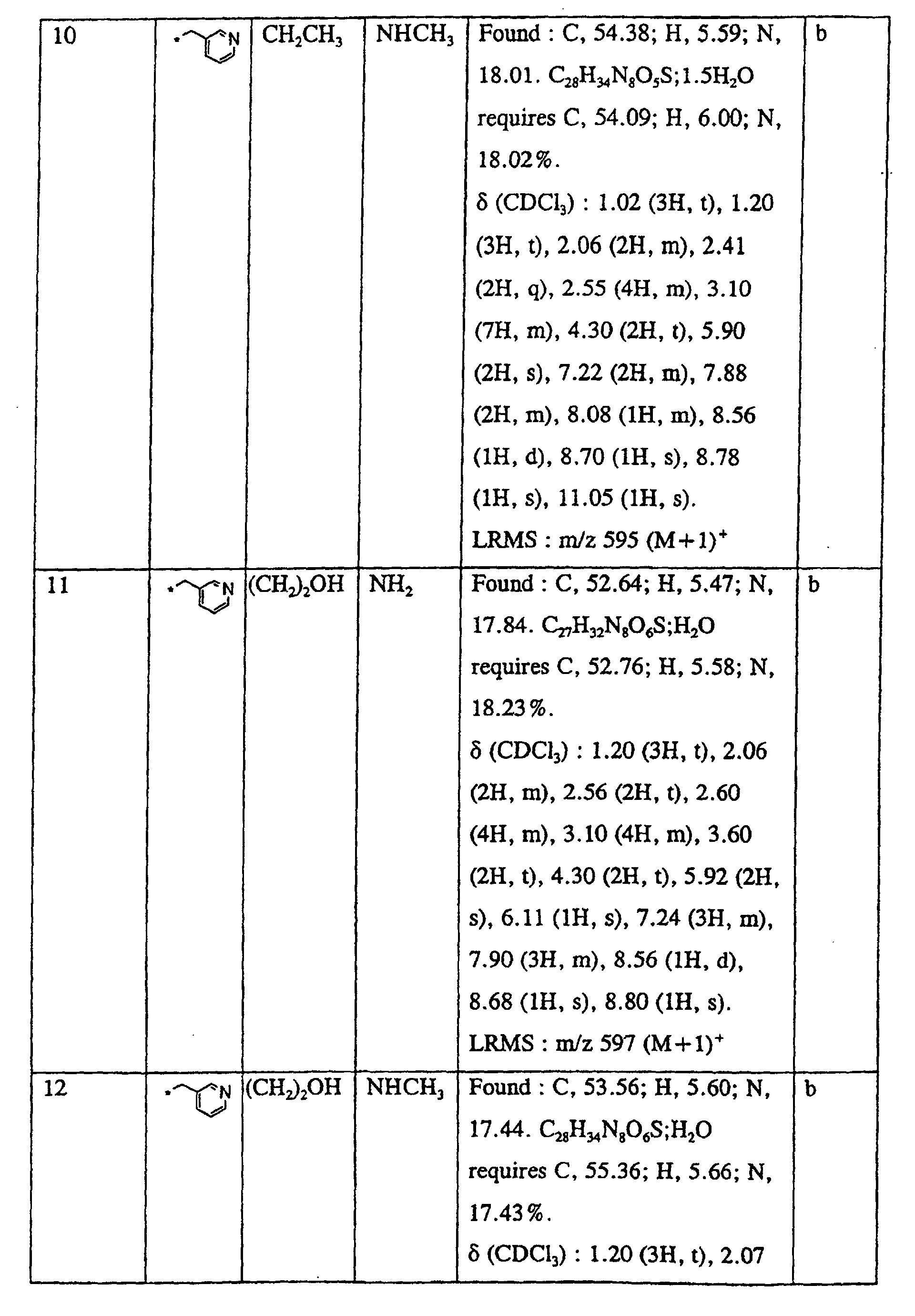

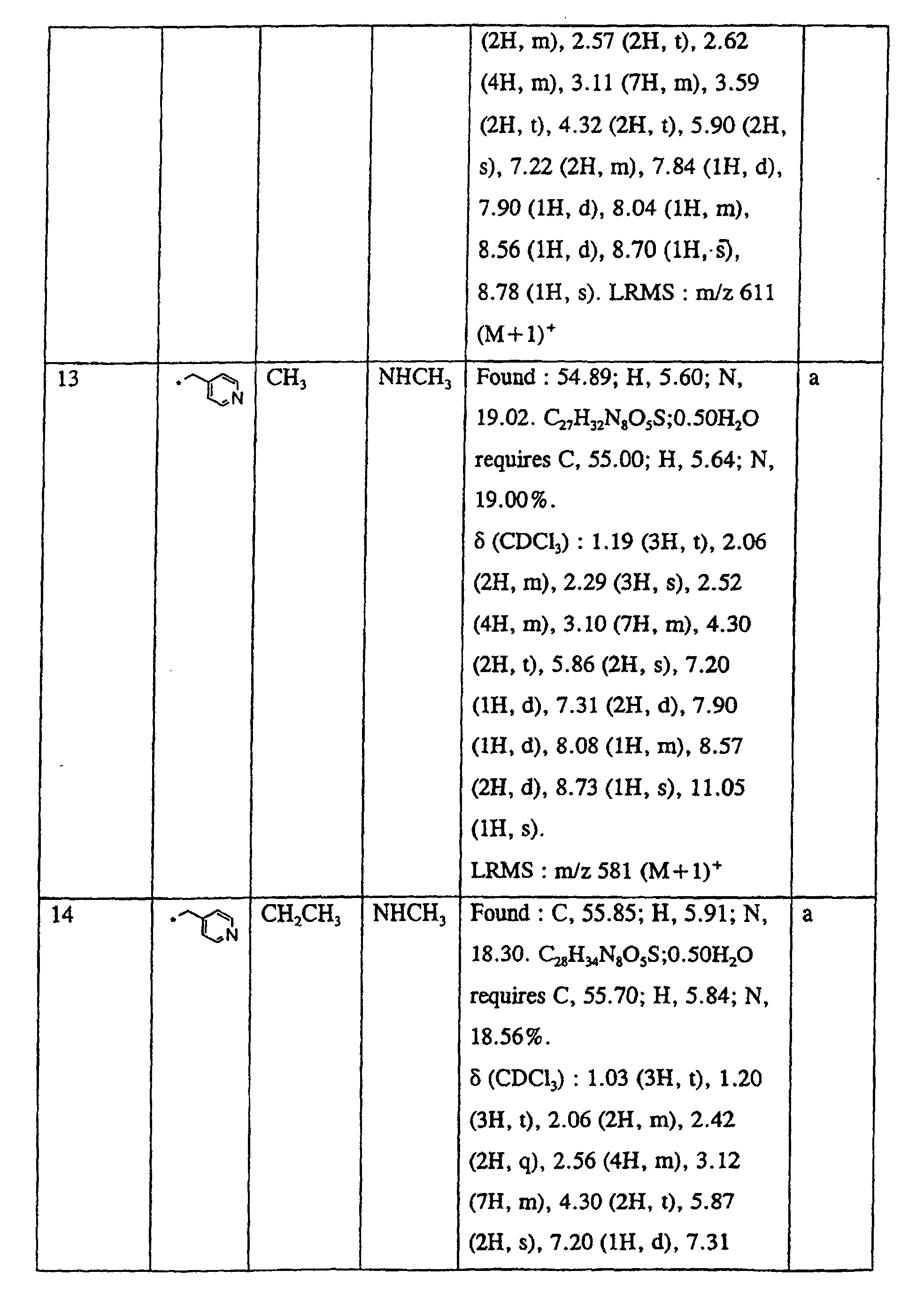

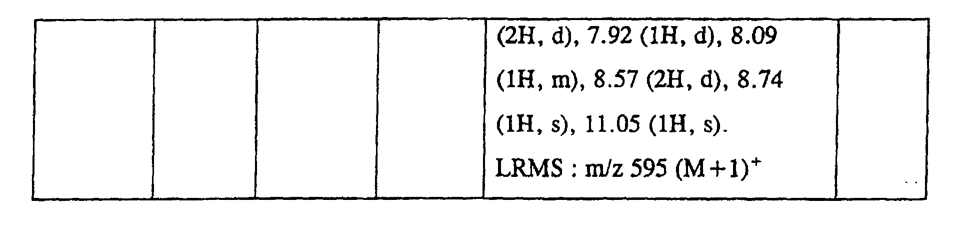

### Example 15

### 1-(4-Bromobenzyl)-5-[5-(4-methylpiperazin-1-ylsulphonyl)-2-n-propoxyphenyl]-7-oxo-1,6-dihydro-1H-pyrazolo[4,3-d]pyrimidine-3-N-methylcarboxamide

Thionyl chloride (81µl, 1.1mmol) and chlorosulphonic acid (0.44µl, 6.67mmol) were added to an ice-cooled flask containing the title compound of Preparation 36 (370mg, 0.74mmol) and the reaction stirred at room temperature for 18 hours. Ice (1g) was carefully added with stirring, and the resulting precipitate filtered, washed with water and dried under suction. N-Methylpiperazine (416µl, 3.75mmol) was added to a suspension of this product in ethanol (5ml), and the reaction stirred at room temperature for an hour. The reaction mixture was evaporated under reduced pressure and the residue purified by column chromatography on silica gel, using dichloromethane:methanol:0.88 ammonia (95:5:1) as eluant. This product was recrystallised from dichloromethane-hexane to afford the title compound (270mg, 55%) as a white solid.
Found : C, 50.08; H, 4.78; N, 14.45. C₂₈H₃₂BrN₇O₅S;H₂O requires C, 49.71; H, 5.07; N, 14.49%.
δ (CDCl₃) : 1.20 (3H, t), 2.07 (2H, m), 2.30 (3H, s), 2.52 (4H, m), 3.12 (7H, m), 4.30 (2H, t), 5.81 (2H, s), 7.21 (1H, d), 7.40 (4H, m), 7.90 (1H, d), 8.06 (1H, m), 8.72 (1H, s), 11.00 (1H, s).
LRMS : m/z 659 (M+1)⁺

### Example 16

### 1-(4-Bromobenzyl)-5-[5-(4-ethylpiperazin-1-ylsulphonyl)-2-n-propoxyphenyl]-7-oxo-1,6-dihydro-1H-pyrazolo[4,3-d]pyrimidine-3-N-methylcarboxamide

Obtained (63%) from the title compound of Preparation 36 and N-ethylpiperazine using the procedure of Example 15.
δ (CDCl₃) : 1.04 (3H, t), 1.20 (3H, t), 2.06 (2H, m), 2.42 (2H, q), 2.56 (4H, m), 3.12 (7H, m), 4.30 (2H, t), 5.81 (2H, s), 7.20 (1H, d), 7.41 (4H, m), 7.91 (1H, d), 8.08 (1H, m), 8.72 (1H, s), 11.00 (1H, s).
LRMS : m/z 673 (M+1)⁺

### Example 17

### 1-(4-Bromobenzyl)-5-{5-[4-(2-hydroxyethyl)piperazin-1-ylsulphonyl]-2-n-propoxyphenyl}-7-oxo-1,6-dihydro-1H-pyrazolo[4,3-d]pyrimidine-3-N-methylcarboxamide

Obtained as fine white crystals after crystallisation from ethanol (70%), from the title compound of Preparation 36 and N-(2-hydroxyethyl)piperazine, using the procedure of Example 15.
Found : C, 50.15; H, 5.01; N, 13.97. C₂₉H₃₄BrN₇O₆S requires C, 50.59; H, 4.98; N, 14.24%.
δ (CDCl₃) : 1.20 (3H, t), 2.06 (2H, m), 2.26 (1H, s), 2.58 (2H, t), 2.63 (4H, m), 3.12 (7H, m), 3.60 (2H, m), 4.32 (2H, t), 5.81 (2H, s), 7.22 (1H, d), 7.40 (4H, m), 7.90 (1H, d), 8.04 (1H, m), 8.75 (1H, s), 11.02 (1H, s).
LRMS : m/z 688 (M)⁺

### Example 18

### 1-Benzyl-5-[2-ethoxy-5-(4-methylpiperazin-1-ylsulphonyl)phenyl]-7-oxo-1,6-dihydro-1H-pyrazolo[4,3-d]pyrimidine-3-carboxamide

N,N'-Carbonyldiimidazole (80mg, 0.5mmol) was added to a suspension of the title compound of Preparation 72 (250mg, 0.46mmol) in tetrahydrofuran (15ml), and the reaction heated under reflux and a nitrogen atmosphere for 4 hours. The solution was cooled in ice, ammonia gas bubbled through for 5 minutes, and then stirred at room temperature for 14 hours. The mixture was filtered, the precipitate washed with ethyl acetate, and dried under suction to afford the title compound (225mg, 89%) as a white solid.
Found : C, 55.32; H, 5.26; N, 17.22. C₂₆H₂₉N₇O₅S;0.75H₂O requires C, 55.36; H, 5.44; N, 17.35%.
δ (DMSOd₆) : 1.33 (3H, t), 2.16 (3H, s), 2.39 (4H, m), 2.91 (4H, m), 4.22 (2H, q), 5.82 (2H, s), 7.37 (6H, m), 7.70 (1H, s), 7.79 (1H, s), 7.84 (1H, m), 7.92 (1H, s), 12.66 (1H, s).
LRMS : m/z 552 (M+1)⁺

### Example 19

### 1-Benzyl-5-[2-ethoxy-5-(4-methylpiperazin-1-ylsulphonyl)phenyl]-7-oxo-1,6-dihydro-1H-pyrazolo[4,3-d]pyrimidine-3-N-methylcarboxamide

1-(3-Dimethylaminopropyl)-3-ethylcarbodiimide hydrochloride (250mg, 0.45mmol), was added to a suspension of the title compound of Preparation 72 (250mg, 0.45mmol), N-methylmorpholine (0.11ml, 1.0mmol), 1-hydroxybenzotriazole hydrate (67mg, 0.50mmol) and methylamine hydrochloride (67mg, 1.0mmol) in dichloromethane (7ml) and the reaction stirred at room temperature for 18 hours. The mixture was partitioned between dichloromethane (15ml) and aqueous sodium bicarbonate solution (15ml), the phases separated and the aqueous layer extracted with dichloromethane (2x15ml). The combined organic extracts were dried (Na₂SO₄) and evaporated under reduced pressure. The residual solid was purified by column chromatography on silica gel, using an elution gradient of dichloromethane:methanol (100:0 to 95:5) to afford the title compound (250mg, 98%) as a white solid.
Found C, 56.60; H, 5.53; N, 16.84. C₂₇H₃₁N₇O₅S;CH₃OH requires C, 57.11; H, 5.60; N, 17.14%.
δ (CDCl₃) :1.67 (3H, t), 2.30 (3H, s), 2.52 (4H, m), 3.12 (7H, m), 4.41 (2H, q), 5.86 (2H, s), 7.20 (1H, d), 7.30 (3H, m), 7.52 (2H, m), 7.90 (1H, d), 8.08 (1H, m), 8.72 (1H, s), 10.98 (1H, s).

### Example 20

### 1-Benzyl-5-[2-ethoxy-5-(4-methylpiperazin-1-ylsulphonyl)phenyl]-7-oxo-1,6-dihydro-1H-pyrazolo[4,3-d]pyrimidine-3-N-ethylcarboxamide

Obtained (77%) from the title compound of Preparation 72 and ethylamine hydrochloride, using the procedure described in Example 19.
Found : C, 57.33; H, 5.73; N, 16.56. C₂₈H₃₃N₇O₅S requires C, 58.01; H, 5.74; N, 16.91%.
δ (CDCl₃) : 1.37 (3H, t), 1.67 (3H, t), 2.26 (3H, s), 2.49 (4H, m), 3.10 (4H, m), 3.60 (2H, m), 4.40 (2H, q), 5.85 (2H, s), 7.20 (1H, d), 7.28 (3H, m), 7.50 (2H, m), 7.88 (1H, d), 8.02 (1H, m), 8.78 (1H, s), 11.05 (1H, s).

### Example 21

### 1-Benzyl-5-[2-ethoxy-5-(4-methylpiperazin-1-ylsulphonyl)phenyl]-7-oxo-1,6-dihydro-1H-pyrazolo[4,3-d]pyrimidine-3-N,N-dimethylcarboxamide

Obtained as a white solid (86%) from the title compound of Preparation 72 and dimethylamine hydrochloride, using the procedure of Example 19. δ (CDCl₃) : 1.66 (3H, t), 2.27 (3H, s), 2.50 (4H, m), 3.10 (4H, m), 3.15 (3H, s), 3.20 (3H, s), 4.39 (2H, q), 5.82 (2H, s), 7.16 (1H, d), 7.31 (3H, m), 7.48 (2H, m), 7.84 (1H, d), 8.82 (1H, s), 11.00 (1H, s).
LRMS : m/z 580 (M+1)⁺

### Example 22

### 1-Benzyl-5-[5-(4-methylpiperazin-1-ylsulphonyl)-2-n-propoxyphenyl]-7-oxo-1,6-dihydro-1H-pyrazolo[4,3-d]pyrimidine-3-N-methylcarboxamide

Triethylamine (140µl, 1.0mmol), palladium (0) tetrakis(triphenyl)-phosphine (40mg, 0.034mmol) and sodium formate (68mg, 1.0mmol) were added to a solution of the title compound of Example 15 (220mg, 0.33mmol) in acetonitrile:dimethylsulphoxide (4ml, 1:1), and the reaction heated under reflux for 18 hours. The cooled reaction mixture was concentrated under reduced pressure, the residue suspended in water (10ml) and extracted with dichloromethane (3x10ml), the combined organic extracts dried (Na₂SO₄) and evaporated under reduced pressure. The residual yellow solid was purified by column chromatography on silica gel, using dichloromethane:methanol:0.88 ammonia (95:5:1) as eluant and recrystallised from ethanol to afford the title compound (94mg, 49%) as a white powder.
Found : C, 57.88; H, 5.78; N, 16.56. C₂₈H₃₃N₇O₅S requires C, 58.02; H, 5.74; N, 16.91 %.
δ (CDCl₃) : 1.20 (3H, t), 2.06 (2H, m), 2.29 (3H, s), 2.50 (4H, m), 3.10 (7H, m), 4.30 (2H, t), 5.86 (2H, s), 7.20 (1H, d), 7.30 (3H, m), 7.50 (2H, m), 7.90 (1H, d), 8.08 (1H, m), 8.73 (1H, s), 10.98 (1H, s).
LRMS : m/z 580 (M+1)⁺

### Example 23

### 1-Benzyl-5-[5-(4-ethylpiperazin-1-ylsulphonyl)-2-n-propoxyphenyl]-7-oxo-1,6-dihydro-1H-pyrazolo[4,3-d]pyrimidine-3-N-methylcarboxamide

Obtained as a white solid (34%) from the title compound of Example 16, using the procedure of Example 22.
Found : C, 58.44; H, 6.04; N, 16.14. C₂₉H₃₅N₇O₅S requires C, 58.67; H, 5.94; N, 16.51%.
δ (CDCl₃) : 1.03 (3H, t), 1.20 (3H, t), 2.05 (2H, m), 2.42 (2H, q), 2.56 (4H, m), 3.12 (7H, m), 4.28 (2H, t), 5.86 (2H, s), 7.20 (1H, d), 7.30 (3H, m), 7.52 (2H, m), 7.90 (1H, d), 8.07 (1H, m), 8.72 (1H, s), 10.98 (1H, s).

### Example 24

### 1-Benzyl-5-{5-[4-(2-hydroxyethyl)piperazin-1-ylsulphonyl]-2-n-propoxyphenyl}-7-oxo-1,6-dihydro-1H-pyrazolo[4,3-d]pyrimidine-3-N-methylcarboxamide

Obtained as a white powder (55%) from the title compound of Example 17, using the procedure of Example 22.
Found : C, 57.01; H, 5.85; N, 15.79. C₂₉H₃₅N₇O₆S requires C, 57.13; H, 5.79; N, 16.08%.
δ (CDCl₃) : 1.20 (3H, t), 2.06 (2H, m), 2.26 (1H, s), 2.58 (2H, t), 2.63 (4H, m), 3.12 (7H, m), 3.59 (2H, m), 4.30 (2H, t), 5.86 (2H, s), 7.20 (1H, d), 7.30 (3H, m), 7.50 (2H, m), 7.90 (1H, d), 8.05 (1H, m), 8.75 (1H, s), 11.00 (1H, s).
LRMS : m/z 611 (M+2)⁺

### Example 25

### 5-[5-(4-Methylpiperazin-1-ylsulphonyl)-2-n-propoxyphenyl]-7-oxo-2,6-dihydro-2H-pyrazolo[4,3-d]pyximidine-3-carboxamide hydrochloride

Thionyl chloride (1ml, 13.7mmol) was added to an ice-cooled solution of the title compound of Preparation 32 (490mg, 1.56mmol) in chlorosulphonic acid (2ml, 30.0mmol), and the reaction stirred at room temperature for 18 hours. The reaction mixture was poured carefully onto ice (10g), and the resulting precipitate filtered, washed with water and dried under suction to give a beige solid (360mg). N-Methylpiperazine (180ml, 1.65mmol) was added to a suspension of this solid in ethanol (20ml) and the reaction stirred at room temperature for 18 hours. The reaction mixture was concentrated under reduced pressure, the residue suspended in water (10ml), and acidified to pH 6 with 2N hydrochloric acid. The resulting precipitate was filtered, washed with water and diethyl ether and dried at 60°C to afford the title compound (305mg, 44%) as an off-white powder.
δ (DMSOd₆) : 0.94 (3H, t), 1.73 (2H, m), 2.23-3.10 (11H, m), 4.14 (2H, t), 7.40 (1H, d), 7.59 (1H, s), 7.85 (2H, m), 7.96 (1H, s), 12.37 (1H, s), 14.90 (1H, s).
LRMS : m/z 476 (M+1)⁺

### Example 26

### 2-Methyl-5-[5-(4-methylpiperazin-1-ylsulphonyl)-2-n-propoxyphenyl]-7-oxo-2,6-dihydro-2H-pyrazolo[4,3-d]pyrimidine-3-carboxamide

Thionyl chloride (500µl, 6.85mmol) was added to an ice-cooled solution of the title compound of Preparation 60 (125mg, 0.37mmol) in chlorosulphonic acid (1.0ml, 15.0mmol) and the reaction stirred at room temperature for 18 hours. The reaction mixture was poured carefully onto ice (5g), and the aqueous solution extracted with dichloromethane (3x15ml). The combined organic extracts were dried (MgSO₄) and evaporated under reduced pressure, and the residue triturated with water and diethyl ether, to give a white solid (90mg). N-Methyl piperazine (40µl, 0.36mmol) was added to a suspension of this product in ethanol (5ml) and the reaction stirred at room temperature for 18 hours. The reaction mixture was concentrated under reduced pressure, the residue dissolved in dichloromethane (25ml), washed with water (2x10ml), dried (MgSO₄) and evaporated under reduced pressure to afford the title compound (82mg, 50%) as a white solid.
Found : C, 50.50; H, 5.64; N, 18.32. C₂₂H₂₉N₇O₅S;H₂O requires C, 50.66; H, 5.99; N, 18.79%.
δ (DMSOd₆) : 0.92 (3H, t), 1.70 (2H, m), 2.15 (3H, s), 2.36 (4H, m), 2.85 (3H, d), 2.92 (4H, m), 4.11 (2H, t), 4.36 (3H, s), 7.38 (1H, d), 7.82 (1H, d), 7.92 (1H, s), 8.28 (1H, m), 12.18 (1H, s).
LRMS : m/z 504 (M+1)⁺

### Example 27

### 1-Methyl-5-[5-(4-methylpiperazin-1-ylsulphonyl)-2-n-propoxyphenyl]-7-oxo-1,6-dihydro-1H-pyrazolo[4,3-d]pyrimidine-3-carboxamide hydrochloride

Obtained as a white solid (80%) from the title compound of Preparation 61 and N-methylpiperazine using the procedure of Example 25.
Found : C, 48.58; H, 5.35; N, 18.59. C₂₂H₂₉N₇O₅S;HCl requires C, 47.95; H, 5.31; N, 18.64%.
δ (DMSOd₆) : 0.94 (3H, t), 1.73 (2H, m), 2.16 (3H, s), 2.39 (4H, m), 2.90 (4H, m), 4.12 (2H, t), 4.28 (3H, s), 7.40 (1H, d), 7.65 (1H, s), 7.72 (1H, s), 7.84 (1H, d), 7.90 (1H, s), 12.54 (1H, s).
LRMS : m/z 490 (M+1)⁺

### Example 28

### 5-[5-(4-Ethylpiperazin-1-ylsulphonyl)-2-n-propoxyphenyl]-1-methyl-7-oxo-1,6-dihydro-1H-pyrazolo[4,3-d]pyrimidine-3-carboxamide

Obtained as a white solid (82%) from the title compound of Preparation 61 and N-ethylpiperazine using the procedure of Example 26.
Found : C, 50.05; H, 5.89; N, 18.12. C₂₂H₂₉N₇O₅S;1.20H₂O requires C, 50.31; H, 6.03; N, 18.67%.
δ (CDCl₃) : 1.03 (3H, t), 1.20 (3H, t), 2.07 (2H, m), 2.41 (2H, q), 2.55 (4H, m), 3.10 (4H, m), 4.30 (2H, t), 4.40 (3H, s), 5.84 (1H, s), 7.21 (1H, d), 7.90 (2H, m), 8.70 (1H, s), 11.00 (1H, s).
LRMS : m/z 504 (M+1)⁺

### Example 29

### 1-Cyclobutylmethyl-5-[5-(4-methylpiperazin-1-ylsulphonyl)-2-n-propoxyphenyl]-7-oxo-1,6-dihydro-1H-pyrazolo[4,3-d]pyrimidine-3-N-methylcarboxamide

Obtained as a white solid (89%) from the title compound of Preparation 37 and N-methylpiperazine, using the procedure of Example 15.
Found : C, 55.60; H, 6.29; N, 17.37. C₂₆H₃₅N₇O₅S requires C, 56.00; H, 6.33; N, 17.58%.
δ (CDCl₃) : 1.18 (3H, t), 1.86 (4H, m), 1.95-2.06 (4H, m), 2.25 (3H, s), 2.49 (4H, m), 3.00 (1H, m), 3.08 (7H, m), 4.28 (2H, t), 4.70 (2H, d), 7.18 (1H, d), 7.85 (1H, d), 8.04 (1H, m), 8.72 (1H, s), 10.95 (1H, s).
LRMS : m/z 559 (M+2)⁺

### Example 30

### 5-[5-(4-Methylpiperazin-1-ylsulphonyl)-2-n-propoxyphenyl]-1-[2-(4-morpholinyl)ethyl]-7-oxo-1,6-dihydro-1H-pyrazolo[4,3-d]pyrimidine-3-N-methylcarboxamide

Obtained (15%) from the title compound of Preparation 31 and N-methylpiperazine using the procedure of Example 1.
δ (CDCl₃) : 1.20 (3H, t), 2.08 (2H, m), 2.30 (3H, s), 2.54 (8H, m), 2.96 (2H, t), 3.11 (4H, m), 3.60 (4H, m), 4.32 (2H, t), 4.83 (2H, t), 5.93 (1H, s), 7.21 (1H, d), 7.90 (1H, d), 7.98 (1H, s), 8.73 (1H, s), 11.03 (1H, s).
LRMS : m/z 589 (M+1)⁺

### Example 31

### 5-[5-(4-Ethylpiperazin-1-ylsulphonyl)-2-(n-propoxy)pyridin-3-yl]-7-oxo-2-(pyridin-2-yl)methyl-2,6-dihydro-2H-pyrazolo[4,3-d]pyrimidine-3-carboxamide and

### Example 32

### 5-[5-(4-Ethylpiperazin-1-ylsulphonyl)-2-(n-propoxy)pyridin-3-yl]-7-oxo-1-(pyridin-2-yl)methyl-1,6-dihydro-1H-pyrazolo[4,3-d]pyrimidine-3-carboxamide

A mixture of the title compound of Preparation 73 (380mg, 0.65mmol) and potassium bis(trimethylsilyl)amide (518mg, 2.60mmol) in ethanol (20ml) was heated at 100°C in a sealed vessel for 18 hours. The cooled reaction mixture was concentrated under reduced pressure, the residue dissolved in water (7ml), neutralised with 50% aqueous citric acid and the aqueous solution extracted with dichloromethane (3x30ml). The combined organic extracts were dried (MgSO₄), and evaporated under reduced pressure. The residual brown foam was purified by column chromatography on silica gel, using an elution gradient of dichloromethane:methanol (98:2 to 95:5), and azeotroped with dichloromethane and diethyl ether, to afford the title compound of Example 31 (8mg, 2%) as a white solid:
δ (CDCl₃) : 1.04 (3H, t), 1.60 (3H, t), 2.42 (2H, q), 2.58 (4H, m), 3.15 (4H, m), 4.79 (2H, q), 5.77 (1H, s), 6.30 (2H, s), 7.11 (1H, d), 7.19 (1H, m), 7.62 (1H, m), 8.15 (1H, s), 8.54 (1H, d), 8.70 (1H, s), 8.88 (1H, s), 10.81 (1H, s);
LRMS : m/z 568 (M+1)⁺; and the title compound of Example 32 (200mg, 54%) as a white solid. Found : C, 52.53; H, 5.08; N, 21.83. C₂₅H₂₉N₉O₅S requires C, 52.90; H, 5.15; N, 22.21%.
δ (CDCl₃) : 1.05 (3H, t), 1.59 (3H, t), 2.42 (2H, q), 2.57 (4H, m), 3.15 (4H, m), 4.79 (2H, q), 5.86 (1H, s), 6.06 (2H, s), 7.19 (2H, m), 7.64 (1H, m), 7.79 (1H, s), 8.54 (1H, d), 8.71 (1H, s), 8.98 (1H, s), 11.02 (1H, s).
LRMS : m/z 568 (M+1)⁺

### Example 33

### 5-[5-(4-Ethylpiperazin-1-ylsulphonyl)-2-(2-methoxyethoxy)pyridin-3-yl]-7-oxo-1-(pyridin-2-yl)methyl-1,6-dihydro-1H-pyrazolo[4,3-d]pyrimidine-3-carboxamide

A mixture of 2-methoxyethanol (10ml) and potassium bis(trimethylsilyl)amide (175.7mg, 0.88mmol) was heated at 90°C for an hour, then cooled. The title compound of Example 32 (100mg, 0.17mmol) was added and the reaction heated at 110°C for 18 hours. The cooled reaction mixture was concentrated under reduced pressure, the residue dissolved in water (5ml) and neutralised with 20% aqueous citric acid. The aqueous solution was extracted with dichloromethane (3x10ml), the combined organic extracts dried (MgSO₄) and evaporated under reduced pressure. The residual brown oil was purified by column chromatography on silica gel, using an elution gradient of dichloromethane:methanol (98:2 to 95:5) and azeotroped with dichloromethane and diethyl ether, to afford the title compound (94mg, 90%) as a white solid.
Found : C, 51.73; H, 5.22; N, 20.49. C₂₆H₃₁N₉O₆S requires C, 52.25; H, 5.23; N, 21.09%.
δ (CDCl₃) : 1.04 (3H, t), 2.43 (2H, q), 2.57 (4H, m), 3.16 (4H, m), 3.58 (3H, s), 3.87 (2H, t), 4.81 (2H, t), 5.85 (1H, s), 6.08 (2H, s), 7.18 (2H, m), 7.64 (1H, m), 7.80 (1H, s), 8.55 (1H, d), 8.70 (1H, s), 8.89 (1H, s), 11.28 (1H, s).
LRMS : m/z 598 (M+1)⁺

### Example 34

### 5-[5-(4-Ethylpiperazin-1-ylsulphonyl)-2-(2-methoxyethoxy)pyridin-3-yl]-7-oxo-2-(pyridin-2-yl)methyl-2,6-dihydro-1H-pyrazolo[4,3-d]pyrimidine-3-N-methylcarboxamide

Obtained as a beige solid (38%) from the title compound of Preparation 74, using a similar procedure to that described in Example 31.
Found : C, 53.25; H, 5.47; N, 21.21. C₂₆H₃₁N₉O₅S requires C, 53.69; H, 5.37; N, 21.67%.
δ (CDCl₃) : 1.05 (3H, t), 1.59 (3H, t), 2.42 (2H, q), 2.57 (4H, m), 3.05 (3H, d), 3.15 (4H, m), 4.79 (2H, q), 6.34 (2H, s), 7.10 (1H, d), 7.18 (1H, m), 7.62 (1H, m), 8.28 (1H, m), 8.54 (1H, d), 8.70 (1H, s), 8.92 (1H, s), 10.75 (1H, s).
LRMS : m/z 582 (M+1)⁺

### Example 35

### 5-[2-Ethoxy-5-(4-ethylpiperazin-1-ylsulphonyl)pyridin-3-yl]-7-oxo-2-methyl-2,6-dihydro-2H-pyrazolo[4,3-d]pyrimidine-3-N-methylcarboxamide

Obtained as a white solid (57%), from the title compound of Preparation 75, using a similar procedure to that described in Example 31.
Found : C, 49.69; H, 5.54; N, 21.85. C₂₁H₂₈N₈O₅S requires C, 49.99; H, 5.59; N, 22.20%.
δ (CDCl₃) : 1.05 (3H, t), 1.59 (3H, t), 2.42 (2H, q), 2.57 (4H, m), 3.09 (3H, d), 3.15 (4H, m), 4.54 (3H, s), 4.79 (2H, q), 8.21 (1H, m), 8.70 (1H, s), 8.90 (1H, s), 10.75 (1H, s).
LRMS : m/z 505 (M+1)⁺

### Example 36

### 5-[5-(4-Ethylpiperazin-1-ylsulphonyl)-2-(2-methoxyethoxy)pyridin-3-yl]-7-oxo-2-methyl-2,6-dihydro-2H-pyrazolo[4,3-d]pyrimidine-3-N-methylcarboxamide

Obtained as a white solid (73%), from the title compound of Example 35, using the procedure of Example 33.
Found C, 48.53; H, 5.76; N, 20.51. C₂₂H₃₀N₈O₆S;0.5H₂O requires C, 48.61; H, 5.75; N, 20.61%.
δ (CDCl₃) : 1.04 (3H, t), 2.43 (2H, q), 2.57 (4H, m), 3.08 (3H, d), 3.16 (4H, m), 3.59 (3H, s), 3.87 (2H, t), 4.53 (3H, s), 4.80 (2H, t), 8.22 (1H, m), 8.68 (1H, s), 8.81 (1H, s), 11.00 (1H, s).
LRMS : m/z 535 (M+1)⁺

### Example 37

### 5-{5-(4-Ethylpiperazin-1-ylsulphonyl)-2-[(pyridin-2-yl)methoxy]pyridin-3-yl]}-7-oxo-2-methyl-2,6-dihydro-2H-pyrazolo[4,3-d]pyrimidine-3-N-methylcarboxamide

Obtained as a white solid (33%) from the tide compound of Example 35 and 2-(hydroxymethyl)pyridine using the procedure of Example 33.
δ (CDCl₃) : 1.04 (3H, t), 2.42 (2H, q), 2.57 (4H, m), 3.09 (3H, d), 3.15 (4H, m), 4.56 (3H, s), 5.98 (2H, s), 7.37 (2H, m), 7.79 (1H, m), 8.30 (1H, m), 8.66 (2H, m), 8.87 (1H, d), 13.68 (1H, s).
LRMS : 568 (M+1)⁺

### Example 38

### 5-[5-(4-Ethylpiperazin-1-ylsulphonyl)-2-n-propoxyphenyl]-3-(pyridin-2-yl)-1,6-dihydro-1H-7H-pyrazolo[4,3-d]pyrimidin-7-one

A mixture of the title compound of Preparation 88 (120mg, 0.35mmol), chlorosulphonic acid (230µl, 3.5mmol) and thionyl chloride (38µl, 0.52mmol) was stirred at room temperature for 18 hours. The mixture was cooled in an ice-bath, ice (1g) added, followed by N-ethylpiperazine (2ml) and ethanol (1ml) and the reaction stirred at room temperature for 5 hours. The mixture was partitioned between dichloromethane (10ml) and sodium bicarbonate solution (5ml), and the phases separated. The aqueous layer was extracted with dichloromethane (3x10ml), the combined organic solutions washed with brine (20ml), dried (Na₂SO₄) and evaporated under reduced pressure. The residue was purified by column chromatography on silica gel, using an elution gradient of dichloromethane:methanol (100:0 to 98:2) to afford the title compound, (120mg, 66%) as a pale pink solid.

Found : C, 56.58; H, 5.63; N, 18.25. C₂₅H₂₉N₇O₄S;0.5H₂O requires C, 56.38; H, 5.68; N, 18.41%.
δ (CDCl₃) : 1.05 (3H, t), 1.21 (3H, t), 2.15 (2H, m), 2.62 (2H, q), 2.78 (4H, m), 3.28 (4H, m), 4.35 (2H, t), 7.15 (1H, m), 7.23 (1H, d), 7.58 (1H, m), 7.94 (1H, d), 8.29 (1H, d), 8.52 (1H, s), 8.77 (1H, s), 10.89 (1H, s), 13.82 (1H, s).
LRMS : m/z 524 (M+1)⁺

### Example 39

### 5-[5-(4-Ethylpiperazin-1-ylsulphonyl)-2-n-propoxyphenyl]-3-(pyridin-3-yl)-1,6-dihydro-1H-7H-pyrazolo[4,3-d]pyrimidin-7-one

A mixture of the title compound of Preparation 89 (150mg, 0.43mmol) chlorosulphonic acid (230µl, 3.46mmol) and thionyl chloride (40µl, 0.52mmol) was stirred at room temperature for 18 hours. Ice (5g) was added, followed by N-ethylpiperazine (3ml) and ethanol (1ml) and the reaction stirred for a further 4 hours. The mixture was partitioned between water (10ml) and dichloromethane (10ml), the layers separated and the aqueous phase extracted with dichloromethane (3x10ml). The combined organic solutions were dried (Na₂SO₄) and evaporated under reduced pressure, and the residue triturated with ethanol to afford the title compound (106mg, 47%) as a pale yellow solid.
δ (CDCl₃) : 0.82 (3H, t), 0.97 (3H, t), 1.82 (2H, m), 2.23 (2H, q), 2.38 (4H, m), 2.96 (4H, m), 4.06 (2H, t), 7.02 (1H, d), 7.19 (1H, m), 7.68 (1H, d), 8.40 (1H, d), 8.50 (2H, m), 9.38 (1H, s).
LRMS : m/z 524 (M+1)⁺

### Example 40

### 5-[5-(4-Ethylpiperazin-1-ylsulphonyl)-2-n-propoxyphenyl]-3-(pyridin-2-yl)-2-(pyridin-2-yl)methyl-2,6-dihydro-7H-pyrazolo[4,3-d]pyrimidin-7-one

Obtained as a white solid (25%) from the title compound of Preparation 91 using a similar procedure to that described in Example 38.
Found : C, 59.63; H, 5.65; N, 17.69. C₃₁H₃₄N₈O₄S;0.5H₂O requires C, 59.70; H, 5.66; N, 17.96%.
δ (CDCl₃) : 1.02 (3H, t), 1.20 (3H, t), 2.04 (2H, m), 2.41 (2H, q), 2.56 (4H, m), 3.15 (4H, m), 4.29 (2H, t), 6.14 (2H, s), 7.05 (1H, d), 7.18 (2H, m), 7.28 (1H, m); 7.60 (1H, m), 7.84 (2H, m), 8.56 (1H, d), 8.68 (1H, d), 8.79 (1H, d), 8.98 (1H, s), 11.01 (1H, s).
LRMS : m/z 615 (M+1)⁺

### Example 41

### 5-[5-(4-Ethylpiperazin-1-ylsulphonyl)-2-n-propoxyphenyl]-3-(pyridin-3-yl)-2-(pyridin-2-yl)methyl-2,6-dihydro-7H-pyrazolo[4,3-d]pyrimidin-7-one

Obtained as a solid (50%) from the title compound of Preparation 92, using a similar procedure to that described in Example 38.
Found : C, 60.23; H, 5.57; N, 18.11. C₃₁H₃₄N₈O₄S requires C, 60.57; H, *5.57;* N, 18.23%.
δ (CDCl₃) : 1.03 (3H, t), 1.20 (3H, t), 2.05 (2H, m), 2.42 (2H, q), 2.59 (4H, m), 3.18 (4H, m), 4.28 (2H, t), 6.05 (2H, s), 7.19 (3H, m), 7.38 (1H, m), 7.63 (1H, m), 7.89 (1H, d), 8.60 (2H, m), 8.71 (1H, d), 8.92 (1H, s), 9.59 (1H, s), 11.01 (1H, s).

### Example 42

### 5-[5-(4-Ethylpiperazin-1-ylsulphonyl)-2-(n-propoxy)pyridin-3-yl]-3-(pyridin-3-yl)-2-(pyrimidin-2-yl)methyl-2,6-dihydro-7H-pyrazolo[4,3-d]pyrimidin-7-one

A mixture of the title compound of Preparation 93 (200mg, 0.40mmol) and sodium hydride (17mg, 60%, 0.43mmol) in dimethylformamide (5ml) was stirred at room temperature for 2 hours. The title compound of Preparation 95 (55mg, 0.43mmol) was added and the reaction stirred at room temperature for 18 hours, then evaporated under reduced pressure. The residue was purified by column chromatography on silica gel, using an elution gradient of dichloromethane:methanol (97:3 to 85:15) to afford the title compound, (56mg, 23%).
Found : C, 54.99; H, 5.00; N, 21.96. C₂₈H₃₀N₁₀O₄S;0.25CH₂Cl₂ requires C, 54.38; H, 4.93; N, 22.45%.
δ (DMSOd₆) : 0.95 (3H, t), 1.37 (3H, t), 2.34 (2H, q), 2.44 (4H, m), 3.00 (4H, m), 4.55 (2H, q), 6.12 (2H, s), 7.50 (2H, m), 8.38 (1H, s), 8.56 (2H, m), 8.68 (1H, s), 8.78 (2H, m), 9.42 (1H, s), 12.62 (1H, s). LRMS : m/z 603 (M+1)⁺

### Example 43

### 5-[2-Ethoxy-5-(4-ethylpiperazin-1-ylsulphonyl)pyridin-3-yl]-3-(4-methoxyphenyl)-2-methyl-2,6-dihydro-7H-pyrazolo[4,3-d]pyrimidin-7-one

Triethylamine (24ml, 0.17mmol), tri(o-tolyl)phosphine (7mg, 0.02mmol), the title compound of Preparation 101 (48mg, 0.09mmol) and finally tris(dibenzylideneacetone)dipalladium(0) (10mg, 0.01mmol) were added to a solution of (4-methoxyphenyl)tri-n-butyltin (Tetrahedron, 1993; 49(25); 5461) (181mg, 0.45mmol) in acetonitrile (5ml) and the reaction heated under reflux for 18 hours. The cooled mixture was evaporated under reduced pressure and the residue purified twice by column chromatography on silica gel, using an elution gradient of methanol:ethyl acetate (5:95 to 10:90). This product was triturated with diethyl ether to afford the title compound (22mg, 43%) as a pale yellow solid.
δ (CDCl₃) : 1.02 (3H, t), 1.60 (3H, t), 2.40 (2H, q), 2.55 (4H, m), 3.12 (4H, m), 3.92 (3H, s), 4.19 (3H, s), 4.77 (2H, q), 7.08 (2H, d), 7.60 (2H, d), 8.62 (1H, s), 8.98 (1H, s), 10.70 (1H, s).
LRMS : m/z 554 (M+1)⁺

### Example 44

### 5-[2-Ethoxy-5-(4-ethylpiperazin-1-ylsulphonyl)pyridin-3-yl]-2-methyl-3-(4-trifluoromethoxyphenyl)-2,6-dihydro-7H-pyrazolo[4,3-d]pyrimidin-7-one

Sodium nitrite (38mg, 0.55mmol) was added to a cooled (-10°C) solution of the title compound of preparation 138 (120mg, 0.27mmol) in acetic acid (4ml) and concentrated hydrochloric acid (4ml), and the solution stirred at 0°C for 90 minutes. The solution was re-cooled to -30°C, liquid sulphur dioxide (4ml) added, followed by a solution of copper (II) chloride (108mg, 0.80mmol) in water (5 drops). The reaction mixture was stirred at 0°C for 30 minutes and at room temperature for an additional 90 minutes. The mixture was then diluted with dichloromethane, and the phases separated. The aqueous phase was extracted with dichloromethane, the combined organic solutions dried (MgSO₄), and evaporated under reduced pressure. The residue was azeotroped with toluene to give a yellow solid. A solution of this intermediate sulphonyl chloride in dichloromethane was cooled in ice. Triethylamine (120µl, 0.86mmol) and N-ethylpiperazine (70µl, 0.54mmol) were added and the reaction stirred at room temperature for 20 hours. The reaction was washed with saturated aqueous sodium bicarbonate solution, and brine, then dried (MgSO₄) and concentrated under reduced pressure. The crude product was purified by column chromatography on silica gel using dichloromethane:methanol (95:5) as eluant to afford the title compound, (84mg, 51%) as a white solid.
δ (CDCl₃) : 1.02 (3H, t), 1.58 (3H, t), 2.20 (2H, q), 2.54 (4H, m), 3.12 (4H, m), 4.20 (3H, s), 4.78 (2H, q), 7.41 (2H, d), 7.75 (2H, d), 8.62 (1H, s), 8.96 (1H, s), 10.71 (1H, s).
LRMS : m/z 608 (M+1)⁺

### Examples 45 to 53

The following compounds of the general structure: 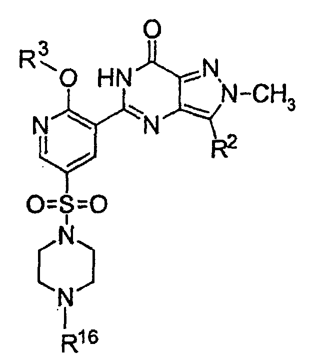 were prepared from the corresponding amino compounds, following a similar procedure to that described in example 44.

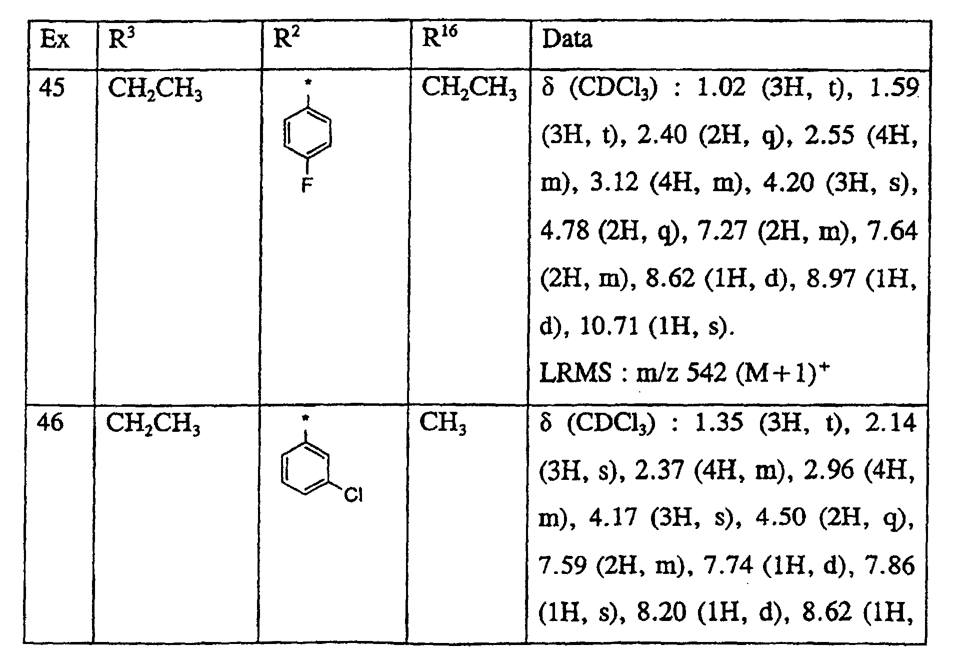

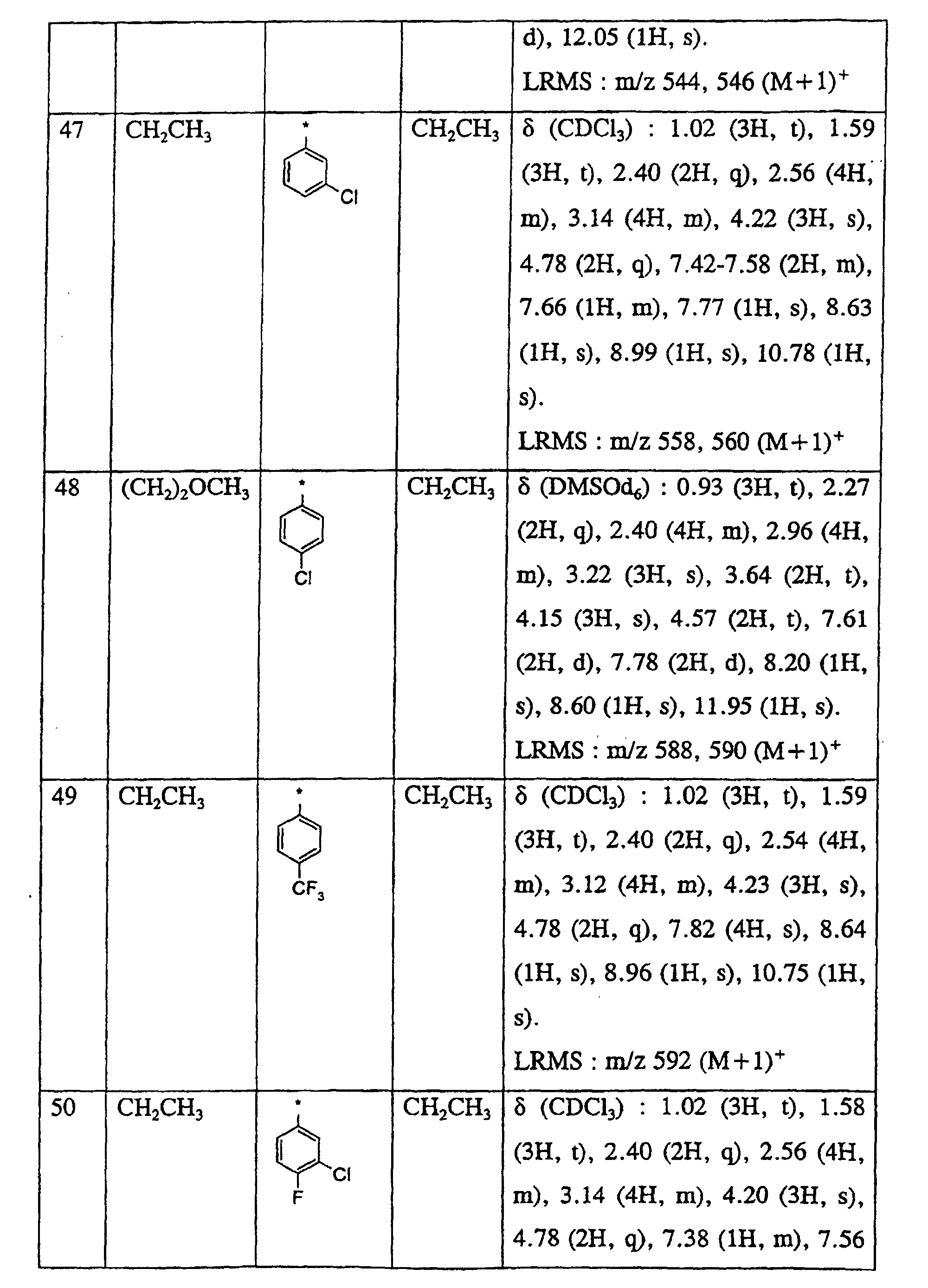

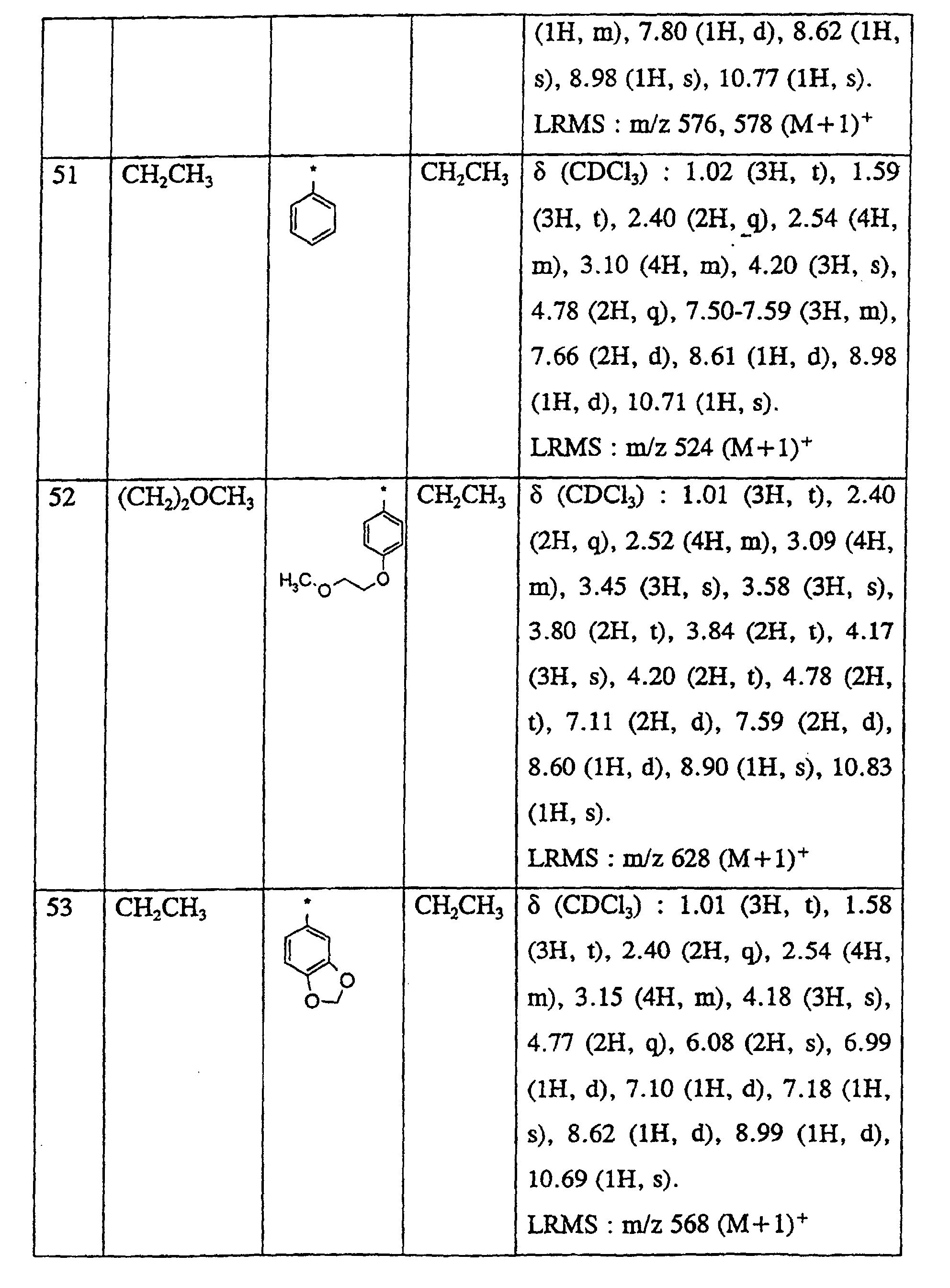

### Example 54

### 5-[2-Ethoxy-5-(4-ethylpiperazin-1-ylsulphonyl)pyridin-3-yl]-3-(2-methoxyphenyl)-2-methyl-2,6-dihydro-7H-pyrazolo[4,3-d]pyrimidin-7-one

A mixture of the title compound of preparation 101 (50mg, 0.095mmol), 2-methoxybenzene boronic acid (19mg, 0.125mmol), cesium fluoride (37.5mg, 0.247mmol), tri(o-tolyl)phosphine (3mg, 0.001mmol) and tris(dibenzylideneacetone)palladium (0) (5mg, 0.005mmol) in 1,2-dimethoxyethane (1ml) was heated under reflux for 18 hours. Tlc analysis showed starting material remaining, so additional tris(dibenzylideneacetone)palladium (0) (5mg, 0.005mmol), tri(o-tolyl)phosphine (9mg, 0.003mmol) and cesium fluoride (9mg, 0.059mmol) were added, and the reaction heated for a further 72 hours under reflux. The cooled reaction mixture was evaporated under reduced pressure. The residue was purified by column chromatography on silica gel using diethylamine:ethyl acetate (95:5) as eluant, and repeated using ethyl acetate as eluant. The product was triturated with diethyl ether to afford the title compound, (6mg, 11 %) as a solid.
δ (CDCl₃) : 1.02 (3H, t), 1.58 (3H, m), 2.40 (2H, q), 2.50 (4H, m), 3.08 (4H, m), 3.86 (3H, s), 4.02 (3H, s), 4.76 (2H, q), 7.14 (2H, m), 7.41 (1H, d), 7.54 (1H, m), 8.60 (1H, d), 8.94 (1H, d), 10.64 (1H, s).
LRMS : m/z 554 (M+1)⁺

### Example 55

### 3-(4-Cyanophenyl)-5-[5-(4-ethylpiperazin-1-ylsulphonyl)-2-(2-methoxyethoxy)pyridin-3-yl]-2-methyl-2,6-dihydro-7H-pyrazolo[4,3-d]pyrimidin-7-one

A mixture of the title compound of preparation 122 (100mg, 0.18mmol), potassium carbonate (50mg, 0.36mmol), and 4-cyanophenylboronic acid (32mg, 0.22mmol) in dioxan (5ml) and water (1ml) was de-gassed and placed under a nitrogen atmosphere. Tetrakis(triphenylphosphine)-palladium (0) (20mg, 0.017mmol) was added and the reaction heated under reflux for 2 hours. The cooled reaction mixture was concentrated under reduced pressure and the residue partitioned between water and dichloromethane. The layers were separated, the aqueous phase extracted with dichloromethane (2x), and the combined organic extracts dried (MgSO₄) and evaporated under reduced pressure. The residue was purified by medium pressure column chromatography on silica gel using an elution gradient of dichloromethane:methanol (99:1 to 98:2) to afford the title compound, (27mg, 26%) as a white solid.
δ (CDCl₃) : 1.02 (3H, t), 2.41 (2H, q), 2.56 (4H, m), 3.12 (4H, m), 3.58 (3H, s), 3.84 (2H, t), 4.22 (3H, s), 4.79 (2H, t), 7.83 (4H, m), 8.62 (1H, d), 8.96 (1H, d), 10.96 (1H, s).
LRMS : m/z 579 (M+1)⁺

### Example 56

### 5-[5-(4-Ethylpiperazin-1-ylsulphonyl)-2-(2-methoxyethoxy)pyridin-3-yl]-2-methyl-3-(pyridin-3-yl)-2,6-dihydro-7H-pyrazolo[4,3-d]pyrimidin-7-one

A mixture of the title compound of preparation 122 (152mg, 0.27mmol), 3-pyridyl boronic acid hydrochloride (56mg, 0.35mmol), and potassium carbonate (113mg, 0.82mmol) in dioxan (4ml) and water (1ml) was de-gassed and placed under an atmosphere of nitrogen. Tetrakis(triphenylphosphine)palladium (0) (31mg, 0.027mmol) was added and the reaction heated under reflux for 90 minutes. The cooled reaction mixture was concentrated under reduced pressure and the residue partitioned between ethyl acetate and water. The resulting suspension was filtered through Celite®, and the filtrate separated. The organic layer was washed with aqueous sodium bicarbonate solution, then brine, dried (MgSO₄) and evaporated under reduced pressure. The residue was triturated with a small volume of ethyl acetate, the solid filtered and dried to afford the title compound, (101mg, 67%) as a light brown solid.
δ (CDCl₃) : 1.02 (3H, t), 2.40 (2H, q), 2.54 (4H, m), 3.10 (4H, m), 3.59 (3H, s), 3.85 (2H, t), 4.20 (3H, s), 4.79 (2H, t), 7.52 (1H, m), 8.01 (1H, m), 8.82 (1H, m), 8.76 (1H, d), 8.90 (2H, m), 10.94 (1H, s).
LRMS : m/z 555 (M+1)⁺

### Example 57

### 3-(6-Aminopyridin-3-yl)-5-[2-ethoxy-5-(4-ethylpiperazin-1-ylsulphonyl)pyridin-3-yl]-2-methyl-2,6-dihydro-7H-pyrazolo[4,3-d]pyrimidin-7-one

*n*-Butyllithium (3.5ml, 1.6M in hexanes, 5.6mmol) was added dropwise to a cooled (-78°C) solution of the title compound of preparation 143 (500mg, 2.27mmol) in tetrahydrofuran (5ml), and the solution stirred for 30 minutes. Triisopropyl borate (0.79ml, 3.29mmol) was added dropwise and the mixture allowed to warm to room temperature over 3 hours. The reaction was quenched by the addition of hydrochloric acid (2N), then evaporated under reduced pressure to give a yellow solid, 1.2g. A mixture of the title compound of preparation 101 (100mg, 0.19mmol), the intermediate boronic acid hydrochloride (120mg), potassium carbonate (104mg, 0.75mmol), and tetrakis(triphenylphosphine)palladium (0) (20mg, 0.017mmol) in dioxan (5ml) and water (1ml) was heated under reflux for 3 hours. Tlc analysis showed starting material remaining, so additional boronic acid (120mg), potassium carbonate (104mg, 0.75mmol) and tetrakis(triphenylphosphine)palladium (0) (20mg, 0.017mmol) were added, and the reaction heated under reflux for a further 18 hours. The cooled reaction mixture was concentrated under reduced pressure and the residue partitioned between water and dichloromethane. The phases were separated, the organic layer washed with brine, dried (MgSO₄) and evaporated under reduced pressure. The crude product was purified by medium pressure column chromatography on silica gel using dichloromethane:methanol (95:5) as eluant to afford the title compound, (17mg, 17%) as a yellow solid.
δ (CDCl₃) : 1.02 (3H, t), 1.59 (3H, t), 2.40 (2H, q), 2.56 (4H, m), 3.12 (4H, m), 4.18 (3H, s), 4.76 (4H, m), 6.68 (1H, d), 7.78 (1H, dd), 8.35 (1H, d), 8.61 (1H, d), 8.98 (1H, d), 10.71 (1H, s).
LRMS : m/z 540 (M+1)⁺

### Example 58

### 5-[2-Ethoxy-5-(4-ethylpiperazin-1-ylsulphonyl)pyridin-3-yl]-2-methyl-3-[6-(methylamino)pyridin-3-yl]-2,6-dihydro-7H-pyrazolo[4,3-d]pyrimidin-7-one

A mixture of the title compounds of preparations 101 (100mg, 0.19mmol) and 147 (75mg, 0.40mmol), potassium carbonate (104mg, 0.75mmol), and tetrakis(triphenylphosphine)palladium (0) (20mg, 0.017mmol) in dioxan (5ml) and water (1ml) was heated under reflux for 4 hours. Tlc analysis showed starting material remaining, so additional boronic acid (75mg, 0.40mmol), tetrakis(triphenylphosphine)palladium (0) (20mg, 0.017mmol) and potassium carbonate (50mg, 0.36mmol) were added and the reaction continued for a further 2 hours. The cooled mixture was concentrated under reduced pressure, and the residue partitioned between water and dichloromethane and the phases separated. The aqueous layer was extracted with dichloromethane and the combined organic solutions washed with brine, dried (MgSO₄) and evaporated under reduced pressure. The residue was triturated with diethyl ether, and the resulting solid, filtered and dried. The crude product was purified by medium pressure column chromatography using an elution gradient of dichloromethane:methanol (99:1 to 97:3) and triturated with diethyl ether to afford the title compound, (63mg, 60%) as a yellow solid.
δ (CDCl₃) : 1.01 (3H, t), 1.58 (3H, t), 2.40 (2H, q), 2.54 (4H, m), 3.02 (3H, d), 3.10 (4H, m), 4.18 (3H, s), 4.76 (2H, q), 4.80 (1H, m), 6.58 (1H, d), 7.78 (1H, d), 8.37 (1H, s), 8.60 (1H, s), 8.98 (1H, s), 10.70 (1H, s).
LRMS : m/z 554 (M+1)⁺

### Example 59

### 3-(6-Dimethylaminopyridin-3-yl)-5-[5-(4-ethylpiperazin-1-ylsulphonyl)-2-(2-methoxyethoxy)pyridin-3-yl]-2-methyl-2,6-dihydro-7H-pyrazolo[4,3-d]pyrimidin-7-one

The title compound was obtained as a yellow solid (66%), from the title compounds of 122 and 148, following the procedure described in example 55.
δ (CDCl₃) : 1.01 (3H, t), 2.40 (2H, q), 2.54 (4H, m), 3.12 (4H, m), 3.19 (6H, s), 3.58 (3H, s), 3.84 (2H, t), 4.18 (3H, s), 4.78 (2H, t), 6.66 (1H, d), 7.78 (1H, d), 8.41 (1H, s), 8.60 (1H, s), 8.90 (1H, s), 10.83 (1H, s).
LRMS : m/z 598 (M+1)⁺

### Example 60

### 3-[6-(Azetidin-1-yl)pyridin-3-yl]-5-[5-(4-ethylpiperazin-1-ylsulphonyl)-2-(2-methoxyethoxy)pyridin-3-yl]-2-methyl-2,6-dihydro-7H-pyrazolo[4,3-d]pyrimidin-7-one

*n*-Butyllithium (3.0ml, 1.6M in hexanes, 4.8mmol) was added dropwise to a cooled (-70°C) solution of the title compound of preparation 144 (900mg, 4.22mmol) in tetrahydrofuran (10ml), and the solution stirred for 30 minutes. A solution of triisopropyl borate (1.46ml, 6.33mmol) in tetrahydrofuran (4ml) was added dropwise, and the reaction then allowed to warm to room temperature over 3 hours. The reaction was quenched by the addition of hydrochloric acid (2N), and the mixture then evaporated under reduced pressure. A mixture of the title compound of preparation 122 (65mg, 0.117mmol), potassium carbonate (65mg, 0.47mmol), the intermediate boronic acid (50mg), and tetrakis(triphenylphosphine)palladium (0) (15mg, 0.013mmol) in dioxan (5ml) and water (1.5ml) was heated under reflux for 2 hours. Tic analysis showed starting material remaining, so additional crude boronic acid (50mg) and tetrakis(triphenylphosphine)palladium (0) (15mg, 0.013mmol) were added and the reaction continued for a further 3 hours. The cooled mixture was concentrated under reduced pressure, and the residue partitioned between water and dichloromethane and the phases separated. The aqueous layer was extracted with dichloromethane and the combined organic solutions washed with brine, dried (MgSO₄) and evaporated under reduced pressure. The crude product was purified by medium pressure column chromatography on silica gel using an elution gradient of dichloromethane:methanol (99:1 to 97:3), then triturated several times with diethyl ether to afford the title compound, (22mg, 31 %) as a solid.
δ (CDCl₃) : 1.01 (3H, t), 2.40 (2H, q), 2.46 (2H, m), 2.54 (4H, m), 3.10 (4H, m), 3.58 (3H, s), 3.86 (2H, t), 4.16 (7H, m), 4.78 (2H, t), 6.40 (1H, d), 7.78 (1H, dd), 8.38 (1H, d), 8.60 (1H, d), 8.92 (1H, d), 10.82 (1H, s).
LRMS : m/z 610 (M+1)⁺

### Example 61

### 5-[2-Ethoxy-5-(4-ethylpiperazin-1-ylsulphonyl)pyridin-3-yl]-3-(furan-2-yl)-2-methyl-2,6-dihydro-7H-pyrazolo[4,3-d]pyrimidin-7-one

The tide compound was prepared from the title compound of preparation 101 and furan-2-boronic acid, following a similar procedure to that described in example 56. The crude product was purified by column chromatography on silica gel using dichloromethane:methanol (100:0 to 97:3) as eluant, to afford the desired compound, (100mg, 68%).
δ (CDCl₃) : 1.01 (3H, t), 1.58 (3H, t), 2.40 (2H, q), 2.57 (4H, m), 3.16 (4H, m), 4.40 (3H, s), 4.78 (2H, q), 6.66 (1H, m), 7.28 (1H, m), 7.64 (1H, s), 8.63 (1H, d), 9.09 (1H, d), 10.75 (1H, s).
LRMS : m/z 514 (M+1)⁺

### Example 62

### 5-[2-Ethoxy-5-(4-ethylpiperazin-1-ylsulphonyl)pyridin-3-yl]-3-(furan-3-yl)-2-methyl-2,6-dihydro-7H-pyrazolo[4,3-d]pyrimidin-7-one

The title compound was prepared from the title compound of preparation 101 and furan-3-boronic acid, following a similar procedure to that described in example 56. The crude product was purified by reverse phase column chromatography on polystyrene gel, using an elution gradient of 0.1% aqueous trifluoroacetic acid:acetonitrile (90:10 to 20:80), to afford the title compound (9.8mg, 10%).
δ (CDCl₃) : 1.02 (3H, t), 1.59 (3H, t), 2.40 (2H, q), 2.57 (4H, m), 3.16 (4H, m), 4.24 (3H, s), 4.77 (2H, q), 7.05 (1H, s), 7.62 (1H, s), 8.03 (1H, s), 8.62 (1H, d), 9.02 (1H, d), 10.71 (1H, s).
LRMS : m/z 514 (M+1)⁺

### Example 63

### 5-[2-Ethoxy-5-(4-ethylpiperazin-1-ylsulphonyl)pyridin-3-yl]-3-(3-methoxyphenyl)-2-methyl-2,6-dihydro-7H-pyrazolo[4,3-d]pyrimidin-7-one

Tris(dibenzylideneacetone)palladium (0) (9mg, 0.009mmol) was added to a mixture of the title compounds of preparation 101 (44mg, 0.083mmol), and 3-methoxyphenyl tri-n-butylstannane (75mg, 0.19mmol), tri(o-tolyl)phosphine (7mg, 0.023mmol) and triethylamine (21µl, 0.15mmol) in acetonitrile (2ml) and the reaction mixture heated under reflux for 18 hours. Tlc analysis showed starting material remaining, so additional stannane (90mg, 0.23mmol), tri(o-tolyl)phosphine (7mg, 0.023mmol), tris(dibenzylideneacetone)palladium (0) (9mg, 0.009mmol) and triethylamine (21µl, 0.15ml) were added, and the reaction heated under reflux for a further 72 hours. The cooled mixture was purified directly by column chromatography on silica gel using ethyl acetate:methanol (95:5) as eluant. The crude product was triturated with diethyl ether to afford the title compound, (5mg, 11 %) as a solid.
δ (CDCl₃) : 1.00 (3H, t), 1.58 (3H, t), 2.40 (2H, q), 2.53 (4H, m), 3.10 (4H, m), 3.92 (3H, s), 4.22 (3H, s), 4.78 (2H, q), 7.04 (1H, m), 7.20 (1H, d), 7.34 (1H, s), 7.47 (1H, m), 8.62 (1H, s), 9.00 (1H, s), 10.76 (1H, s).
LRMS : m/z 554 (M+1)⁺

### Example 64

### 5-[5-(4-Ethylpiperazin-1-ylsulphonyl)-2-(2-methoxyethoxy)pyridin-3-yl]-3-(5-methylpyridin-2-yl)-2-methyl-2,6-dihydro-7H-pyrazolo[4,3-d]pyrimidin-7-one

Tetrakis(triphenylphosphine)palladium (0) (31mg, 0.027mmol) was added to a mixture of the title compounds of preparations 122 (150mg, 0.27mmol), and 149 (154mg, 0.40mmol), lithium chloride (113mg, 2.69mmol) and copper (I) iodide (8mg, 0.04mmol) in dioxan (6ml) and the reaction heated under reflux under nitrogen for 18 hours. The cooled reaction was partitioned between water and ethyl acetate, the layers separated, and the organic phase dried (MgSO₄), and evaporated under reduced pressure. The residue was purified by column chromatography on silica gel using dichloromethane:methanol (95:5) as eluant and triturated with an diethyl ether:isopropyl alcohol solution (50:50) to afford the title compound, (92mg, 60%) as a yellow solid.
δ (CDCl₃) : 1.02 (3H, t), 2.40 (5H, m), 2.56 (4H, m), 3.16 (4H, m), 3.60 (3H, s), 3.88 (2H, t), 4.56 (3H, s), 4.80 (2H, t), 7.65 (1H, s), 8.36 (1H, d), 8.59 (1H, s), 8.63 (1H, s), 9.00 (1H, s), 10.90 (1H, s).
LRMS : mlz 569 (M+1)⁺
Found: C, 52.59; H, 5.43; N, 18.82. C₂₆H₃₂N₈O₅S:1.5H₂O requires, 52.48; H, 5.92; N, 18.81%.

### Example 65

### 5-[5-(4-Ethylpiperazin-1-ylsulphonyl)-2-(2-methoxyethoxy)pyridin-3-yl]-3-(6-ethylpyridin-3-yl)-2-methyl-2,6-dihydro-7H-pyrazolo[4,3-d]pyrimidin-7-one

The title compound was obtained (57%), after crystallistion from isopropyl alcohol, as a white powder, from the title compounds of preparations 122 and 150, following a similar procedure to that described in example 64.
δ (CDCl₃) : 1.02 (3H, t), 1.40 (3H, t), 2.40 (2H, q), 2.55 (4H, m), 2.97 (2H, q), 3.12 (4H, m), 3.59 (3H, s), 3.86 (2H, t), 4.20 (3H, s), 4.80 (2H, t), 7.40 (1H, d), 7.96 (1H, d), 8.61 (1H, s), 8.80 (1H, s), 8.88 (1H, s), 10.91 (1H, s).
LRMS : m/z 583 (M+1)⁺
Found: C, 54.40; H, 5.91; N, 18.78. C₂₇H₃₄N₈O₅S;0.5H₂O requires C, 54.81; H, 5.96; N, 18.94%.

### Example 66

### 3-(2-Aminopyrimidin-5-yl)-5-[2-ethoxy-5-(4-ethylpiperazin-1-ylsulphonyl)pyridin-3-yl]-2-methyl-2,6-dihydro-7H-pyrazolo[4,3-d]pyrimidin-7-one

The title compound was prepared from the tide compounds of preparations 101 and 153, following a similar procedure to that described in example 64. The crude product was purified by column chromatography on silica gel using an elution gradient of dichloromethane:methanol:acetic acid (100:0:0 to 95:5:0 to 90:10:1) to give the desired product (30mg, 21 %) as a yellow solid.
δ (CDCl₃) : 1.02 (3H, t), 1.58 (3H, t), 2.43 (2H, q), 2.59 (4H, m), 3.16 (4H, m), 3.28-3.72 (2H, br, s), 4.18 (3H, s), 4.77 (2H, q), 8.58 (2H, s), 8.62 (1H, s), 8.92 (1H, d), 10.84 (1H, s).

### Example 67

### 3-(2-Dimethylamino-pyrimidin-5-yl)-5-[5-(4-ethylpiperazin-1-ylsulphonyl)-2-(2-methoxyethoxy)pyridin-3-yl]-2-methyl-2,6-dihydro-7H-pyrazolo[4,3-d]pyrimidin-7-one

A mixture of the title compounds of preparations 122 (100mg, 0.18mmol) and 148 (77mg, 0.27mmol), copper (I) iodide (5mg, 0.027mmol), and lithium chloride (76mg, 1.8mmol) in dioxan (5ml) was de-gassed, and placed under an atmosphere of nitrogen. Tetrakis(triphenylphosphine)palladium (0) (20mg, 0.017mmol) was added and the reaction mixture heated under reflux for 5 ½ hours. Tlc analysis showed no stannane remaining, so additional tetrakis(triphenylphosphine)palladium (0) (20mg, 0.017mmol), copper (I) iodide (5mg, 0,027mmol) and the title compound of preparation 148 (25mg, 0.087mmol) were added, and the reaction heated for a further hour under reflux. The cooled reaction was diluted with aqueous 10% potassium fluoride solution (5ml), the mixture stirred for 20 minutes, then filtered through Celite® washing through well with dichloromethane. The filtrate was separated, the aqueous layer extracted with dichloromethane, and the combined organic solutions dried (MgSO₄) and evaporated under reduced pressure. The residue was purified by column chromatography on silica gel using an elution gradient of dichloromethane:methanol (100:0 to 97:3) to give a gum. This was crystallised from isopropyl alcohol, the solid filtered and triturated with pentane to afford the title compound, (66mg, 61 %) as a solid.
δ (CDCl₃) : 1.03 (3H, t), 2.41 (2H, q), 2.57 (4H, m), 3.16 (4H, m), 3.30 (6H, s), 3.58 (3H, s), 3.88 (2H, t), 4.18 (3H, s), 4.79 (2H, t), 8.60 (2H, s), 8.62 (1H, d), 8.92 (1H, d), 10.86 (1H, s).
LRMS : m/z 599 (M+1)⁺

### Example 68

### 5-[5-(4-Ethylpiperazin-1-ylsulphonyl)-2-(2-methoxyethoxy)pyridin-3-yl]-3-(6-methoxypyridin-3-yl)-2-methyl-2,6-dihydro-7H-pyrazolo[4,3-d]pyrimidin-7-one

The title compound was prepared from the title compound of preparation 122 and 2-methoxy-5-(tri-n-butylstannyl)pyridine, following a similar procedure to that described in example 67. The crude product was purified by medium pressure column chromatography on silica gel using dichloromethane:methanol (97:3) as eluant, and triturated with diethyl ether to afford the desired compound, (12.6mg, 12%) as a yellow solid.
δ (CDCl₃) : 1.02 (3H, t), 2.40 (2H, q), 2.55 (4H, m), 3.10 (4H, m), 3.58 (3H, s), 3.85 (3H, s), 3.84 (2H, t), 4.02 (3H, s), 4.18 (3H, s), 4.78 (2H, q), 6.95 (1H, d), 7.88 (1H, dd), 8.43 (1H, s), 8.61 (1H, d), 8.90 (1H, d), 10.88 (1H, s).
LRMS : m/z 585 (M+1)⁺

### Example 69

### 5-[2-Ethoxy-5-(4-ethylpiperazin-1-ylsulphonyl)pyridin-3-yl]-2-methyl-3-(pyrazin-2-yl)-2,6-dihydro-7H-pyrazolo[4,3-d]pyrimidin-7-one

The title compound was obtained (70%) from the title compounds of preparations 101 and 151, following a similar procedure to that described in example 68.
δ (CDCl₃) : 1.02 (3H, t), 1.60 (3H, t), 2.41 (2H, q), 2.58 (4H, m), 3.19 (4H, m), 4.56 (3H, s), 4.78 (2H, q), 8.58 (1H, d), 8.68 (2H, s), 9.07 (1H, d), 9.74 (1H, s), 10.78 (1H, s).
LRMS : m/z 526 (M+1)⁺

### Example 70

### 3-(2-Chloropyrimidin-5-yl)-5-[2-ethoxy-5-(4-ethylpiperazin-1-ylsulphonyl)pyridin-3-yl]-2-methyl-2,6-dihydro-7H-pyrazolo[4,3-d]pyrimidin-7-one

A mixture of the title compounds from preparations 101 (150mg, 0.285mmol) and 152 (142mg, 0.43mmol), copper (I) iodide (8mg, 0.042mmol), and lithium chloride (120mg, 2.85mmol) in dioxan (10ml) was de-gassed, and placed under an atmosphere of nitrogen. Tetrakis(triphenylphosphine)palladium (0) (33mg, 0.029mmol) was added and the reaction mixture heated under reflux for 6 hours. Tlc analysis showed no stannane remaining, so additional tetrakis(triphenylphosphine)palladium (0) (33mg, 0.029mmol), and stannane (143mg, 0.43mmol) were added, and the reaction heated for a further 18 hours under reflux. Further tetrakis(triphenylphosphine)palladium (0)(33mg, 0.029mmol), and stannane (143mg, 0.43mmol) were added, and the reaction heated for an additional 12 hours. The cooled mixture was partitioned between dichloromethane and brine, the layers separated and the organic phase dried (MgSO₄) and evaporated under reduced pressure. The residue was purified by column chromatography on silica gel using dichloromethane as eluant. The crude product was further purified by HPLC using a reverse phase silica gel column, and an elution gradient of 0.1 % aqueous trifluoroacetic acid:acetonitrile (90:10 to 20:80). The combined column fractions were basified to pH 8 using sodium carbonate, and the mixture extracted with dichloromethane. The combined organic solutions were dried (MgSO₄) and evaporated under reduced pressure to afford the title compound, 22mg, 14%. δ (CDCl₃) : 1.10 (3H, m), 1.58 (3H, m), 2.41-2.71 (6H, m), 3.18 (4H, m), 4.26 (3H, s), 4.78 (2H, q), 8.68 (1H, d), 8.96 (1H, d), 9.02 (2H, s), 10.81 (1H, s).
LRMS : m/z 560 (M+1)⁺

### Example 71

### 5-[5-(4-Ethylpiperazin-1-ylsulphonyl)-2-(2-methoxyethoxy)pyridin-3-yl]-3-(3-imidazo[1,2-a]pyridin-6-yl)-2-methyl-2,6-dihydro-7H-pyrazolo[4,3-d]pyrimidin-7-one trifluoroacetate

The title compound was prepared from the title compounds of preparations 122 and 154, following a similar procedure to that described in example 66. The product was further purified by HPLC using a reverse phase silica gel column, and an elution gradient of 0.1% aqueous trifluoroacetic acid:acetonitrile (98:2 to 70:30) to afford the title compound (16mg, 8.4%) as a white solid.
δ (CDCl₃) : 1.40 (3H, t), 2.20 (6H, m), 2.98 (2H, m), 3.16 (2H, q), 3.59 (3H, s), 3.72 (2H, m), 3.96 (2H, t), 4.30 (3H, s), 4.80 (2H, t), 7.99 (1H, d), 8.17 (1H, m), 8.64 (1H, d), 8.77 (1H, d), 9.00 (1H, s), 1.08 (1H, s).
LRMS : m/z 594 (M+1)⁺

### Example 72

### 5-[2-Ethoxy-5-(4-ethylpiperazin-1-ylsulphonyl)pyridin-3-yl]-3-(1-ethylpyrazol-4-yl)-2-methyl-2,6-dihydro-7H-pyrazolo[4,3-d]pyrimidin-7-one

The title compound was prepared from the title compounds of preparations 101 and 155, following a similar procedure to that described in example 70. The crude product was triturated with an diethyl ether:methanol (95:5) solution, the resulting solid filtered, and recrystallised from isopropyl alcohol to afford the desired compound, (73mg, 47%) as a yellow solid.
δ (CDCl₃) : 1.00 (3H, t), 1.58 (6H, m), 2.40 (2H, q), 2.56 (4H, m), 3.14 (4H, m), 4.25 (3H, s), 4.34 (2H, q), 4.78 (2H, q), 7.99 (1H, s), 8.18 (1H, s), 8.63 (1H, d), 9.04 (1H, d), 10.70 (1H, s).
LRMS : m/z 542 (M+1)⁺

### Example 73

### 5-[5-(4-Ethylpiperazin-1-ylsulphonyl)-2-(methoxyethoxy)pyridin-3-yl]-3-(1-ethylpyrazol-4-yl)-2-methyl-2,6-dihydro-7H-pyrazolo[4,3-d]pyrimidin-7-one

Potassium bis(trimethylsilyl)amide (59mg, 0.295mmol) was added to a suspension of the title compound of example 72 (40mg, 0.073mmol) in 2-methoxyethanol (6ml), and the reaction heated under reflux for 18 hours. The cooled mixture was evaporated under reduced pressure and the residue purified by column chromatography on silica gel using dichloromethane:methanol (95:5) as eluant. The product was recrystallised from isopropyl alcohol to afford the title compound, (22mg, 53%) as a yellow solid.
δ (CDCl₃) : 1.01 (3H, t), 1.60 (3H, t), 2.41 (2H, m), 2.58 (4H, m), 3.15 (4H, m), 3.58 (3H, s), 3.86 (2H, t), 4.22 (3H, s), 4.30 (2H, q), 4.78 (2H, t), 7.98 (1H, s), 8.17 (1H, s), 8.61 (1H, d), 8.99 (1H, d), 10.76 (1H, s).
LRMS : m/z 572 (M+1)⁺

### Example 74

### 5-[5-(4-Ethylpiperazin-1-ylsulphonyl)-2-(2-methoxyethoxy)pyridin-3-yl]-2-methyl-3-phenyl-2,6-dihydro-7H-pyrazolo[4,3-d]pyrimidin-7-one

Potassium bis(trimethylsilyl)amide (115mg, 0.58mmol) was added to a solution of the title compound from example 51 (76mg, 0.145mmol) in 2-methoxyethanol (3ml), and the reaction heated at 120°C for 3 hours under a nitrogen atmosphere. The cooled mixture was neutralised using hydrochloric acid (1N), and concentrated under reduced pressure. The residue was partitioned between dichloromethane and aqueous sodium bicarbonate solution, and the layers separated. The organic phase was washed with additional aqueous sodium bicarbonate solution, and brine, then dried (MgSO₄) and evaporated under reduced pressure. The crude product was purified by column chromatography on silica gel using an elution gradient of dichloromethane:methanol (100:0 to 97:3) to afford the tide compound (66mg, 82%).
δ (CDCl₃) : 1.01 (3H, t), 2.40 (2H, q), 2.52 (4H, m), 3.10 (4H, m), 3.58 (3H, s), 3.85 (2H, t), 4.20 (3H, s), 4.78 (2H, t), 7.48-7.59 (3H, m), 7.65 (2H, m), 8.61 (1H, d), 8.92 (1H, d), 10.88 (1H, s).
LRMS : m/z 554 (M+1)⁺

### Examples 75 to 77

The following compounds of the general structure: 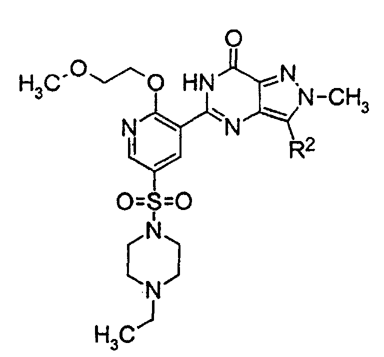 were prepared from the corresponding pyrazolo[4,3-d]pyrimidin-7-one, following a similar procedure to that described in example 74.

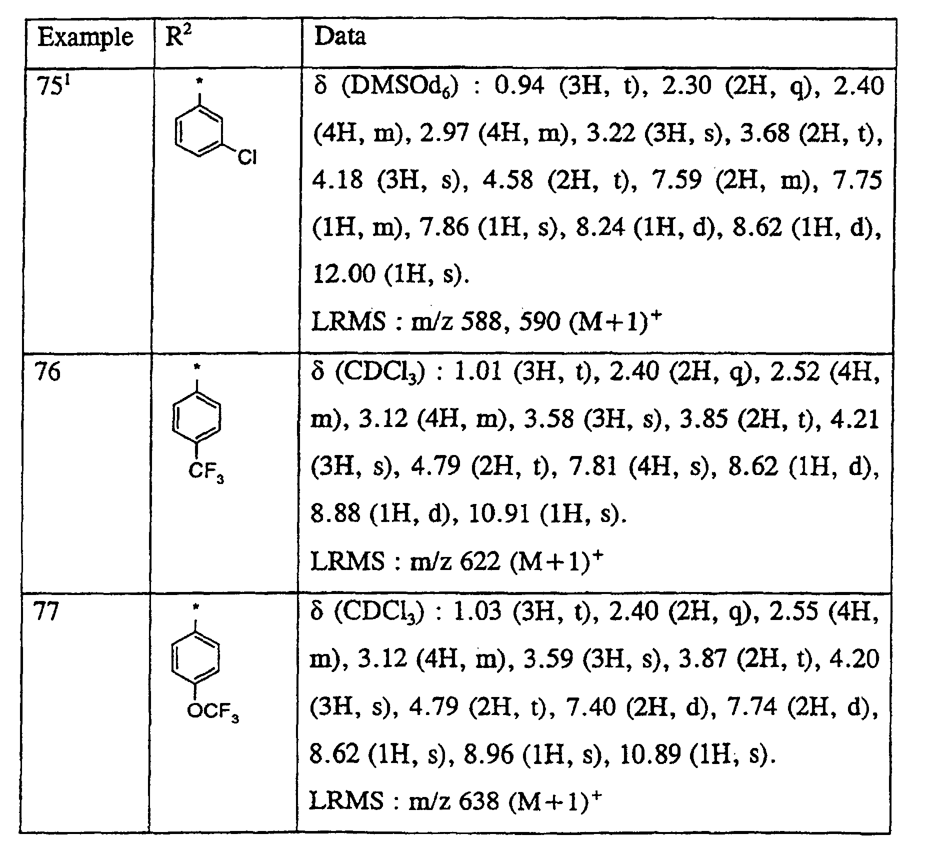

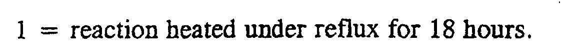

### Example 78

### 5-[2-Ethoxy-5-(4-ethylpiperazin-1-ylsulphonyl)pyridin-3-yl]-2-methyl-3-(5-methyl-1,3,4-oxadiazol-2-yl)-2,6-dihydro-7H-pyrazolo[4,3-d]pyrimidin-7-one

Acetic hydrazide (80mg, 1.08mmol), followed by triethylamine (0.34ml, 2.44mmol) were added to a suspension of the title compound of preparation 158 (500mg, 0.98mmol) in dichloromethane (15ml), and the reaction stirred at room temperature for 4 hours. The reaction mixture was partitioned between dichloromethane and aqueous sodium bicarbonate solution, and the layers separated. The aqueous phase was extracted with dichloromethane, and the combined organic solutions dried (Na₂SO₄) and evaporated under reduced pressure, to give a solid, 520mg. Thionyl chloride (5ml) was added to this intermediate hydrazide (350mg), and the solution stirred at 80°C for 3 hours. The cooled reaction was partitioned between dichloromethane and aqueous sodium bicarbonate solution, and the layers separated. The organic phase was dried (Na₂SO₄) and concentrated under reduced pressure. The residue was purified by column chromatography on silica gel using an elution gradient of dichloromethane:methanol (99:1 to 94:6) to give the title compound, 55mg.
δ (CDCl₃) : 1.01 (3H, t), 1.60 (3H, t), 2.41 (2H, q), 2.58 (4H, m), 2.75 (3H, s), 3.17 (4H, m), 4.58 (3H, s), 4.80 (2H, q), 8.69 (1H, s), 9.19 (1H, s), 10.88 (1H, s).

### Example 79

### 5-[2-Ethoxy-5-(4-ethylpiperazin-1-ylsulphonyl)-pyridin-3-yl]-2-methyl-3-(tetrahydrofuran-2-yl)-2,6-dihydro-7H-pyrazolo[4,3-d]pyrimidin-7-one

A mixture of the title compound from example 61 (80mg, 0.16mmol) and 10% palladium on charcoal (10mg) in ethanol (4.5ml) and water (0.5ml) was hydrogenated at 60 psi (414 kPa) and 40°C for 18 hours. The reaction mixture was filtered through Celite®, and the filtrate evaporated under reduced pressure to afford the title compound, (27mg, 32%).
δ (CDCl₃) : 1.01 (3H, t), 1.59 (3H, t), 2.15 (1H, m), 2.26 (1H, m), 2.40 (3H, m), 2.56 (4H, m), 2.86 (1H, m), 3.14 (4H, m), 3.98 (1H, m), 4.02 (1H, m), 4.20 (3H, s), 4.77 (2H, q), 5.36 (1H, m), 8.62 (1H, d), 9.00 (1H, d), 10.68 (1H, s).
LRMS : m/z 518 (M+1)⁺

### Example 80

### 3-Ethoxy-5-[2-ethoxy-5-(4-ethylpiperazin-1-ylsulphonyl)-pyridin-3-yl]-2-methyl-2,6-dihydro-7H-pyrazolo[4,3-d]pyrimidin-7-one

Sodium nitrite (12mg, 0.18mmol) was added to a cooled (-10°C) solution of the title compound of preparation 120 (40mg, 0.12mmol) in acetic acid (0.45ml) and concentrated hydrochloric acid (0.45ml) and the solution allowed to warm to 0°C over 2 hours. The solution was re-cooled to -20°C, liquid sulphur dioxide (0.36ml) and a solution of copper (II) chloride (48mg, 0.48mmol) in water (2ml) and acetic acid (1ml) added, and the reaction then allowed to warm to room temperature over 2 hours. The mixture was extracted with dichloromethane, the combined organic extracts dried (Na₂SO₄), concentrated under reduced pressure and azeotroped with toluene. The brown residue was dissolved in ethanol (10ml), N-ethylpiperazine (50µl, 0.38mmol) added and the reaction stirred at room temperature for 18 hours. The mixture was concentrated under reduced pressure and the crude product purified by column chromatography on silica gel using dichloromethane:methanol:0.88 ammonia (98:2:0.5) to give the title compound, (12mg, 20%).
δ (CDCl₃) : 1.02 (3H, t), 1.57 (6H, m), 2.41 (2H, q), 2.57 (4H, m), 3.12 (4H, m), 3.90 (3H, s), 4.74 (2H, q), 4.88 (2H, q), 8.60 (1H, d), 8.92 (1H, d), 10.56 (1H, s).
LRMS : m/z 492 (M+ 1)⁺

### Example 81

### 5-[2-n-Butoxy-5-(4-ethylpiperazin-1-ylsulphonyl)pyridin-3-yl]-3-ethoxy-2-methyl-2,6-dihydro-7H-pyrazolo[4,3-d]pyrimidin-7-one

Potassium bis(trimethylsilyl)amide (10mg, 0.05mmol) was added to a suspension of the title compound of example 80 (10mg, 0.02mmol) in n-butanol (4ml), and the reaction mixture heated under reflux for 6 hours. The cooled mixture was evaporated under reduced pressure and the residue purified by column chromatography on silica gel using dichloromethane:methanol:0.88 ammonia (98:2:0.5) as eluant, to afford the title compound, (8mg, 76%).
δ (CDCl₃) : 1.02 (6H, t), 1.57 (5H, m), 1.96 (2H, m), 2.42 (2H, q), 2.56 (4H, m), 3.13 (4H, m), 3.90 (3H, s), 4.65 (2H, t), 4.88 (2H, q), 8.60 (1H, s), 8.90 (1H, s), 10.53 (1H, s).
LRMS : m/z 520 (M+1)⁺

### Example 82

### 5-[2-Ethoxy-5-(4-ethylpiperazin-1-ylsulphonyl)-pyridin-3-yl]-3-(1-ethoxyvinyl)-2-methyl-2,6-dihydro-7H-pyrazolo[4,3-d]pyrimidin-7-one

A mixture of the title compound or preparation 101 (100mg, 0.19mmol), lithium chloride (80mg, 1.9mmol), copper (I) iodide (6mg, 0.03mmol) and (1-ethoxyvinyl)(tri-n-butyl)stannane (90mg, 0.247mmol) in dioxan (10ml) was de-gassed and placed under an atmosphere of nitrogen. Tetrakis(triphenylphosphine)palladium (0) (20mg, 0.017mmol) was added, and the reaction heated under reflux for 5 hours. The cooled mixture was concentrated under reduced pressure, and the residue stirred vigorously in a solution of ethyl acetate: 10% aqueous potassium fluoride solution for 10 minutes. The resulting suspension was filtered through Arbocel®, and the filtrate separated. The organic layer was washed with 10% aqueous potassium fluoride solution, then brine, dried (MgSO₄) and evaporated under reduced pressure. The crude product was purified by medium pressure column chromatography on silica gel using an elution gradient of dichloromethane:methanol (100:0 to 98:2) to afford the title compound (84mg, 85%) as a foam.
δ (CDCl₃) : 1.02 (3H, t), 1.46 (3H, t), 1.58 (3H, t), 2.41 (2H, q), 2.57 (4H, m), 3.15 (4H, m), 4.02 (2H, q), 4.24 (3H, s), 4.69 (1H, d), 4.77 (2H, q), 5.23 (1H, d), 8.62 (1H, d), 9.06 (1H, d), 10.70 (1H, s).
LRMS : m/z 518 (M+1)⁺

### Example 83

### N,2-Dimethyl-5-[2-ethoxy-5-(4-ethylpiperazin-1-ylsulphonyl)pyridin-3-yl)-7-oxo-6,7-dihydro-2H-pyrazolo[4,3-d]pyrimidine-3-carboxamide

A mixture of the title compound of preparation 159 (50mg, 0.108mmol), acetyl chloride (7µl, 0.108mmol) and pyridine (9µl, 0.11mmol) in dichloromethane (2ml) was stirred at room temperature for 18 hours. The reaction mixture was evaporated under reduced pressure and the residue purified by column chromatography on silica gel using an elution gradient of dichloromethane:methanol (100:0 to 80:20) to afford the title compound (45mg, 82%).
δ (CDCl₃) : 1.08 (3H, m), 1.58 (3H, t), 2.38 (3H, s), 2.44-2.70 (6H, m), 3.18 (4H, m), 4.01 (3H, s), 4.74 (2H, q), 8.60 (1H, d), 8.95 (1H, d), 10.67 (1H, s).
LRMS : m/z 504 (M+2)⁺

### Biological Activity

Compounds of the invention were found to have *in vitro* activities as inhibitors of cGMP PDE5 with IC₅₀ values of less than about 100 nM.

The following Table illustrates the *in vitro* activities for a range of compounds of the invention as inhibitors of cGMP PDE5.

| Example | IC₅₀ (nM) |
|---|---|
| 5 | 2.80 |
| 8 | 4.10 |
| 19 | 3.40 |
| 33 | 2.80 |
| 42 | 2.26 |

## Claims

1. A compound of formula IA, or of formula IB: 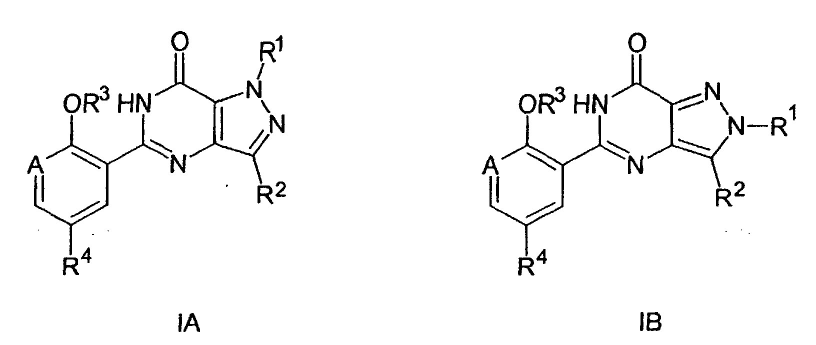 wherein
A represents N;
R¹ represents H, C₁₋₆ alkyl, Het, alkylHet, aryl or alkylaryl, which latter five groups are all optionally substituted (and/or, in the case of C₁₋₆ alkyl, optionally terminated) by one or more substituents selected from halo, cyano, nitro, C₁₋₆ alkyl, OR⁵, C(O)R⁶, C(O)OR⁷, C(O)NR⁸R⁹, NR^{10a}R^{10b} and SO₂NR^{11a}R^{11b};
R² represents C(O)NR¹²R¹³, C(O)OR¹², NR¹²R¹³, N(H)SO₂R¹², N(H)SO₂NR¹²R¹³, N(H)C(O)R¹², OR^{12a}, C₁₋₆ alkyl (which alkyl group is interrupted by one or more of O, S or N(R¹²) and/or substituted or terminated by C(O)NR¹²R¹³, C(O)OR¹² or aryl or Het¹), cyano, aryl or Het¹;
R³, R¹² and R¹³ independently represent H or C₁₋₆ alkyl, which alkyl group is optionally substituted and/or optionally terminated by one or more substituents selected from aryl, Het, halo, cyano, nitro, OR⁵, C(O)R⁶, C(O)OR⁷, C(O)NR⁸R⁹_{,} NR^{10a}R^{10b} and SO₂NR^{11a}R^{11b};
R⁴ represents SO₂NR¹⁴R¹⁵;
R¹⁴ and R¹⁵, together with the nitrogen to which they are attached, form Het;
Het represents an optionally substituted four- to twelve-membered heterocyclic group, which group contains at least one nitrogen atom and, optionally, one or more further heteroatoms selected from nitrogen, oxygen and sulphur;
Het¹ represents an optionally substituted four- to twelve-membered heterocyclic group, which group contains at least one nitrogen atom or at least one oxygen atom and, optionally, one or more further heteroatoms selected from nitrogen, oxygen and sulphur; and
R⁵, R⁶, R⁷, R⁸, R⁹, R^{11a}, R^{11b} and R^{12a} independently represent, at each occurrence when used herein, H or C₁₋₆ alkyl;
R^{10a} and R^{10b}, at each occurrence when used herein, either independently represent, H or C₁₋₆ alkyl or, together with the nitrogen atom to which they are attached, represent azetidinyl, pyrollidinyl or piperidinyl;
or a salt or solvate thereof.

2. A compound as claimed in Claim 1, wherein R¹ represents H, a linear, branched, cyclic, acyclic and/or part cycliclacyclic C₁₋₆ alkyl group, alkylHet, or alkylaryl.

3. A compound as claimed in Claim 1 or Claim 2, wherein R² represents a linear or branched, optionally unsaturated C₁₋₆ alkyl group (which alkyl group is optionally interrupted by one or more of O, S or N(R¹²)), C(O)NR¹²R¹³, NR¹²R¹³, N(H)C(O)R¹², OR^{12a}, aryl or Het¹.

4. A compound as claimed in any one of the preceding claims, wherein R³ represents linear, branched, cyclic and/or acyclic C₁₋₆ alkyl which is optionally substituted or terminated by one or more substituents selected from Het or OR⁵.

5. A compound as claimed in any one of the preceding claims, wherein R¹² and R¹³ independently represent H, or linear or branched C₁₋₆ alkyl, provided that, in the case where R² represents NR¹²R¹³, R¹² and R¹³ do not both represent H.

6. A compound as claimed in any one of the preceding claims, wherein R¹⁴ and R¹⁵, together with the nitrogen to which they are attached represent 4-R¹⁶-piperazinyl, in which R¹⁶ represents H or C₁₋₆ alkyl, which latter group is optionally substituted or terminated by one or more substituents selected from aryl, Het, halo, cyano, nitro, OR⁵, C(O)R⁶, C(O)OR⁷, C(O)NR⁸R⁹_{,} NR^{10a}R^{10b}, SO₂NR^{11a}R^{11b} and N(H)SO₂R¹² wherein R⁵, R⁶, R⁷, R⁸, R⁹, R^{10a}, R^{10b}, R^{11a}, R^{11b} and R¹² are as defined in Claim 1.

7. A compound as defined in any one of Claims 1 to 6 for use as a pharmaceutical.

8. A compound as defined in any one of Claims 1 to 6 for use as an animal medicament.

9. A formulation comprising a compound as defined in any one of Claims 1 to 6 in admixture with a pharmaceutically or veterinarily acceptable adjuvant, diluent or carrier.

10. A formulation as claimed in Claim 9, which is a pharmaceutical formulation.

11. A formulation as claimed in Claim 9, which is a veterinary formulation.

12. The use of a compound as defined any one of Claims 1 to 6 for the manufacture of a medicament for the curative or prophylactic treatment of a medical condition for which inhibition of cGMP PDE5 is desired.

13. Use as claimed in Claim 12, wherein the condition is male erectile dysfunction, female sexual dysfunction, premature labour, dsymenorrhoea, benign prostatic hyperplasia (BPH), bladder outlet obstruction, incontinence, stable or unstable variant (Prinzmetal) angina, hypertension, pulmonary hypertension, congestive heart failure, atherosclerosis, stroke, peripheral vascular disease, conditions of reduced blood vessel patency, chronic asthma, bronchitis, allergic asthma, allergic rhinitis, glaucoma, a disease **characterised by** disorders of gut motility, pre-eclampsia, Kawasaki's syndrome, nitrate tolerance, multiple sclerosis, peripheral diabetic neuropathy, stroke, Alzheimer's disease, acute respiratory failure, psoriasis, skin necrosis, cancer metastasis, baldness, nutcracker oesophagus, anal fissure and hypoxic vasoconstriction.

14. A process for the preparation of a compound of formula IA, or of formula IB, as defined in Claim 1, which comprises:
(a) cyclisation of a corresponding compound of formula IIA, or of formula IIB, respectively: 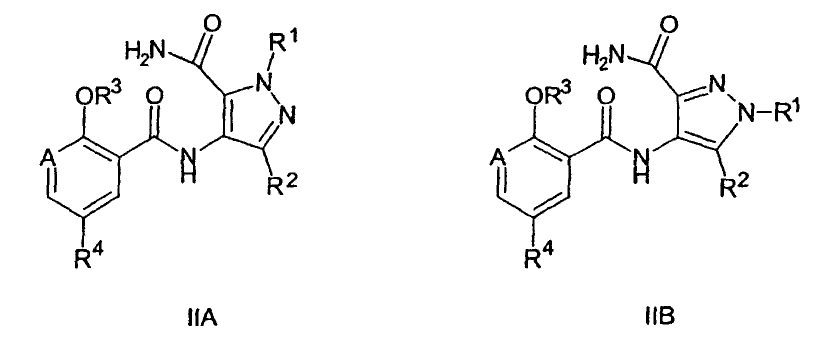 wherein R¹, R², R³, R⁴ and A are as defined in Claim 1;
(b) for compounds of formulae IA and IB, in which R² represents C(O)NR¹²R¹³, reaction of a corresponding compound of formula IA, or of formula IB, in which R² represents C(O)OH (or a carboxylic acid derivative thereof) with a compound of formula
HNR¹²R¹³,
in which R¹² and R¹³ are as defined in Claim 1;
(c) for compounds of formulae IA and IB, in which R² represents C(O)OR¹², cyclisation of a corresponding compound of formula VIA, or of formula VIB, respectively: 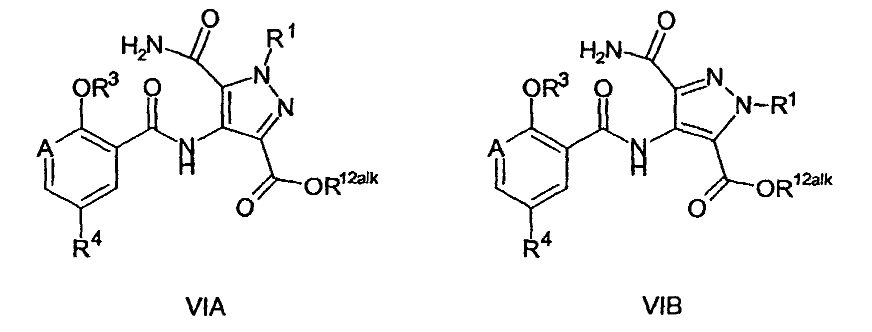 wherein R¹, R³, R⁴ and A are as defined in Claim 1, and R^{12alk} represents an optionally substituted C₁₋₆ alkyl group, followed by removal of the alkyl group R^{12alk} (if required) by hydrolysis, and/or (if required) exchange with a further optionally substituted alkyl group;
(d) reaction of a corresponding compound of formulae VIIIA, or of formula VIIIB, respectively: 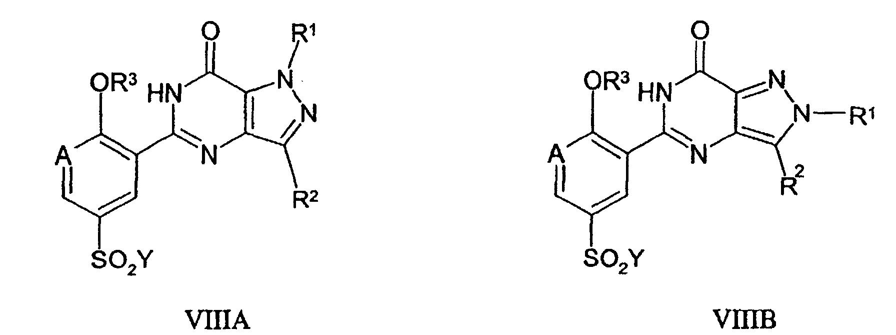 wherein Y is a leaving group, and R¹, R², R³ and A are as defined in Claim 1, with a compound of formula IX:
R¹⁴R¹⁵NH IX
wherein R¹⁴ and R¹⁵ are as defined in Claim 1;
(e) for compounds of formulae IA and IB, in which R¹ represents C₁₋₆ alkyl, alkylHet or alkylaryl, alkylation of a corresponding compound of formula IA, or of formula IB, respectively, in which R¹ represents H;
(f) for compounds of formulae IA and IB, in which R² represents C₁₋₆ alkyl (which alkyl group is branched and unsaturated at the carbon atom that is attached to the rest of the molecule), NR¹²R¹³, cyano, aryl or Het¹ (which Het¹ group is either aromatic or unsaturated at the carbon atom that is attached to the rest of the molecule), cross-coupling of a corresponding compound of formula XXIIIA, or of formula XXIIIB, respectively: 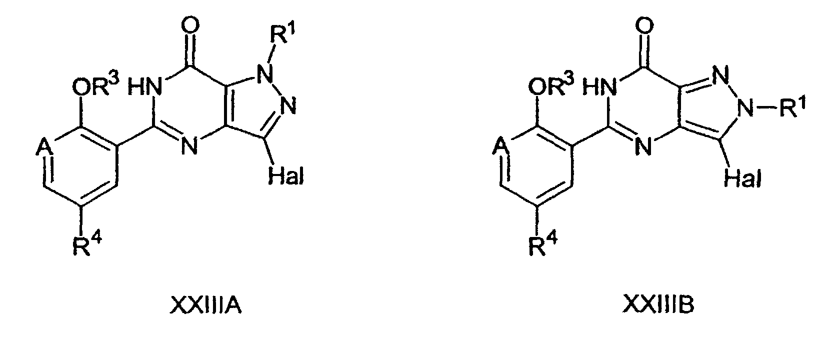 wherein Hal represents Cl, Br or I and R¹, R³, R⁴ and A are as defined in Claim 1, using a compound of formula
R^{2a}M
wherein R^{2a} represents C₁₋₆ alkyl (which alkyl group is branched and unsaturated at the carbon atom that is attached to M), NR¹²R¹³, cyano, aryl or Het¹ (which Het¹ group is either aromatic or unsaturated at the carbon atom that is attached to M), R¹² and R¹³ are as defined in Claim 1 and M represents an optionally substituted metal or boron group, which group is suitable for cross-coupling reactions;
(g) for compounds of formulae IA and IB in which R² represents N(H)C(O)R¹², acylation of a corresponding compound of formula IA or IB in which R² represents NH₂, using a compound of formula XXVI,
L¹C(O)R¹² XXVI
in which L¹ represents a leaving group and R¹² is as defined in Claim 1;
(h) for compounds of formulae IA and IB in which R² represents NR¹²R¹³ in which one of R¹² and R¹³ does not represent H, alkylation of a corresponding compound of formula IA or IB in which R² represents NH₂;
(i) for compounds of formulae IA and IB in which R² represents NR¹²R¹³ in which one of R¹² and R¹³ does not represent H, reductive amination of a compound of formula IA or IB in which R² represents NH₂, using an appropriate carbonyl compound;
(j) for compounds of formulae IA and IB in which R² represents NH₂, reduction of a corresponding compound of formula XXVIIA, or of formula XXVIIB, respectively: 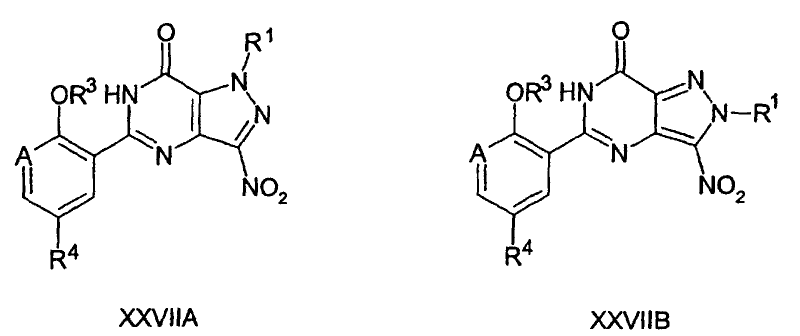 wherein A, R¹, R³ and R⁴ are as defined in Claim 1;
(k) conversion, removal or introduction of a substituent on an aryl, or a Het or Het¹, group in, or on the phenyl/pyridinyl, or pyrazolo, unit of, a compound of formula IA or IB;
(l) conversion of one R³ group to another by alkoxide exchange;
(m) for compounds of formula IA or IB in which R¹⁴ and R¹⁵, together with the nitrogen to which they are attached, form a 4-R¹⁶-piperazinyl group in which R¹⁶ represents alkyl, alkylation of a corresponding compound of formula IA or IB in which R¹⁶ represents hydrogen; or
(n) deprotection of a protected derivative of a compound of formula IA or of formula IB.

15. A compound of formula IIA, or formula IIB, as defined in Claim 14.

16. A compound of formula VIA, or formula VIB, is as defined in Claim 14.

17. A compound of formula VIIIA, or formula VIIIB, as defined in Claim 14.

18. A compound of formula XXIIIA, or formula XXIIIB, as defined in Claim 14.

19. A compound of formula XXVIIA, or formula XXVIIB, as defined in Claim 14.

## Patentansprüche

1. Verbindung der Formel IA oder Formel IB: 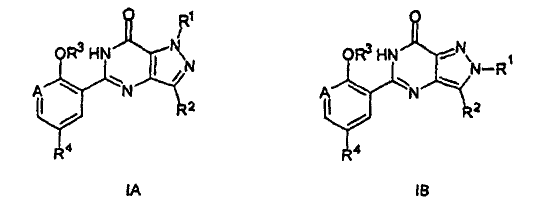 worin A N wiedergibt;
R¹ H, C₁₋₆-Alkyl, Het, AlkylHet, Aryl oder Alkylaryl wiedergibt, wobei die letzteren fünf Gruppen alle gegebenenfalls substituiert (und/oder im Fall von C₁₋₆-Alkyl gegebenenfalls beendet) sind mit einem oder mehreren Substituenten, ausgewählt aus Halogen, Cyano, Nitro, C₁₋₆-Alkyl, OR⁵, C(O)R⁶, C(O)OR⁷, C(O)NR⁸R⁹, NR^{10a}R^{10b} und SO₂NR^{11a}R^{11b};
R² C(O)NR¹²R¹³, C(O)OR¹², NR¹²R¹³, N(H)SO₂R¹², N(H)SO₂NR¹²R¹³, N(H)C(O)R¹², OR^{12a}, C₁₋₆-Alkyl (wobei die Alkylgruppe durch ein oder mehrere von O, S oder N(R¹²) unterbrochen und/oder substituiert oder beendet ist mit C(O)NR¹²R¹³, C(O)OR¹² oder Aryl oder Het¹), Cyano, Aryl oder Het¹ wiedergibt;
R³, R¹² und R¹³ unabhängig H oder C₁₋₆-Alkyl wiedergeben, wobei die Alkylgruppe gegebenenfalls substituiert und/oder gegebenenfalls beendet ist mit einem oder mehreren Substituenten, ausgewählt aus Aryl, Het, Halogen, Cyano, Nitro, OR⁵, C(O)R⁶, C(O)OR⁷, C(O)NR⁸R⁹, NR^{10a}R^{10b} und SO₂NR^{11a}R^{11b};
R⁴ SO₂NR¹⁴R¹⁵ wiedergibt;
R¹⁴ und R¹⁵ zusammen mit dem Stickstoffatom, an das sie gebunden sind, Het bilden;
Het eine gegebenenfalls substituierte vier- bis zwölfgliedrige heterocyclische Gruppe wiedergibt, wobei die Gruppe mindestens ein Stickstoffatom und gegebenenfalls ein oder mehrere weitere Heteroatome, ausgewählt aus Stickstoff, Sauerstoff und Schwefel, enthält;
Het¹ eine gegebenenfalls substituierte vier- bis zwölfgliedrige heterocyclische Gruppe wiedergibt, wobei die Gruppe mindestens ein Stickstoffatom oder mindestens ein Sauerstoffatom und gegebenenfalls ein oder mehrere weitere Heteroatome, ausgewählt aus Stickstoff, Sauerstoff und Schwefel, enthält, und
R⁵, R⁶, R⁷, R⁸, R⁹, R^{11a}, R^{11b} und R^{12a} unabhängig, wenn hierin verwendet, bei jedem Vorkommen H oder C₁₋₆-Alkyl wiedergeben;
R^{10a} und R^{10b}, wenn hierin verwendet, bei jedem Vorkommen, jeweils unabhängig H oder C₁₋₆-Alkyl wiedergeben oder zusammen mit dem Stickstoffatom, an das sie gebunden sind, Azetidinyl, Pyrrolidinyl oder Piperidinyl wiedergeben; oder ein Salz oder Solvat davon.

2. Verbindung nach Anspruch 1, worin R¹ H, eine lineare, verzweigte, cyclische, acyclische und/oder teilcyclische/acyclische C₁₋₆-Alkylgruppe, AlkylHet oder Alkylaryl wiedergibt.

3. Verbindung nach Anspruch 1 oder Anspruch 2, worin R² eine lineare oder verzweigte, gegebenenfalls ungesättigte C₁₋₆-Alkylgruppe (wobei die Alkylgruppe gegebenenfalls durch ein oder mehrere von O, S oder N(R¹²) unterbrochen ist), C(O)NR¹²R¹³, NR¹²R¹³, N(H)C(O)R¹², OR^{12a}, Aryl oder Het¹ wiedergibt.

4. Verbindung nach einem der vorangehenden Ansprüche, worin R³ lineares, verzweigtes, cyclisches und/oder acyclisches C₁₋₆-Alkyl wiedergibt, das gegebenenfalls mit einem oder mehreren Substituenten, ausgewählt aus Het oder OR⁵, substituiert oder beendet ist.

5. Verbindung nach einem der vorangehenden Ansprüche, worin R¹² und R¹³ unabhängig H oder lineares oder verzweigtes C₁₋₆-Alkyl wiedergeben, mit der Maßgabe, dass im Fall, wenn R² NR¹²R¹³ wiedergibt, R¹² und R¹³ beide nicht H wiedergeben.

6. Verbindung nach einem der vorangehenden Ansprüche, worin R¹⁴ und R¹⁵ zusammen mit dem Stickstoffatom, an das sie gebunden sind, 4-R¹⁶-Piperazinyl wiedergeben, worin R¹⁶ H oder C₁₋₆-Alkyl wiedergibt, wobei die letztere Gruppe gegebenenfalls substituiert oder beendet ist mit einem oder mehreren Substituenten, ausgewählt aus Aryl, Het, Halogen, Cyano, Nitro, OR⁵, C(O)R⁶, C(O)OR⁷, C(O)NR⁸R⁹, NR^{10a}R^{10b}, SO₂NR^{11a}R^{11b} und N(H)SO₂R¹², worin R⁵, R⁶, R⁷, R⁸, R⁹, R^{10a}, R^{10b}, R^{11a}, R^{11b} und R¹² wie in Anspruch 1 definiert sind.

7. Verbindung nach einem der Ansprüche 1 bis 6 zur Verwendung als Arzneimittel.

8. Verbindung nach einem der Ansprüche 1 bis 6 zur Verwendung als Tiermedikament.

9. Formulierung, umfassend eine Verbindung, wie in einem der Ansprüche 1 bis 6 definiert, in Anmischung mit einem pharmazeutisch oder veterinär verträglichen Hilfsmittel, Verdünnungsmittel oder Träger.

10. Formulierung nach Anspruch 9, die eine pharmazeutische Formulierung darstellt.

11. Formulierung nach Anspruch 9, die eine Veterinäre Formulierung darstellt.

12. Verwendung einer Verbindung, wie in einem der Ansprüche 1 bis 6 definiert, zur Herstellung eines Arzneimittels für die heilende oder prophylaktische Behandlung eines medizinischen Zustands, für den eine Inhibierung von cGMP PDE5 erwünscht ist.

13. Verwendung nach Anspruch 12, wobei der Zustand männliche Erektionsfunktionsstörung, weibliche Sexualfunktionsstörung, vorzeitige Wehen, Dysmenorrhoe, gutartige Prostatahyperplasie (BPH), Blasenauslassobstruktion, Inkontinenz, stabile oder instabile variante (Prinzmetall) Angina, Hypertension, pulmonale Hypertension, Herzstauungsinsuffizienz, Arteriosklerose, Schlaganfall, periphere vaskuläre Erkrankung, Zustände von verminderter Blutgefäßdurchgängigkeit, chronisches Asthma, Bronchitis, allergisches Asthma, allergischer Schnupfen, Glaukom, eine Erkrankung, **gekennzeichnet durch** Störungen der Darmmotilität, Präeclampsie, Kawasaki-Syndrom, Nitrattoleranz, multiple Sklerose, periphere diabetische Neuropathie, Schlaganfall, Alzheimer-Krankheit, akutes Atemversagen, Psoriasis, Hautnekrose, Krebsmetastasen, Kahlheit, Nussknackerösophagus, Analfissur und hypoxische Vasoconstriction ist.

14. Verfahren zur Herstellung einer Verbindung der Formel IA oder Formel IB, wie in Anspruch 1 definiert, das umfasst:
(a) Cyclisierung einer entsprechenden Verbindung der Formel IIA bzw. Formel IIB: 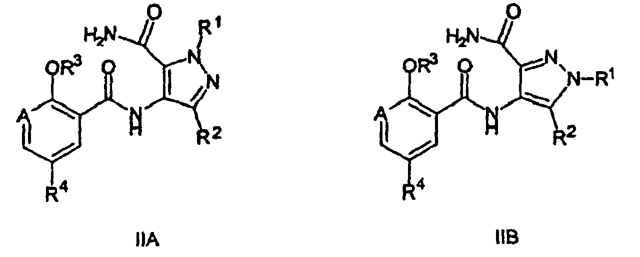 worin R¹, R², R³, R⁴ und A wie in Anspruch 1 definiert sind;
(b) für Verbindungen der Formeln IA und IB, worin R² C(O)NR¹²R¹³ wiedergibt, Reaktion einer entsprechenden Verbindung der Formel IA oder Formel IB, worin R² C(O)OH (oder ein Carbonsäurederivat davon) wiedergibt, mit einer Verbindung der Formel
HNR¹²R¹³,
worin R¹² und R¹³ wie in Anspruch 1 definiert sind;
(c) für Verbindungen der Formeln IA und IB, worin R² C(O)OR¹² wiedergibt, Cyclisierung einer entsprechenden Verbindung der Formel VIA bzw. der Formel VIB: 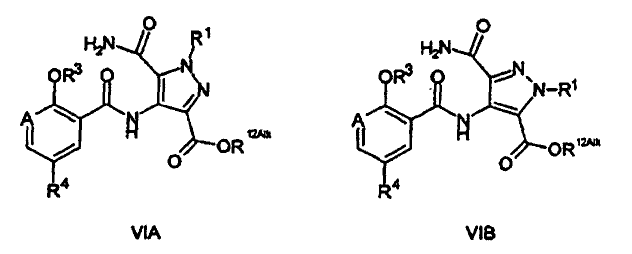 worin R¹, R³, R⁴ und A wie in Anspruch 1 definiert sind und R^{12Alk} eine gegebenenfalls substituierte C₁₋₆-Alkylgruppe wiedergibt, gefolgt von Entfernung der Alkylgruppe R^{12Alk} (falls erforderlich) durch Hydrolyse und/oder (falls erforderlich) Austausch mit einer weiteren gegebenenfalls substituierten Alkylgruppe;
(d) Reaktion einer entsprechenden Verbindung der Formel VIIIA bzw. der Formel VIIIB: 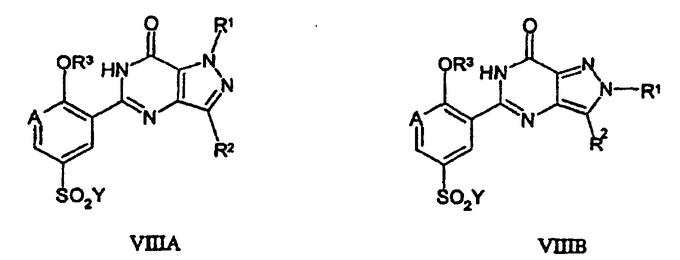 worin Y eine Abgangsgruppe darstellt und R¹, R², R³ und A wie in Anspruch 1 definiert sind, mit einer Verbindung der Formel IX:
R¹⁴R¹⁵NH IX
worin R¹⁴ und R¹⁵ wie in Anspruch 1 definiert sind;
(e) für Verbindungen der Formeln IA und IB, worin R¹ C₁₋₆-Alkyl, AlkylHet oder Alkylaryl wiedergibt, Alkylierung einer entsprechenden Verbindung der Formel IA bzw. der Formel IB, worin R¹ H wiedergibt;
(f) für Verbindungen der Formeln IA und IB, worin R² C₁₋₆-Alkyl (wobei die Alkylgruppe an dem Kohlenstoffatom, das an den Rest des Moleküls gebunden ist, verzweigt und ungesättigt ist), NR¹²R¹³, Cyano, Aryl oder Het¹ (wobei die Gruppe Het¹ an dem Kohlenstoffatom, das an den Rest des Moleküls gebunden ist, entweder aromatisch oder ungesättigt ist) wiedergibt, Kreuzkuppeln einer entsprechenden Verbindung der Formel XXIIIA bzw. der Formel XXIIIB: 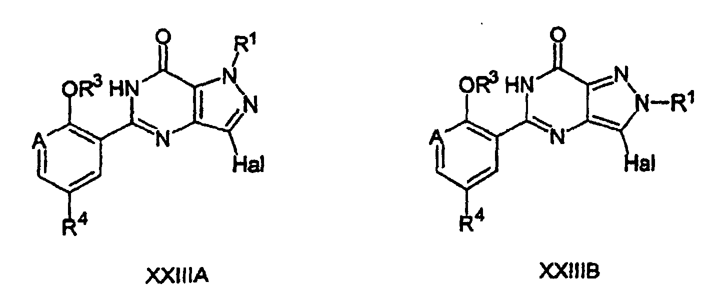 worin Hal Cl, Br oder I wiedergibt und R¹, R³, R⁴ und A wie in Anspruch 1 definiert sind, unter Verwendung einer Verbindung der Formel
R^{2a}M
worin R^{2a} C₁₋₆-Alkyl (wobei die Alkylgruppe verzweigt und an dem Kohlenstoff, das an M gebunden ist, ungesättigt ist), NR¹²R¹³, Cyano, Aryl oder Het¹ (wobei die Gruppe Het¹ an dem Kohlenstoffatom, das an M gebunden ist, entweder aromatisch oder ungesättigt ist) wiedergibt, R¹² und R¹³ wie in Anspruch 1 definiert sind, und M eine gegebenenfalls substituierte Metall- oder Borgruppe wiedergibt, wobei die Gruppe für Kreuzkupplungsreaktionen geeignet ist;
(g) für Verbindungen der Formeln IA und IB, worin R² N(H)C(O)R¹² wiedergibt, Acylierung einer entsprechenden Verbindung der Formel IA oder IB, worin R² NH₂ wiedergibt, unter Verwendung einer Verbindung der Formel XXVI
L¹C(O)R¹² XXVI
worin L¹ eine Abgangsgruppe darstellt und R¹² wie in Anspruch 1 definiert ist;
(h) für Verbindungen der Formeln IA und IB, worin R² NR¹²R¹³ wiedergibt, wobei einer von R¹² und R¹³ nicht H wiedergibt, Alkylierung einer entsprechenden Verbindung der Formel IA oder IB, worin R² NH₂ wiedergibt;
(i) für Verbindungen der Formeln IA und IB, worin R² NR¹²R¹³ wiedergibt, worin einer von R¹² und R¹³ nicht H wiedergibt, reduktive Aminierung einer Verbindung der Formel IA oder IB, worin R² NH₂ wiedergibt, unter Verwendung einer geeigneten Carbonylverbindung;
(j) für Verbindungen der Formeln IA und IB, worin R² NH₂ wiedergibt, Reduktion einer entsprechenden Verbindung der Formel XXVIIA bzw. der Formel XXVIIB: 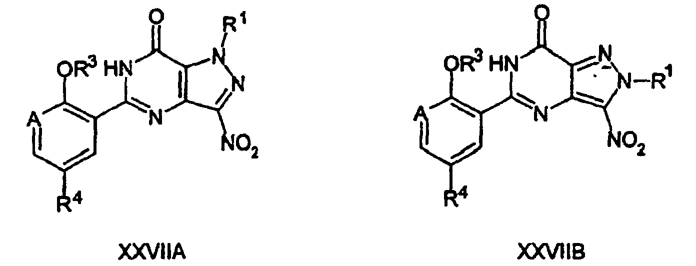 worin A, R¹, R³ und R⁴ wie in Anspruch 1 definiert sind;
(k) Umwandlung, Entfernung oder Einführung eines Substituenten an einer Gruppe Aryl oder Het oder Het¹, in die oder an der Einheit Phenyl/Pyridinyl, oder Pyrazolo, einer Verbindung der Formel IA oder IB;
(l) Umwandlung einer Gruppe R³ in eine weitere durch Alkoxidaustausch;
(m) für Verbindungen der Formel IA oder IB, worin R¹⁴ und R¹⁵ zusammen mit dem Stickstoffatom, an das sie gebunden sind, eine 4-R¹⁶-Piperazinylgruppe bilden, worin R¹⁶ Alkyl wiedergibt, Alkylierung einer entsprechenden Verbindung der Formel IA oder IB, worin R¹⁶ Wasserstoff wiedergibt, oder
(n) Schutzgruppenentfernung eines geschützten Derivats einer Verbindung der Formel IA oder der Formel IB.

15. Verbindung der Formel IIA oder Formel IIB, wie in Anspruch 14 definiert.

16. Verbindung der Formel VIA oder Formel VIB, wie in Anspruch 14 definiert.

17. Verbindung der Formel VIIIA oder Formel VIIIB, wie in Anspruch 14 definiert.

18. Verbindung der Formel XXIIIA oder Formel XXIIIB, wie in Anspruch 14 definiert.

19. Verbindung der Formel XXVIIA oder Formel XXVIIB, wie in Anspruch 14 definiert.

## Revendications

1. Composé de formule IA ou formule IB : 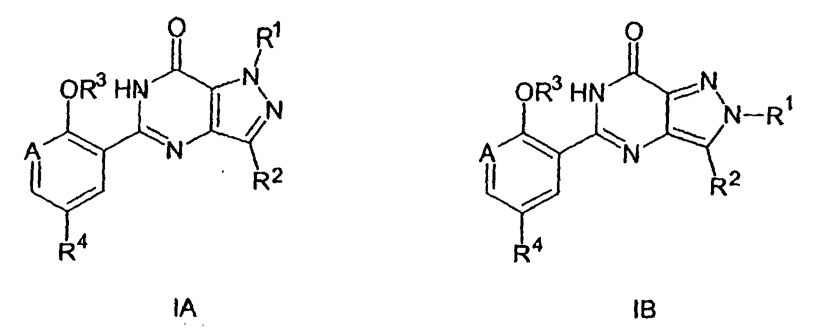 dans laquelle
A représente N ;
R¹ représente H, un groupe alkyle en C₁ à C₆, Het, alkylHet, aryle ou alkylaryle, ces cinq derniers groupes étant tous facultativement substitués (et/ou, dans le cas d'un groupe alkyle en C₁ à C₆, facultativement terminés) avec un ou plusieurs substituants choisis entre des substituants halogéno, cyano, nitro, alkyle en C₁ à C₆, OR⁵, C(O)R⁶, C(O)OR⁷, C(O)NR⁸R⁹, NR¹⁰R^{10b} et SO₂NR^{11a}R^{11b} ;
R² représente un groupe C(O)NR¹²R¹³, C(O)OR¹², NR¹²R¹³, N(H)SO₂R¹², N(H)SO₂NR¹²R¹³, N(H)C(O)R¹², OR^{12a}, alkyle en C₁ à C₆ (groupe alkyle qui est interrompu par un ou plusieurs atomes de O, S ou groupe N(R¹²) et/ou substitué ou terminé avec un groupe C(O)NR¹²R¹³, C(O)OR¹², aryle ou Het¹), cyano, aryle ou Het¹ ;
R³, R¹² et R¹³ représentent, indépendamment, H ou un groupe alkyle en C₁ à C₆, groupe alkyle qui est facultativement substitué et/ou facultativement terminé avec un ou plusieurs substituants choisis entre des substituants aryle, Het, halogéno, cyano, nitro, OR⁵, C(O)R⁶, C(O)OR⁷, C(O)NR⁸R⁹, NR^{10a}R^{10b} et SO₂NR^{11a}R^{11b} ;
R⁴ représente un groupe SO₂NR¹⁴R¹⁵ ;
R¹⁴ et R¹⁵, conjointement avec l'atome d'azote auquel ils sont fixés, forment un groupe Het ;
Het représente un groupe hétérocyclique tétra- à dodécagonal facultativement substitué, groupe qui contient au moins un atome d'azote et, facultativement, un ou plusieurs hétéroatomes supplémentaires choisis entre l'azote, l'oxygène et le soufre ;
Het¹ représente un groupe hétérocyclique tétra- à dodécagonal facultativement substitué, groupe qui contient au moins un atome d'azote ou au moins un atome d'oxygène et, facultativement, un ou plusieurs hétéroatomes supplémentaires choisis entre l'azote, l'oxygène et le soufre ; et
R⁵, R⁶, R⁷, R⁸, R⁹, R^{11a}, R^{11b} et R^{12a} représentent, indépendamment, à chaque emplacement lorsqu'ils sont utilisés dans ce cas, H ou un groupe alkyle en C₁ à C₆ ;
R^{10a} et R^{10b}, à chaque emplacement lorsqu'ils sont utilisés dans ce cas, représentent, indépendamment, H ou un groupe alkyle en C₁ à C₆ ou bien, conjointement avec l'atome d'azote auquel ils sont fixés, ils représentent un groupe azétidinyle, pyrrolidinyle ou pipéridinyle ;
ou un de ses sels ou produits de solvatation.

2. Composé suivant la revendication 1, dans lequel R¹ représente H, un groupe alkyle en C₁ à C₆ linéaire, ramifié, cyclique, acyclique et/ou cyclique/acyclique partiel, alkylHet ou alkylaryle.

3. Composé suivant la revendication 1 ou la revendication 2, dans lequel R² représente un groupe alkyle en C₁ à C₆ linéaire ou ramifié, facultativement insaturé (groupe alkyle qui est facultativement interrompu par un ou plusieurs atomes de O, S ou groupes N(R¹²)), C(O)NR¹²R¹³, NR¹²R¹³, N(H)C(O)R¹², OR^{12a}, aryle ou Het¹.

4. Composé suivant l'une quelconque des revendications précédentes, dans lequel R³ représente un groupe alkyle en C₁ à C₆ linéaire, ramifié, cyclique et/ou acyclique qui est facultativement substitué ou terminé avec un ou plusieurs substituants choisis entre des substituants Het et OR⁵.

5. Composé suivant l'une quelconque des revendications précédentes, dans lequel R¹² et R¹³ représentent, indépendamment, H ou un groupe alkyle en C₁ à C₆ linéaire
ou ramifié, sous réserve que, dans le cas où R² représente un groupe NR¹²R¹³, R¹² et R¹³ ne représentent ni l'un ni l'autre H.

6. Composé suivant l'une quelconque des revendications précédentes, dans lequel R¹⁴ et R¹⁵, conjointement avec l'atome d'azote auquel ils sont fixés, représentent un groupe 4-R¹⁶-pipérazinyle, dans lequel R¹⁶ représente H ou un groupe alkyle en C₁ à C₆, ce dernier groupe étant facultativement substitué ou terminé avec un ou plusieurs substituants choisis entre des substituants aryle, Het, halogéno, cyano, nitro, OR⁵, C(O)R⁶, C(O)OR⁷, C(O)NR⁸R⁹, NR^{10a}R^{10b}, SO₂NR^{11a}R^{11b} et N(H)SO₂R¹², dans lesquels R⁵, R⁶, R⁷, R⁸, R⁹, R^{10a}, R^{10b}, R^{11a}, R^{11b} et R¹² répondent aux définitions suivant la revendication 1.

7. Composé suivant l'une quelconque des revendications 1 à 6, destiné à être utilisé comme agent pharmaceutique.

8. Composé suivant l'une quelconque des revendications 1 à 6, destiné à être utilisé comme médicament pour animaux.

9. Formulation comprenant un composé suivant l'une quelconque des revendications 1 à 6 en mélange avec un adjuvant, diluant ou support acceptable du point de vue pharmaceutique ou vétérinaire.

10. Formulation suivant la revendication 9, qui est une formulation pharmaceutique.

11. Formulation suivant la revendication 9, qui est une formulation vétérinaire.

12. Utilisation d'un composé suivant l'une quelconque des revendications 1 à 6 pour la production d'un médicament destiné au traitement curatif ou prophylactique d'un état médical pour lequel une inhibition de la cGMP-PDE5 est désirée.

13. Utilisation suivant la revendication 12, dans laquelle la condition est un dysfonctionnement de l'érection masculine, le dysfonctionnement sexuel féminin, le travail prématuré, la dysménorrhée, l'hyperplasie prostatique bénigne (BPH), une obstruction de la sortie de la vessie, l'incontinence, l'angor variant stable ou instable (le Prinzmétal), l'hypertension, l'hypertension pulmonaire, l'insuffisance cardiaque congestive, l'athérosclérose, un ictus, une maladie vasculaire périphérique, des affections comportant une patence réduite des vaisseaux sanguins, l'asthme chronique, la bronchite, l'asthme allergique, la rhinite allergique, le glaucome, une maladie **caractérisée par** des troubles de motilité instestinale, la pré-éclampsie, le syndrome de Kawasaki, la tolérance aux nitrates, la sclérose en plaques, la neuropathie diabétique périphérique, un ictus, la maladie d'Alzheimer, l'insuffisance respiratoire aiguë, le psoriasis, une nécrose cutanée, une métastase cancéreuse, la calvitie, l'oesophage en casse-noix, une fissure anale et une vasoconstriction hypoxique.

14. Procédé pour la préparation d'un composé de formule IA, ou de formule IB, suivant la revendication 1, qui comprend :
(a) la cyclisation d'un composé correspondant de formue IIA, ou de formule IIB, respectivement : 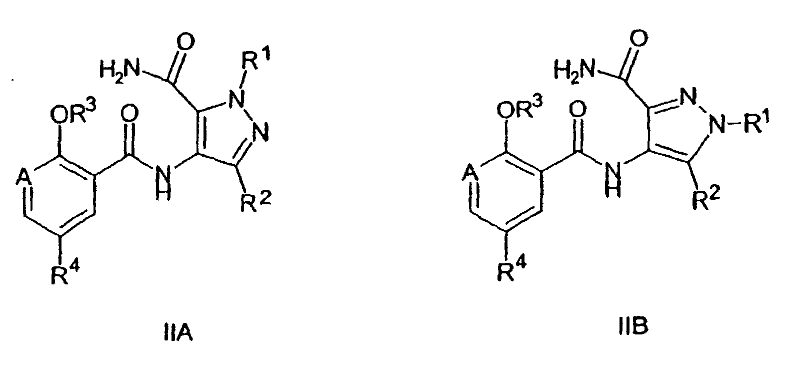 dans laquelle R¹, R², R³, R⁴ et A répondent aux définitions suivant la revendication 1 ;
(b) pour les composés de formules IA et IB, dans lesquelles R² représente un groupe C(O)NR¹²R¹³, la réaction d'un composé correspondant de formule IA, ou de formule IB, dans laquelle R² représente un groupe C(O)OH (ou de ses dérivés d'acide carboxylique) avec un composé de formule
HNR¹²R¹³,
dans laquelle R¹² et R¹³ répondent aux définitions suivant la revendication 1 ;
(c) pour des composés de formules IA et IB, dans lesquelles R² représente un groupe C(O)OR¹², la cyclisation d'un composé correspondant de formule VIA, ou de formule VIB, respectivement : 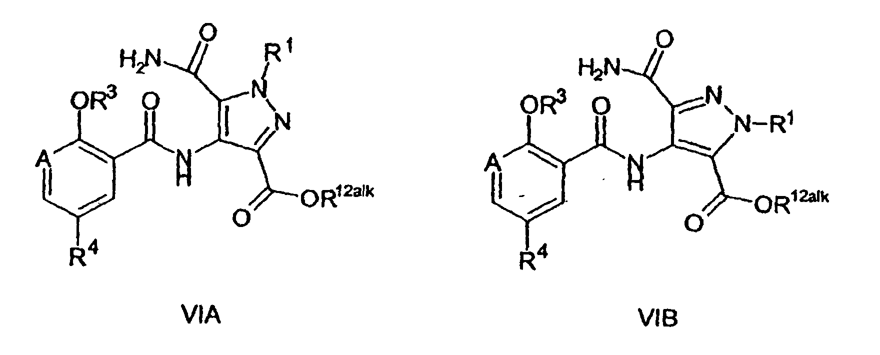 dans laquelle R¹, R³, R⁴ et A répondent aux définitions suivant la revendication 1, et R^{12alk} représente un groupe alkyle en C₁ à C₆ facultativement substitué, puis l'élimination du groupe alkyle R^{12alk} (si besoin) par hydrolyse, et/ou (si besoin) un échange avec un autre groupe alkyle facultativement substitué ;
(d) la réaction d'un composé correspondant de formule VIIIA, ou de formule VIIIB, respectivement : 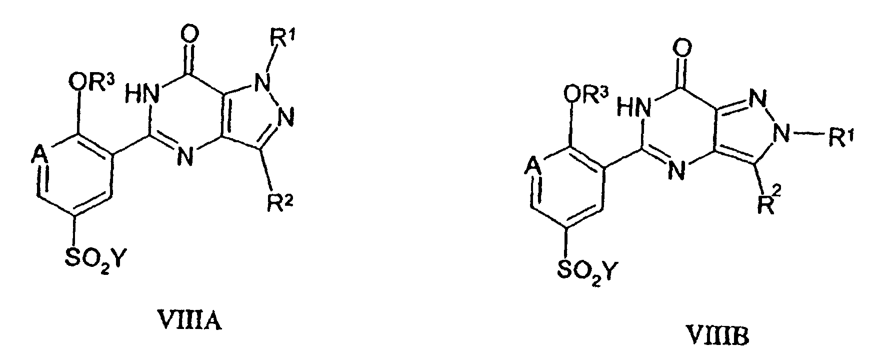 dans laquelle Y représente un groupe partant, R¹, R², R³ et A répondent aux définitions suivant la revendication 1, avec un composé de formule IX :
R¹⁴R¹⁵NH IX
dans laquelle R¹⁴ et R¹⁵ répondent aux définitions suivant la revendication 1 ;
(e) pour des composés de formules IA et IB, dans lesquelles R¹ représente un groupe alkyle en C₁ à C₆, alkylHet ou alkylaryle, l'alkylation d'un composé correspondant de formule IA, ou de formule IB, respectivement, dans laquelle R¹ représente H ;
(f) pour des composés de formules IA et IB, dans lesquelles R² représente un groupe alkyle en C₁ à C₆ (groupe alkyle qui est ramifié et insaturé au niveau de l'atome de carbone qui est fixé au reste de la molécule) , NR¹²R¹³, cyano, aryle ou Het¹ (groupe Het¹ qui est aromatique ou insaturé au niveau de l'atome de carbone qui est fixé au reste de la molécule), le couplage croisé d'un composé correspondant de formule XXIIIA, ou de formule XXIIIB, respectivement : 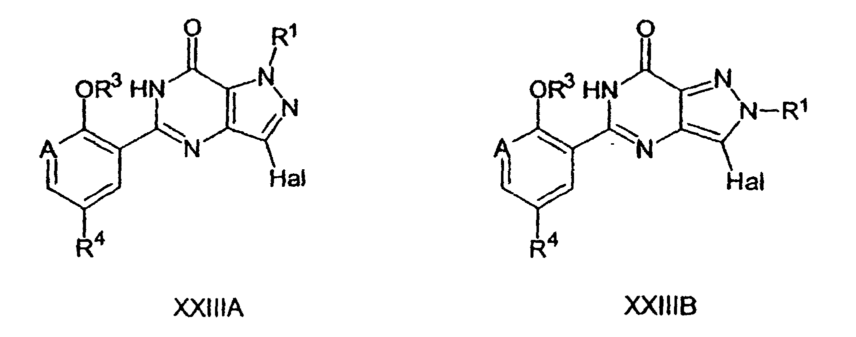 dans laquelle Hal représente Cl, Br ou I et R¹, R³, R⁴ et A répondent aux définitions suivant la revendication 1, en utilisant un composé de formule
R^{2a}M
dans laquelle R^{2a} représente un groupe alkyle en C₁ à C₆ (groupe alkyle qui est ramifié et insaturé au niveau de l'atome de carbone qui est fixé à M), NR¹²R¹³, cyano, aryle
ou Het¹ (groupe Het¹ qui est aromatique ou insaturé au niveau de l'atome de carbone qui est fixé à M), R¹² et R¹³ répondent aux définitions suivant la revendication 1 et M représente un groupe métal ou bore facultativement substitué, groupe qui est apte à des réactions de couplage croisé ;
(g) pour des composés de formules IA et IB dans lesquelles R² représente un groupe N(H)C(O)R¹², l'acylation d'un composé correspondant de formule IA ou IB dans laquelle R² représente un groupe NH₂, en utilisant un composé de formule XXVI
L¹C(O)R¹² XXVI
dans laquelle L¹ représente un groupe partant et R¹² répond à la définition suivant la revendication 1 ;
(h) pour des composés de formules IA et IB dans lesquelles R² représente un groupe NR¹²R¹³ dans lequel un des groupes R¹² et R¹³ ne représente pas H, l'alkylation d'un composé correspondant de formule IA ou IB dans laquelle R² représente un groupe NH₂ ;
(i) pour des composés de formules IA et IB dans lesquelles R² représente un groupe NR¹²R¹³ dans lequel un des groupes R¹² et R¹³ ne représente pas H, l'amination réductrice d'un composé de formule IA ou IB dans laquelle R² représente un groupe NH₂, en utilisant un composé carbonylé approprié ;
(j) pour des composés de formules IA et IB dans lesquelles R² représente un groupe NH₂, la réduction d'un composé correspondant de formule XXVIIA, ou de formule XXVIIB, respectivement : 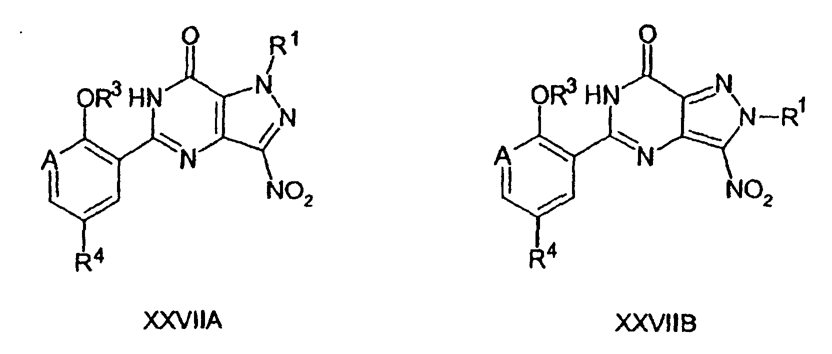 dans laquelle A, R¹, R³ et R⁴ répondent aux définitions suivant la revendication 1 ;
(k) la conversion, l'élimination ou l'introduction de substituant sur un groupe aryle ou un groupe Het ou Het¹, dans ou sur le motif phényle/pyridinyle ou pyrazolo d'un composé de formule IA ou IB ;
(l) la conversion d'un groupe R³ en un autre par échange d'alcoolate ;
(m) pour des composés de formule IA ou IB dans laquelle R¹⁴ et R¹⁵, conjointement avec l'atome d'azote auquel ils sont fixés, forment un groupe 4-R¹⁶-pipérazinyle dans lequel R¹⁶ représente un groupe alkyle, l'alkylation d'un composé correspondant de formule IA ou IB dans laquelle R¹⁶ représente un atome d'hydrogène ; ou
(n) la suppression de la protection d'un dérivé protégé d'un composé de formule IA ou de formule IB.

15. Composé de formule IIa, ou de formule IIB, suivant la revendication 14.

16. Composé de formule VIA, ou de formule VIB, suivant la revendication 14.

17. Composé de formule VIIIA, ou de formule VIIIB, suivant la revendication 14.

18. Composé de formule XXIIIA, ou de formule XXIIIB, suivant la revendication 14.

19. Composé de formule XXVIIA, ou de formule XXVIIB, suivant la revendication 14.
